(19)

Europäisches Patentamt
European Patent Office
Office européen des brevets

(11) **EP 3 770 269 A1**

(12) **EUROPEAN PATENT APPLICATION**

(43) Date of publication:
    **27.01.2021 Bulletin 2021/04**

(21) Application number: **19187963.4**

(22) Date of filing: **23.07.2019**

(51) Int Cl.:
    *C12P 21/00* (2006.01)          *C07K 14/31* (2006.01)
    *C07K 14/205* (2006.01)         *C07K 14/21* (2006.01)
    *C07K 14/22* (2006.01)          *C07K 14/245* (2006.01)
    *C07K 14/33* (2006.01)          *C12Q 1/37* (2006.01)
    *A61K 39/085* (2006.01)         *A61K 39/02* (2006.01)
    *G01N 33/68* (2006.01)

(84) Designated Contracting States:
    **AL AT BE BG CH CY CZ DE DK EE ES FI FR GB
    GR HR HU IE IS IT LI LT LU LV MC MK MT NL NO
    PL PT RO RS SE SI SK SM TR**
    Designated Extension States:
    **BA ME**
    Designated Validation States:
    **KH MA MD TN**

(71) Applicant: **GlaxoSmithKline Biologicals S.A.
    1330 Rixensart (BE)**

(72) Inventor: **The designation of the inventor has not
    yet been filed**

(74) Representative: **Sanderson, Andrew John
    GlaxoSmithKline
    Global Patents, CN9.25.1
    980 Great West Road
    Brentford
    Middlesex TW8 9GS (GB)**

Remarks:
    Claims filed after the date of filing of the application
    (Rule 68(4) EPC).

(54) **QUANTIFICATION OF BIOCONJUGATE GLYCOSYLATION**

(57)    The present invention provides analytical tools for the characterisation of bioconjugates, in particular the measurement of glycosylation levels. Methods for absolute quantification of glycosylation sequences are disclosed, as are novel sequences and glycosylation sites for use in such methods.

Fig 1

1. In silico Design of consensus sequences

2. Assess the behavior of the PTP in MS

3. Assess the efficacy of bioconjugation of the selected consensus sequence

4. Quantification of the extent of glycosylation of a carrier protein with single consensus sequence

5. Quantification of the extend of glycosylation of a carrier with multiple consensus sequence

SEQUENCE LISTING

<110> GlaxoSmithKline Biologicals

<120> QUANTIFICATION OF BIOCONJUGATE GLYCOSYLATION

<130> VB66747P EP

<150> 19187963.4
<151> 2019-07-23

<160> 47

<170> PatentIn version 3.5

<210> 1
<211> 613
<212> PRT
<213> Pseudomonas aeruginosa

<400> 1

Ala Glu Glu Ala Phe Asp Leu Trp Asn Glu Cys Ala Lys Ala Cys Val
1               5                   10                  15


Leu Asp Leu Lys Asp Gly Val Arg Ser Ser Arg Met Ser Val Asp Pro
            20                  25                  30


Ala Ile Ala Asp Thr Asn Gly Gln Gly Val Leu His Tyr Ser Met Val
        35                  40                  45


Leu Glu Gly Gly Asn Asp Ala Leu Lys Leu Ala Ile Asp Asn Ala Leu
    50                  55                  60


Ser Ile Thr Ser Asp Gly Leu Thr Ile Arg Leu Glu Gly Gly Val Glu
65                  70                  75                  80


Pro Asn Lys Pro Val Arg Tyr Ser Tyr Thr Arg Gln Ala Arg Gly Ser
            85                  90                  95


Trp Ser Leu Asn Trp Leu Val Pro Ile Gly His Glu Lys Pro Ser Asn
            100                 105                 110


Ile Lys Val Phe Ile His Glu Leu Asn Ala Gly Asn Gln Leu Ser His
            115                 120                 125


Met Ser Pro Ile Tyr Thr Ile Glu Met Gly Asp Glu Leu Leu Ala Lys
        130                 135                 140


Leu Ala Arg Asp Ala Thr Phe Phe Val Arg Ala His Glu Ser Asn Glu
145                 150                 155                 160


Met Gln Pro Thr Leu Ala Ile Ser His Ala Gly Val Ser Val Val Met

                    165                         170                         175

Ala Gln Ala Gln Pro Arg Arg Glu Lys Arg Trp Ser Glu Trp Ala Ser
            180                     185                 190

Gly Lys Val Leu Cys Leu Leu Asp Pro Leu Asp Gly Val Tyr Asn Tyr
            195                     200                 205

Leu Ala Gln Gln Arg Cys Asn Leu Asp Asp Thr Trp Glu Gly Lys Ile
            210                     215                 220

Tyr Arg Val Leu Ala Gly Asn Pro Ala Lys His Asp Leu Asp Ile Lys
225                     230                     235                 240

Pro Thr Val Ile Ser His Arg Leu His Phe Pro Glu Gly Gly Ser Leu
                245                     250                 255

Ala Ala Leu Thr Ala His Gln Ala Cys His Leu Pro Leu Glu Ala Phe
            260                     265                 270

Thr Arg His Arg Gln Pro Arg Gly Trp Glu Gln Leu Glu Gln Cys Gly
            275                     280                 285

Tyr Pro Val Gln Arg Leu Val Ala Leu Tyr Leu Ala Ala Arg Leu Ser
        290                     295                 300

Trp Asn Gln Val Asp Gln Val Ile Arg Asn Ala Leu Ala Ser Pro Gly
305                     310                     315                 320

Ser Gly Gly Asp Leu Gly Glu Ala Ile Arg Glu Gln Pro Glu Gln Ala
                325                     330                 335

Arg Leu Ala Leu Thr Leu Ala Ala Ala Glu Ser Glu Arg Phe Val Arg
            340                     345                 350

Gln Gly Thr Gly Asn Asp Glu Ala Gly Ala Ala Ser Ala Asp Val Val
        355                     360                 365

Ser Leu Thr Cys Pro Val Ala Ala Gly Glu Cys Ala Gly Pro Ala Asp
        370                     375                 380

Ser Gly Asp Ala Leu Leu Glu Arg Asn Tyr Pro Thr Gly Ala Glu Phe
385                     390                     395                 400

Leu Gly Asp Gly Gly Asp Val Ser Phe Ser Thr Arg Gly Thr Gln Asn
                405                     410                 415

```
Trp Thr Val Glu Arg Leu Leu Gln Ala His Arg Gln Leu Glu Glu Arg
        420             425             430

Gly Tyr Val Phe Val Gly Tyr His Gly Thr Phe Leu Glu Ala Ala Gln
        435             440             445

Ser Ile Val Phe Gly Gly Val Arg Ala Arg Ser Gln Asp Leu Asp Ala
        450             455             460

Ile Trp Arg Gly Phe Tyr Ile Ala Gly Asp Pro Ala Leu Ala Tyr Gly
465             470             475             480

Tyr Ala Gln Asp Gln Glu Pro Asp Ala Arg Gly Arg Ile Arg Asn Gly
            485             490             495

Ala Leu Leu Arg Val Tyr Val Pro Arg Trp Ser Leu Pro Gly Phe Tyr
        500             505             510

Arg Thr Gly Leu Thr Leu Ala Ala Pro Glu Ala Ala Gly Glu Val Glu
        515             520             525

Arg Leu Ile Gly His Pro Leu Pro Leu Arg Leu Asp Ala Ile Thr Gly
        530             535             540

Pro Glu Glu Glu Gly Gly Arg Leu Glu Thr Ile Leu Gly Trp Pro Leu
545             550             555             560

Ala Glu Arg Thr Val Val Ile Pro Ser Ala Ile Pro Thr Asp Pro Arg
            565             570             575

Asn Val Gly Gly Asp Leu Asp Pro Ser Ser Ile Pro Asp Lys Glu Gln
        580             585             590

Ala Ile Ser Ala Leu Pro Asp Tyr Ala Ser Gln Pro Gly Lys Pro Pro
        595             600             605

Arg Glu Asp Leu Lys
        610


<210>   2
<211>   612
<212>   PRT
<213>   Artificial Sequence

<220>
<223>   Amino acid sequence of EPA with L552V/deltaE553 detoxifying mutation

<400>   2

Ala Glu Glu Ala Phe Asp Leu Trp Asn Glu Cys Ala Lys Ala Cys Val
```

53

```
        1               5                   10                  15

        Leu Asp Leu Lys Asp Gly Val Arg Ser Ser Arg Met Ser Val Asp Pro
                    20                  25                  30

        Ala Ile Ala Asp Thr Asn Gly Gln Gly Val Leu His Tyr Ser Met Val
                    35                  40                  45

        Leu Glu Gly Gly Asn Asp Ala Leu Lys Leu Ala Ile Asp Asn Ala Leu
                50                  55                  60

        Ser Ile Thr Ser Asp Gly Leu Thr Ile Arg Leu Glu Gly Gly Val Glu
        65                  70                  75                  80

        Pro Asn Lys Pro Val Arg Tyr Ser Tyr Thr Arg Gln Ala Arg Gly Ser
                        85                  90                  95

        Trp Ser Leu Asn Trp Leu Val Pro Ile Gly His Glu Lys Pro Ser Asn
                    100                 105                 110

        Ile Lys Val Phe Ile His Glu Leu Asn Ala Gly Asn Gln Leu Ser His
                    115                 120                 125

        Met Ser Pro Ile Tyr Thr Ile Glu Met Gly Asp Glu Leu Leu Ala Lys
                130                 135                 140

        Leu Ala Arg Asp Ala Thr Phe Phe Val Arg Ala His Glu Ser Asn Glu
        145                 150                 155                 160

        Met Gln Pro Thr Leu Ala Ile Ser His Ala Gly Val Ser Val Val Met
                        165                 170                 175

        Ala Gln Ala Gln Pro Arg Arg Glu Lys Arg Trp Ser Glu Trp Ala Ser
                    180                 185                 190

        Gly Lys Val Leu Cys Leu Leu Asp Pro Leu Asp Gly Val Tyr Asn Tyr
                    195                 200                 205

        Leu Ala Gln Gln Arg Cys Asn Leu Asp Asp Thr Trp Glu Gly Lys Ile
                210                 215                 220

        Tyr Arg Val Leu Ala Gly Asn Pro Ala Lys His Asp Leu Asp Ile Lys
        225                 230                 235                 240

        Pro Thr Val Ile Ser His Arg Leu His Phe Pro Glu Gly Gly Ser Leu
                        245                 250                 255
```

Ala Ala Leu Thr Ala His Gln Ala Cys His Leu Pro Leu Glu Ala Phe
        260             265             270

Thr Arg His Arg Gln Pro Arg Gly Trp Glu Gln Leu Glu Gln Cys Gly
        275             280             285

Tyr Pro Val Gln Arg Leu Val Ala Leu Tyr Leu Ala Ala Arg Leu Ser
        290             295             300

Trp Asn Gln Val Asp Gln Val Ile Arg Asn Ala Leu Ala Ser Pro Gly
305             310             315             320

Ser Gly Gly Asp Leu Gly Glu Ala Ile Arg Glu Gln Pro Glu Gln Ala
        325             330             335

Arg Leu Ala Leu Thr Leu Ala Ala Ala Glu Ser Glu Arg Phe Val Arg
        340             345             350

Gln Gly Thr Gly Asn Asp Glu Ala Gly Ala Ala Ser Ala Asp Val Val
        355             360             365

Ser Leu Thr Cys Pro Val Ala Ala Gly Glu Cys Ala Gly Pro Ala Asp
        370             375             380

Ser Gly Asp Ala Leu Leu Glu Arg Asn Tyr Pro Thr Gly Ala Glu Phe
385             390             395             400

Leu Gly Asp Gly Gly Asp Val Ser Phe Ser Thr Arg Gly Thr Gln Asn
                405             410             415

Trp Thr Val Glu Arg Leu Leu Gln Ala His Arg Gln Leu Glu Glu Arg
        420             425             430

Gly Tyr Val Phe Val Gly Tyr His Gly Thr Phe Leu Glu Ala Ala Gln
        435             440             445

Ser Ile Val Phe Gly Gly Val Arg Ala Arg Ser Gln Asp Leu Asp Ala
        450             455             460

Ile Trp Arg Gly Phe Tyr Ile Ala Gly Asp Pro Ala Leu Ala Tyr Gly
465             470             475             480

Tyr Ala Gln Asp Gln Glu Pro Asp Ala Arg Gly Arg Ile Arg Asn Gly
                485             490             495

Ala Leu Leu Arg Val Tyr Val Pro Arg Trp Ser Leu Pro Gly Phe Tyr
        500             505             510

```
Arg Thr Gly Leu Thr Leu Ala Ala Pro Glu Ala Ala Gly Glu Val Glu
        515                 520                 525

Arg Leu Ile Gly His Pro Leu Pro Leu Arg Leu Asp Ala Ile Thr Gly
        530                 535                 540

Pro Glu Glu Glu Gly Gly Arg Val Thr Ile Leu Gly Trp Pro Leu Ala
545                 550                 555                 560

Glu Arg Thr Val Val Ile Pro Ser Ala Ile Pro Thr Asp Pro Arg Asn
                565                 570                 575

Val Gly Gly Asp Leu Asp Pro Ser Ser Ile Pro Asp Lys Glu Gln Ala
                580                 585                 590

Ile Ser Ala Leu Pro Asp Tyr Ala Ser Gln Pro Gly Lys Pro Pro Arg
        595                 600                 605

Glu Asp Leu Lys
        610


<210>  3
<211>  1315
<212>  PRT
<213>  Clostridium tetani

<400>  3

Met Pro Ile Thr Ile Asn Asn Phe Arg Tyr Ser Asp Pro Val Asn Asn
1               5                   10                  15

Asp Thr Ile Ile Met Met Glu Pro Pro Tyr Cys Lys Gly Leu Asp Ile
        20                  25                  30

Tyr Tyr Lys Ala Phe Lys Ile Thr Asp Arg Ile Trp Ile Val Pro Glu
        35                  40                  45

Arg Tyr Glu Phe Gly Thr Lys Pro Glu Asp Phe Asn Pro Pro Ser Ser
        50                  55                  60

Leu Ile Glu Gly Ala Ser Glu Tyr Tyr Asp Pro Asn Tyr Leu Arg Thr
65                  70                  75                  80

Asp Ser Asp Lys Asp Arg Phe Leu Gln Thr Met Val Lys Leu Phe Asn
                85                  90                  95

Arg Ile Lys Asn Asn Val Ala Gly Glu Ala Leu Leu Asp Lys Ile Ile
            100                 105                 110
```

Asn Ala Ile Pro Tyr Leu Gly Asn Ser Tyr Ser Leu Leu Asp Lys Phe
        115                 120                 125

Asp Thr Asn Ser Asn Ser Val Ser Phe Asn Leu Leu Glu Gln Asp Pro
        130                 135                 140

Ser Gly Ala Thr Thr Lys Ser Ala Met Leu Thr Asn Leu Ile Ile Phe
145                 150                 155                 160

Gly Pro Gly Pro Val Leu Asn Lys Asn Glu Val Arg Gly Ile Val Leu
                165                 170                 175

Arg Val Asp Asn Lys Asn Tyr Phe Pro Cys Arg Asp Gly Phe Gly Ser
            180                 185                 190

Ile Met Gln Met Ala Phe Cys Pro Glu Tyr Val Pro Thr Phe Asp Asn
        195                 200                 205

Val Ile Glu Asn Ile Thr Ser Leu Thr Ile Gly Lys Ser Lys Tyr Phe
    210                 215                 220

Gln Asp Pro Ala Leu Leu Leu Met His Glu Leu Ile His Val Leu His
225                 230                 235                 240

Gly Leu Tyr Gly Met Gln Val Ser Ser His Glu Ile Ile Pro Ser Lys
                245                 250                 255

Gln Glu Ile Tyr Met Gln His Thr Tyr Pro Ile Ser Ala Glu Glu Leu
            260                 265                 270

Phe Thr Phe Gly Gly Gln Asp Ala Asn Leu Ile Ser Ile Asp Ile Lys
        275                 280                 285

Asn Asp Leu Tyr Glu Lys Thr Leu Asn Asp Tyr Lys Ala Ile Ala Asn
    290                 295                 300

Lys Leu Ser Gln Val Thr Ser Cys Asn Asp Pro Asn Ile Asp Ile Asp
305                 310                 315                 320

Ser Tyr Lys Gln Ile Tyr Gln Gln Lys Tyr Gln Phe Asp Lys Asp Ser
            325                 330                 335

Asn Gly Gln Tyr Ile Val Asn Glu Asp Lys Phe Gln Ile Leu Tyr Asn
        340                 345                 350

Ser Ile Met Tyr Gly Phe Thr Glu Ile Glu Leu Gly Lys Lys Phe Asn
        355                 360                 365

```
Ile Lys Thr Arg Leu Ser Tyr Phe Ser Met Asn His Asp Pro Val Lys
    370                 375                 380

Ile Pro Asn Leu Leu Asp Asp Thr Ile Tyr Asn Asp Thr Glu Gly Phe
385                 390                 395                 400

Asn Ile Glu Ser Lys Asp Leu Lys Ser Glu Tyr Lys Gly Gln Asn Met
            405                 410                 415

Arg Val Asn Thr Asn Ala Phe Arg Asn Val Asp Gly Ser Gly Leu Val
            420                 425                 430

Ser Lys Leu Ile Gly Leu Cys Lys Lys Ile Ile Pro Pro Thr Asn Ile
        435                 440                 445

Arg Glu Asn Leu Tyr Asn Arg Thr Ala Ser Leu Thr Asp Leu Gly Gly
    450                 455                 460

Glu Leu Cys Ile Lys Ile Lys Asn Glu Asp Leu Thr Phe Ile Ala Glu
465                 470                 475                 480

Lys Asn Ser Phe Ser Glu Glu Pro Phe Gln Asp Glu Ile Val Ser Tyr
            485                 490                 495

Asn Thr Lys Asn Lys Pro Leu Asn Phe Asn Tyr Ser Leu Asp Lys Ile
        500                 505                 510

Ile Val Asp Tyr Asn Leu Gln Ser Lys Ile Thr Leu Pro Asn Asp Arg
        515                 520                 525

Thr Thr Pro Val Thr Lys Gly Ile Pro Tyr Ala Pro Glu Tyr Lys Ser
    530                 535                 540

Asn Ala Ala Ser Thr Ile Glu Ile His Asn Ile Asp Asp Asn Thr Ile
545                 550                 555                 560

Tyr Gln Tyr Leu Tyr Ala Gln Lys Ser Pro Thr Thr Leu Gln Arg Ile
                565                 570                 575

Thr Met Thr Asn Ser Val Asp Asp Ala Leu Ile Asn Ser Thr Lys Ile
            580                 585                 590

Tyr Ser Tyr Phe Pro Ser Val Ile Ser Lys Val Asn Gln Gly Ala Gln
        595                 600                 605

Gly Ile Leu Phe Leu Gln Trp Val Arg Asp Ile Ile Asp Asp Phe Thr
```

```
            610                      615                      620

Asn Glu Ser Ser Gln Lys Thr Thr Ile Asp Lys Ile Ser Asp Val Ser
625                 630             635                 640

Thr Ile Val Pro Tyr Ile Gly Pro Ala Leu Asn Ile Val Lys Gln Gly
                645             650                 655

Tyr Glu Gly Asn Phe Ile Gly Ala Leu Glu Thr Thr Gly Val Val Leu
            660             665                 670

Leu Leu Glu Tyr Ile Pro Glu Ile Thr Leu Pro Val Ile Ala Ala Leu
            675             680                 685

Ser Ile Ala Glu Ser Ser Thr Gln Lys Glu Lys Ile Ile Lys Thr Ile
            690             695                 700

Asp Asn Phe Leu Glu Lys Arg Tyr Glu Lys Trp Ile Glu Val Tyr Lys
705                 710             715                 720

Leu Val Lys Ala Lys Trp Leu Gly Thr Val Asn Thr Gln Phe Gln Lys
                725             730                 735

Arg Ser Tyr Gln Met Tyr Arg Ser Leu Glu Tyr Gln Val Asp Ala Ile
            740             745                 750

Lys Lys Ile Ile Asp Tyr Glu Tyr Lys Ile Tyr Ser Gly Pro Asp Lys
            755             760                 765

Glu Gln Ile Ala Asp Glu Ile Asn Asn Leu Lys Asn Lys Leu Glu Glu
            770             775                 780

Lys Ala Asn Lys Ala Met Ile Asn Ile Asn Ile Phe Met Arg Glu Ser
785                 790             795                 800

Ser Arg Ser Phe Leu Val Asn Gln Met Ile Asn Glu Ala Lys Lys Gln
                805             810                 815

Leu Leu Glu Phe Asp Thr Gln Ser Lys Asn Ile Leu Met Gln Tyr Ile
            820             825                 830

Lys Ala Asn Ser Lys Phe Ile Gly Ile Thr Glu Leu Lys Lys Leu Glu
            835             840                 845

Ser Lys Ile Asn Lys Val Phe Ser Thr Pro Ile Pro Phe Ser Tyr Ser
850                 855             860
```

```
Lys Asn Leu Asp Cys Trp Val Asp Asn Glu Glu Asp Ile Asp Val Ile
865             870         875             880

Leu Lys Lys Ser Thr Ile Leu Asn Leu Asp Ile Asn Asn Asp Ile Ile
            885             890             895

Ser Asp Ile Ser Gly Phe Asn Ser Ser Val Ile Thr Tyr Pro Asp Ala
        900             905             910

Gln Leu Val Pro Gly Ile Asn Gly Lys Ala Ile His Leu Val Asn Asn
        915             920             925

Glu Ser Ser Glu Val Ile Val His Lys Ala Met Asp Ile Glu Tyr Asn
    930             935             940

Asp Met Phe Asn Asn Phe Thr Val Ser Phe Trp Leu Arg Val Pro Lys
945             950             955             960

Val Ser Ala Ser His Leu Glu Gln Tyr Gly Thr Asn Glu Tyr Ser Ile
            965             970             975

Ile Ser Ser Met Lys Lys His Ser Leu Ser Ile Gly Ser Gly Trp Ser
        980             985             990

Val Ser Leu Lys Gly Asn Asn Leu  Ile Trp Thr Leu Lys  Asp Ser Ala
        995             1000            1005

Gly Glu  Val Arg Gln Ile Thr  Phe Arg Asp Leu Pro  Asp Lys Phe
    1010            1015            1020

Asn Ala  Tyr Leu Ala Asn Lys  Trp Val Phe Ile Thr  Ile Thr Asn
    1025            1030            1035

Asp Arg  Leu Ser Ser Ala Asn  Leu Tyr Ile Asn Gly  Val Leu Met
    1040            1045            1050

Gly Ser  Ala Glu Ile Thr Gly  Leu Gly Ala Ile Arg  Glu Asp Asn
    1055            1060            1065

Asn Ile  Thr Leu Lys Leu Asp  Arg Cys Asn Asn Asn  Asn Gln Tyr
    1070            1075            1080

Val Ser  Ile Asp Lys Phe Arg  Ile Phe Cys Lys Ala  Leu Asn Pro
    1085            1090            1095

Lys Glu  Ile Glu Lys Leu Tyr  Thr Ser Tyr Leu Ser  Ile Thr Phe
    1100            1105            1110
```

60

```
Leu Arg  Asp Phe Trp Gly Asn  Pro Leu Arg Tyr Asp  Thr Glu Tyr
    1115            1120             1125

Tyr Leu  Ile Pro Val Ala Ser  Ser Ser Lys Asp Val  Gln Leu Lys
    1130            1135             1140

Asn Ile  Thr Asp Tyr Met Tyr  Leu Thr Asn Ala Pro  Ser Tyr Thr
    1145            1150             1155

Asn Gly  Lys Leu Asn Ile Tyr  Tyr Arg Arg Leu Tyr  Asn Gly Leu
    1160            1165             1170

Lys Phe  Ile Ile Lys Arg Tyr  Thr Pro Asn Asn Glu  Ile Asp Ser
    1175            1180             1185

Phe Val  Lys Ser Gly Asp Phe  Ile Lys Leu Tyr Val  Ser Tyr Asn
    1190            1195             1200

Asn Asn  Glu His Ile Val Gly  Tyr Pro Lys Asp Gly  Asn Ala Phe
    1205            1210             1215

Asn Asn  Leu Asp Arg Ile Leu  Arg Val Gly Tyr Asn  Ala Pro Gly
    1220            1225             1230

Ile Pro  Leu Tyr Lys Lys Met  Glu Ala Val Lys Leu  Arg Asp Leu
    1235            1240             1245

Lys Thr  Tyr Ser Val Gln Leu  Lys Leu Tyr Asp Asp  Lys Asn Ala
    1250            1255             1260

Ser Leu  Gly Leu Val Gly Thr  His Asn Gly Gln Ile  Gly Asn Asp
    1265            1270             1275

Pro Asn  Arg Asp Ile Leu Ile  Ala Ser Asn Trp Tyr  Phe Asn His
    1280            1285             1290

Leu Lys  Asp Lys Ile Leu Gly  Cys Asp Trp Tyr Phe  Val Pro Thr
    1295            1300             1305

Asp Glu  Gly Trp Thr Asn Asp
    1310            1315
```

```
<210>  4
<211>  535
<212>  PRT
```

```
<213>  Corynebacterium diphtheriae

<400>  4

Gly Ala Asp Asp Val Val Asp Ser Ser Lys Ser Phe Val Met Glu Asn
1               5               10              15

Phe Ser Ser Tyr His Gly Thr Lys Pro Gly Tyr Val Asp Ser Ile Gln
            20              25              30

Lys Gly Ile Gln Lys Pro Lys Ser Gly Thr Gln Gly Asn Tyr Asp Asp
            35              40              45

Asp Trp Lys Gly Phe Tyr Ser Thr Asp Asn Lys Tyr Asp Ala Ala Gly
        50              55              60

Tyr Ser Val Asp Asn Glu Asn Pro Leu Ser Gly Lys Ala Gly Gly Val
65              70              75              80

Val Lys Val Thr Tyr Pro Gly Leu Thr Lys Val Leu Ala Leu Lys Val
            85              90              95

Asp Asn Ala Glu Thr Ile Lys Lys Glu Leu Gly Leu Ser Leu Thr Glu
            100             105             110

Pro Leu Met Glu Gln Val Gly Thr Glu Glu Phe Ile Lys Arg Phe Gly
        115             120             125

Asp Gly Ala Ser Arg Val Val Leu Ser Leu Pro Phe Ala Glu Gly Ser
    130             135             140

Ser Ser Val Glu Tyr Ile Asn Asn Trp Glu Gln Ala Lys Ala Leu Ser
145             150             155             160

Val Glu Leu Glu Ile Asn Phe Glu Thr Arg Gly Lys Arg Gly Gln Asp
            165             170             175

Ala Met Tyr Glu Tyr Met Ala Gln Ala Cys Ala Gly Asn Arg Val Arg
        180             185             190

Arg Ser Val Gly Ser Ser Leu Ser Cys Ile Asn Leu Asp Trp Asp Val
    195             200             205

Ile Arg Asp Lys Thr Lys Thr Lys Ile Glu Ser Leu Lys Glu His Gly
    210             215             220

Pro Ile Lys Asn Lys Met Ser Glu Ser Pro Asn Lys Thr Val Ser Glu
225             230             235             240
```

62

```
Glu Lys Ala Lys Gln Tyr Leu Glu Glu Phe His Gln Thr Ala Leu Glu
            245                 250             255

His Pro Glu Leu Ser Glu Leu Lys Thr Val Thr Gly Thr Asn Pro Val
            260                 265             270

Phe Ala Gly Ala Asn Tyr Ala Ala Trp Ala Val Asn Val Ala Gln Val
            275                 280             285

Ile Asp Ser Glu Thr Ala Asp Asn Leu Glu Lys Thr Thr Ala Ala Leu
            290                 295             300

Ser Ile Leu Pro Gly Ile Gly Ser Val Met Gly Ile Ala Asp Gly Ala
305                 310             315                 320

Val His His Asn Thr Glu Glu Ile Val Ala Gln Ser Ile Ala Leu Ser
            325                 330             335

Ser Leu Met Val Ala Gln Ala Ile Pro Leu Val Gly Glu Leu Val Asp
            340                 345             350

Ile Gly Phe Ala Ala Tyr Asn Phe Val Glu Ser Ile Ile Asn Leu Phe
            355                 360             365

Gln Val Val His Asn Ser Tyr Asn Arg Pro Ala Tyr Ser Pro Gly His
            370                 375             380

Lys Thr Gln Pro Phe Leu His Asp Gly Tyr Ala Val Ser Trp Asn Thr
385                 390                 395                 400

Val Glu Asp Ser Ile Ile Arg Thr Gly Phe Gln Gly Glu Ser Gly His
            405                 410             415

Asp Ile Lys Ile Thr Ala Glu Asn Thr Pro Leu Pro Ile Ala Gly Val
            420                 425             430

Leu Leu Pro Thr Ile Pro Gly Lys Leu Asp Val Asn Lys Ser Lys Thr
            435                 440             445

His Ile Ser Val Asn Gly Arg Lys Ile Arg Met Arg Cys Arg Ala Ile
            450                 455             460

Asp Gly Asp Val Thr Phe Cys Arg Pro Lys Ser Pro Val Tyr Val Gly
465                 470                 475                 480

Asn Gly Val His Ala Asn Leu His Val Ala Phe His Arg Ser Ser Ser
            485                 490             495
```

```
Glu Lys Ile His Ser Asn Glu Ile Ser Ser Asp Ser Ile Gly Val Leu
        500                 505             510

Gly Tyr Gln Lys Thr Val Asp His Thr Lys Val Asn Ser Lys Leu Ser
        515                 520             525

Leu Phe Phe Glu Ile Lys Ser
    530                 535
```

```
<210>  5
<211>  535
<212>  PRT
<213>  Artificial Sequence

<220>
<223>  CRM197, non-toxic mutant of diphtheria toxin

<400>  5
```

```
Gly Ala Asp Asp Val Val Asp Ser Ser Lys Ser Phe Val Met Glu Asn
1               5                 10                  15

Phe Ser Ser Tyr His Gly Thr Lys Pro Gly Tyr Val Asp Ser Ile Gln
        20                  25                  30

Lys Gly Ile Gln Lys Pro Lys Ser Gly Thr Gln Gly Asn Tyr Asp Asp
        35                  40                  45

Asp Trp Lys Glu Phe Tyr Ser Thr Asp Asn Lys Tyr Asp Ala Ala Gly
    50                  55                  60

Tyr Ser Val Asp Asn Glu Asn Pro Leu Ser Gly Lys Ala Gly Gly Val
65                  70                  75                  80

Val Lys Val Thr Tyr Pro Gly Leu Thr Lys Val Leu Ala Leu Lys Val
            85                  90                  95

Asp Asn Ala Glu Thr Ile Lys Lys Glu Leu Gly Leu Ser Leu Thr Glu
            100                 105                 110

Pro Leu Met Glu Gln Val Gly Thr Glu Glu Phe Ile Lys Arg Phe Gly
        115                 120                 125

Asp Gly Ala Ser Arg Val Val Leu Ser Leu Pro Phe Ala Glu Gly Ser
        130                 135                 140

Ser Ser Val Glu Tyr Ile Asn Asn Trp Glu Gln Ala Lys Ala Leu Ser
145                 150                 155                 160
```

Val Glu Leu Glu Ile Asn Phe Glu Thr Arg Gly Lys Arg Gly Gln Asp
165 170 175

Ala Met Tyr Glu Tyr Met Ala Gln Ala Cys Ala Gly Asn Arg Val Arg
180 185 190

Arg Ser Val Gly Ser Ser Leu Ser Cys Ile Asn Leu Asp Trp Asp Val
195 200 205

Ile Arg Asp Lys Thr Lys Thr Lys Ile Glu Ser Leu Lys Glu His Gly
210 215 220

Pro Ile Lys Asn Lys Met Ser Glu Ser Pro Asn Lys Thr Val Ser Glu
225 230 235 240

Glu Lys Ala Lys Gln Tyr Leu Glu Glu Phe His Gln Thr Ala Leu Glu
245 250 255

His Pro Glu Leu Ser Glu Leu Lys Thr Val Thr Gly Thr Asn Pro Val
260 265 270

Phe Ala Gly Ala Asn Tyr Ala Ala Trp Ala Val Asn Val Ala Gln Val
275 280 285

Ile Asp Ser Glu Thr Ala Asp Asn Leu Glu Lys Thr Thr Ala Ala Leu
290 295 300

Ser Ile Leu Pro Gly Ile Gly Ser Val Met Gly Ile Ala Asp Gly Ala
305 310 315 320

Val His His Asn Thr Glu Glu Ile Val Ala Gln Ser Ile Ala Leu Ser
325 330 335

Ser Leu Met Val Ala Gln Ala Ile Pro Leu Val Gly Glu Leu Val Asp
340 345 350

Ile Gly Phe Ala Ala Tyr Asn Phe Val Glu Ser Ile Ile Asn Leu Phe
355 360 365

Gln Val Val His Asn Ser Tyr Asn Arg Pro Ala Tyr Ser Pro Gly His
370 375 380

Lys Thr Gln Pro Phe Leu His Asp Gly Tyr Ala Val Ser Trp Asn Thr
385 390 395 400

Val Glu Asp Ser Ile Ile Arg Thr Gly Phe Gln Gly Glu Ser Gly His
405 410 415

Asp Ile Lys Ile Thr Ala Glu Asn Thr Pro Leu Pro Ile Ala Gly Val
420 425 430

Leu Leu Pro Thr Ile Pro Gly Lys Leu Asp Val Asn Lys Ser Lys Thr
435 440 445

His Ile Ser Val Asn Gly Arg Lys Ile Arg Met Arg Cys Arg Ala Ile
450 455 460

Asp Gly Asp Val Thr Phe Cys Arg Pro Lys Ser Pro Val Tyr Val Gly
465 470 475 480

Asn Gly Val His Ala Asn Leu His Val Ala Phe His Arg Ser Ser Ser
485 490 495

Glu Lys Ile His Ser Asn Glu Ile Ser Ser Asp Ser Ile Gly Val Leu
500 505 510

Gly Tyr Gln Lys Thr Val Asp His Thr Lys Val Asn Ser Lys Leu Ser
515 520 525

Leu Phe Phe Glu Ile Lys Ser
530 535

<210> 6
<211> 162
<212> PRT
<213> Pseudomonas aeruginosa

<400> 6

Met Ala Val Asp Met Phe Ile Lys Ile Gly Asp Val Lys Gly Glu Ser
1 5 10 15

Lys Asp Lys Thr His Ala Glu Glu Ile Asp Val Leu Ala Trp Ser Trp
20 25 30

Gly Met Ser Gln Ser Gly Ser Met His Met Gly Gly Gly Gly Gly Ala
35 40 45

Gly Lys Val Asn Val Gln Asp Leu Ser Phe Thr Lys Tyr Ile Asp Lys
50 55 60

Ser Thr Pro Asn Leu Met Met Ala Cys Ser Ser Gly Lys His Tyr Pro
65 70 75 80

Gln Ala Lys Leu Thr Ile Arg Lys Ala Gly Gly Glu Asn Gln Val Glu
85 90 95

```
Tyr Leu Ile Ile Thr Leu Lys Glu Val Leu Val Ser Ser Val Ser Thr
        100             105             110

Gly Gly Ser Gly Gly Glu Asp Arg Leu Thr Glu Asn Val Thr Leu Asn
        115             120             125

Phe Ala Gln Val Gln Val Asp Tyr Gln Pro Gln Lys Ala Asp Gly Ala
        130             135             140

Lys Asp Gly Gly Pro Ile Lys Tyr Gly Trp Asn Ile Arg Gln Asn Val
145             150             155             160

Gln Ala
```

```
<210>  7
<211>  221
<212>  PRT
<213>  Escherichia coli

<400>  7
```

```
Gly Ile Phe Ser Arg Phe Ala Asp Ile Val Asn Ala Asn Ile Asn Ala
1               5               10              15

Leu Leu Glu Lys Ala Glu Asp Pro Gln Lys Leu Val Arg Leu Met Ile
        20              25              30

Gln Glu Met Glu Asp Thr Leu Val Glu Val Arg Ser Thr Ser Ala Arg
        35              40              45

Ala Leu Ala Glu Lys Lys Gln Leu Thr Arg Arg Ile Glu Gln Ala Ser
        50              55              60

Ala Arg Glu Val Glu Trp Gln Glu Lys Ala Glu Leu Ala Leu Leu Lys
65              70              75              80

Glu Arg Glu Asp Leu Ala Arg Ala Ala Leu Ile Glu Lys Gln Lys Leu
                85              90              95

Thr Asp Leu Ile Lys Ser Leu Glu His Glu Val Thr Leu Val Asp Asp
        100             105             110

Thr Leu Ala Arg Met Lys Lys Glu Ile Gly Glu Leu Glu Asn Lys Leu
        115             120             125

Ser Glu Thr Arg Ala Arg Gln Gln Ala Leu Met Leu Arg His Gln Ala
        130             135             140
```

```
Ala Asn Ser Ser Arg Asp Val Arg Arg Gln Leu Asp Ser Gly Lys Leu
145                 150             155             160

Asp Glu Ala Met Ala Arg Phe Glu Ser Phe Glu Arg Arg Ile Asp Gln
                165             170             175

Met Glu Ala Glu Ala Glu Ser His Ser Phe Gly Lys Gln Lys Ser Leu
            180             185             190

Asp Asp Gln Phe Ala Glu Leu Lys Ala Asp Asp Ala Ile Ser Glu Gln
        195             200             205

Leu Ala Gln Leu Lys Ala Lys Met Lys Gln Asp Asn Gln
    210             215             220
```

```
<210>  8
<211>  396
<212>  PRT
<213>  Escherichia coli

<400>  8
```

```
Met Lys Ile Lys Thr Gly Ala Arg Ile Leu Ala Leu Ser Ala Leu Thr
1               5               10              15

Thr Met Met Phe Ser Ala Ser Ala Leu Ala Lys Ile Glu Glu Gly Lys
            20              25              30

Leu Val Ile Trp Ile Asn Gly Asp Lys Gly Tyr Asn Gly Leu Ala Glu
            35              40              45

Val Gly Lys Lys Phe Glu Lys Asp Thr Gly Ile Lys Val Thr Val Glu
        50              55              60

His Pro Asp Lys Leu Glu Glu Lys Phe Pro Gln Val Ala Ala Thr Gly
65              70              75              80

Asp Gly Pro Asp Ile Ile Phe Trp Ala His Asp Arg Phe Gly Gly Tyr
            85              90              95

Ala Gln Ser Gly Leu Leu Ala Glu Ile Thr Pro Asp Lys Ala Phe Gln
            100             105             110

Asp Lys Leu Tyr Pro Phe Thr Trp Asp Ala Val Arg Tyr Asn Gly Lys
        115             120             125

Leu Ile Ala Tyr Pro Ile Ala Val Glu Ala Leu Ser Leu Ile Tyr Asn
    130             135             140
```

Lys Asp Leu Leu Pro Asn Pro Pro Lys Thr Trp Glu Glu Ile Pro Ala
145            150            155            160

Leu Asp Lys Glu Leu Lys Ala Lys Gly Lys Ser Ala Leu Met Phe Asn
            165            170            175

Leu Gln Glu Pro Tyr Phe Thr Trp Pro Leu Ile Ala Ala Asp Gly Gly
            180            185            190

Tyr Ala Phe Lys Tyr Glu Asn Gly Lys Tyr Asp Ile Lys Asp Val Gly
            195            200            205

Val Asp Asn Ala Gly Ala Lys Ala Gly Leu Thr Phe Leu Val Asp Leu
            210            215            220

Ile Lys Asn Lys His Met Asn Ala Asp Thr Asp Tyr Ser Ile Ala Glu
225            230            235            240

Ala Ala Phe Asn Lys Gly Glu Thr Ala Met Thr Ile Asn Gly Pro Trp
            245            250            255

Ala Trp Ser Asn Ile Asp Thr Ser Lys Val Asn Tyr Gly Val Thr Val
            260            265            270

Leu Pro Thr Phe Lys Gly Gln Pro Ser Lys Pro Phe Val Gly Val Leu
            275            280            285

Ser Ala Gly Ile Asn Ala Ala Ser Pro Asn Lys Glu Leu Ala Lys Glu
            290            295            300

Phe Leu Glu Asn Tyr Leu Leu Thr Asp Glu Gly Leu Glu Ala Val Asn
305            310            315            320

Lys Asp Lys Pro Leu Gly Ala Val Ala Leu Lys Ser Tyr Glu Glu Glu
            325            330            335

Leu Ala Lys Asp Pro Arg Ile Ala Ala Thr Met Glu Asn Ala Gln Lys
            340            345            350

Gly Glu Ile Met Pro Asn Ile Pro Gln Met Ser Ala Phe Trp Tyr Ala
            355            360            365

Val Arg Thr Ala Val Ile Asn Ala Ala Ser Gly Arg Gln Thr Val Asp
            370            375            380

Glu Ala Leu Lys Asp Ala Gln Thr Arg Ile Thr Lys

385                    390                    395


<210>   9
<211>   445
<212>   PRT
<213>   Neisseria gonorrhoeae

<400>   9

Met Ile Pro Gln Tyr Glu Gln Pro Lys Val Glu Val Ala Glu Thr Phe
1               5                   10                  15


Gln Asn Asp Thr Ser Val Ser Ser Ile Arg Ala Val Asp Leu Gly Trp
                20                  25                  30


His Asp Tyr Phe Ala Asp Pro Arg Leu Gln Lys Leu Ile Asp Ile Ala
            35                  40                  45


Leu Glu Arg Asn Thr Ser Leu Arg Thr Ala Val Leu Asn Ser Glu Ile
        50                  55                  60


Tyr Arg Lys Gln Tyr Met Ile Glu Arg Asn Asn Leu Leu Pro Thr Leu
65                  70                  75                  80


Ala Ala Asn Ala Asn Gly Ser Arg Gln Gly Ser Leu Ser Gly Gly Asn
                85                  90                  95


Val Ser Ser Ser Tyr Asn Val Gly Leu Gly Ala Ala Ser Tyr Glu Leu
            100                 105                 110


Asp Leu Phe Gly Arg Val Arg Ser Ser Ser Glu Ala Ala Leu Gln Gly
            115                 120                 125


Tyr Phe Ala Ser Val Ala Asn Arg Asp Ala Ala His Leu Ser Leu Ile
            130                 135                 140


Ala Thr Val Ala Lys Ala Tyr Phe Asn Glu Arg Tyr Ala Glu Glu Ala
145                 150                 155                 160


Met Ser Leu Ala Gln Arg Val Leu Lys Thr Arg Glu Glu Thr Tyr Asn
                165                 170                 175


Ala Val Arg Ile Ala Val Gln Gly Arg Arg Asp Phe Arg Arg Arg Pro
            180                 185                 190


Ala Pro Ala Glu Ala Leu Ile Glu Ser Ala Lys Ala Asp Tyr Ala His
            195                 200                 205


Ala Ala Arg Ser Arg Glu Gln Ala Arg Asn Ala Leu Ala Thr Leu Ile

EP 3 770 269 A1

210 215 220

Asn Arg Pro Ile Pro Glu Asp Leu Pro Ala Gly Leu Pro Leu Asp Lys
225 230 235 240

Gln Phe Phe Val Glu Lys Leu Pro Ala Gly Leu Ser Ser Glu Val Leu
245 250 255

Leu Asp Arg Pro Asp Ile Arg Ala Ala Glu His Ala Leu Lys Gln Ala
260 265 270

Asn Ala Asn Ile Gly Ala Ala Arg Ala Ala Phe Phe Pro Ser Ile Arg
275 280 285

Leu Thr Gly Ser Val Gly Thr Gly Ser Val Glu Leu Gly Gly Leu Phe
290 295 300

Lys Ser Gly Thr Gly Val Trp Ala Phe Ala Pro Ser Ile Thr Leu Pro
305 310 315 320

Ile Phe Thr Trp Gly Thr Asn Lys Ala Asn Leu Asp Val Ala Lys Leu
325 330 335

Arg Gln Gln Ala Gln Ile Val Ala Tyr Glu Ser Ala Val Gln Ser Ala
340 345 350

Phe Gln Asp Val Ala Asn Ala Leu Ala Ala Arg Glu Gln Leu Asp Lys
355 360 365

Ala Tyr Asp Ala Leu Ser Lys Gln Ser Arg Ala Ser Lys Glu Ala Leu
370 375 380

Arg Leu Val Gly Leu Arg Tyr Lys His Gly Val Ser Gly Ala Leu Asp
385 390 395 400

Leu Leu Asp Ala Glu Arg Ser Ser Tyr Ser Ala Glu Gly Ala Ala Leu
405 410 415

Ser Ala Gln Leu Thr Arg Ala Glu Asn Leu Ala Asp Leu Tyr Lys Ala
420 425 430

Leu Gly Gly Gly Leu Lys Arg Asp Thr Gln Thr Gly Lys
435 440 445

<210> 10
<211> 522
<212> PRT
<213> Staphylococcus aureus

71

<400> 10

Ala Ser Glu Asn Ser Val Thr Gln Ser Asp Ser Ala Ser Asn Glu Ser
1               5                   10                  15

Lys Ser Asn Asp Ser Ser Ser Val Ser Ala Ala Pro Lys Thr Asp Asp
            20                  25                  30

Thr Asn Val Ser Asp Thr Lys Thr Ser Ser Asn Thr Asn Asn Gly Glu
            35                  40                  45

Thr Ser Val Ala Gln Asn Pro Ala Gln Gln Glu Thr Thr Gln Ser Ser
        50                  55                  60

Ser Thr Asn Ala Thr Thr Glu Glu Thr Pro Val Thr Gly Glu Ala Thr
65                  70                  75                  80

Thr Thr Thr Thr Asn Gln Ala Asn Thr Pro Ala Thr Thr Gln Ser Ser
                85                  90                  95

Asn Thr Asn Ala Glu Glu Leu Val Asn Gln Thr Ser Asn Glu Thr Thr
            100                 105                 110

Phe Asn Asp Thr Asn Thr Val Ser Ser Val Asn Ser Pro Gln Asn Ser
            115                 120                 125

Thr Asn Ala Glu Asn Val Ser Thr Thr Gln Asp Thr Ser Thr Glu Ala
    130                 135                 140

Thr Pro Ser Asn Asn Glu Ser Ala Pro Gln Ser Thr Asp Ala Ser Asn
145                 150                 155                 160

Lys Asp Val Val Asn Gln Ala Val Asn Thr Ser Ala Pro Arg Met Arg
                165                 170                 175

Ala Phe Ser Leu Ala Ala Val Ala Ala Asp Ala Pro Ala Ala Gly Thr
            180                 185                 190

Asp Ile Thr Asn Gln Leu Thr Asn Val Thr Val Gly Ile Asp Ser Gly
            195                 200                 205

Thr Thr Val Tyr Pro His Gln Ala Gly Tyr Val Lys Leu Asn Tyr Gly
    210                 215                 220

Phe Ser Val Pro Asn Ser Ala Val Lys Gly Asp Thr Phe Lys Ile Thr
225                 230                 235                 240

72

```
Val Pro Lys Glu Leu Asn Leu Asn Gly Val Thr Ser Thr Ala Lys Val
                245                 250                 255

Pro Pro Ile Met Ala Gly Asp Gln Val Leu Ala Asn Gly Val Ile Asp
                260                 265                 270

Ser Asp Gly Asn Val Ile Tyr Thr Phe Thr Asp Tyr Val Asn Thr Lys
                275                 280                 285

Asp Asp Val Lys Ala Thr Leu Thr Met Pro Ala Tyr Ile Asp Pro Glu
                290                 295                 300

Asn Val Lys Lys Thr Gly Asn Val Thr Leu Ala Thr Gly Ile Gly Ser
305                 310                 315                 320

Thr Thr Ala Asn Lys Thr Val Leu Val Asp Tyr Glu Lys Tyr Gly Lys
                325                 330                 335

Phe Tyr Asn Leu Ser Ile Lys Gly Thr Ile Asp Gln Ile Asp Lys Thr
                340                 345                 350

Asn Asn Thr Tyr Arg Gln Thr Ile Tyr Val Asn Pro Ser Gly Asp Asn
                355                 360                 365

Val Ile Ala Pro Val Leu Thr Gly Asn Leu Lys Pro Asn Thr Asp Ser
                370                 375                 380

Asn Ala Leu Ile Asp Gln Gln Asn Thr Ser Ile Lys Val Tyr Lys Val
385                 390                 395                 400

Asp Asn Ala Ala Asp Leu Ser Glu Ser Tyr Phe Val Asn Pro Glu Asn
                405                 410                 415

Phe Glu Asp Val Thr Asn Ser Val Asn Ile Thr Phe Pro Asn Pro Asn
                420                 425                 430

Gln Tyr Lys Val Glu Phe Asn Thr Pro Asp Asp Gln Ile Thr Thr Pro
                435                 440                 445

Tyr Ile Val Val Val Asn Gly His Ile Asp Pro Asn Ser Lys Gly Asp
    450                 455                 460

Leu Ala Leu Arg Ser Thr Leu Tyr Gly Tyr Asn Ser Asn Ile Ile Trp
465                 470                 475                 480

Arg Ser Met Ser Trp Asp Asn Glu Val Ala Phe Asn Asn Gly Ser Gly
                485                 490                 495
```

```
Ser Gly Asp Gly Ile Asp Lys Pro Val Val Pro Glu Gln Pro Asp Glu
        500             505         510

Pro Gly Glu Ile Glu Pro Ile Pro Glu Asp
        515             520


<210>  11
<211>  340
<212>  PRT
<213>  Staphylococcus aureus

<400>  11

Val Ala Ala Asp Ala Pro Ala Ala Gly Thr Asp Ile Thr Asn Gln Leu
1               5               10              15

Thr Asn Val Thr Val Gly Ile Asp Ser Gly Thr Thr Val Tyr Pro His
        20              25              30

Gln Ala Gly Tyr Val Lys Leu Asn Tyr Gly Phe Ser Val Pro Asn Ser
        35              40              45

Ala Val Lys Gly Asp Thr Phe Lys Ile Thr Val Pro Lys Glu Leu Asn
    50              55              60

Leu Asn Gly Val Thr Ser Thr Ala Lys Val Pro Pro Ile Met Ala Gly
65              70              75              80

Asp Gln Val Leu Ala Asn Gly Val Ile Asp Ser Asp Gly Asn Val Ile
            85              90              95

Tyr Thr Phe Thr Asp Tyr Val Asn Thr Lys Asp Asp Val Lys Ala Thr
        100             105             110

Leu Thr Met Pro Ala Tyr Ile Asp Pro Glu Asn Val Lys Lys Thr Gly
        115             120             125

Asn Val Thr Leu Ala Thr Gly Ile Gly Ser Thr Thr Ala Asn Lys Thr
    130             135             140

Val Leu Val Asp Tyr Glu Lys Tyr Gly Lys Phe Tyr Asn Leu Ser Ile
145             150             155             160

Lys Gly Thr Ile Asp Gln Ile Asp Lys Thr Asn Asn Thr Tyr Arg Gln
            165             170             175

Thr Ile Tyr Val Asn Pro Ser Gly Asp Asn Val Ile Ala Pro Val Leu
        180             185             190
```

```
Thr Gly Asn Leu Lys Pro Asn Thr Asp Ser Asn Ala Leu Ile Asp Gln
        195                 200                 205

Gln Asn Thr Ser Ile Lys Val Tyr Lys Val Asp Asn Ala Ala Asp Leu
        210                 215                 220

Ser Glu Ser Tyr Phe Val Asn Pro Glu Asn Phe Glu Asp Val Thr Asn
225                 230                 235                 240

Ser Val Asn Ile Thr Phe Pro Asn Pro Asn Gln Tyr Lys Val Glu Phe
                245                 250                 255

Asn Thr Pro Asp Asp Gln Ile Thr Thr Pro Tyr Ile Val Val Val Asn
                260                 265                 270

Gly His Ile Asp Pro Asn Ser Lys Gly Asp Leu Ala Leu Arg Ser Thr
        275                 280                 285

Leu Tyr Gly Tyr Asn Ser Asn Ile Ile Trp Arg Ser Met Ser Trp Asp
        290                 295                 300

Asn Glu Val Ala Phe Asn Asn Gly Ser Gly Ser Gly Asp Gly Ile Asp
305                 310                 315                 320

Lys Pro Val Val Pro Glu Gln Pro Asp Glu Pro Gly Glu Ile Glu Pro
                325                 330                 335

Ile Pro Glu Asp
                340


<210>  12
<211>  340
<212>  PRT
<213>  Artificial Sequence

<220>
<223>  ClfAN2N3P116S/Y118A

<400>  12

Val Ala Ala Asp Ala Pro Ala Ala Gly Thr Asp Ile Thr Asn Gln Leu
1                   5                   10                  15

Thr Asn Val Thr Val Gly Ile Asp Ser Gly Thr Thr Val Tyr Pro His
                20                  25                  30

Gln Ala Gly Tyr Val Lys Leu Asn Tyr Gly Phe Ser Val Pro Asn Ser
        35                  40                  45
```

Ala Val Lys Gly Asp Thr Phe Lys Ile Thr Val Pro Lys Glu Leu Asn
        50              55              60

Leu Asn Gly Val Thr Ser Thr Ala Lys Val Pro Pro Ile Met Ala Gly
        65              70              75              80

Asp Gln Val Leu Ala Asn Gly Val Ile Asp Ser Asp Gly Asn Val Ile
                85              90              95

Tyr Thr Phe Thr Asp Tyr Val Asn Thr Lys Asp Asp Val Lys Ala Thr
            100             105             110

Leu Thr Met Ser Ala Ala Ile Asp Pro Glu Asn Val Lys Lys Thr Gly
            115             120             125

Asn Val Thr Leu Ala Thr Gly Ile Gly Ser Thr Thr Ala Asn Lys Thr
        130             135             140

Val Leu Val Asp Tyr Glu Lys Tyr Gly Lys Phe Tyr Asn Leu Ser Ile
145             150             155             160

Lys Gly Thr Ile Asp Gln Ile Asp Lys Thr Asn Asn Thr Tyr Arg Gln
            165             170             175

Thr Ile Tyr Val Asn Pro Ser Gly Asp Asn Val Ile Ala Pro Val Leu
            180             185             190

Thr Gly Asn Leu Lys Pro Asn Thr Asp Ser Asn Ala Leu Ile Asp Gln
        195             200             205

Gln Asn Thr Ser Ile Lys Val Tyr Lys Val Asp Asn Ala Ala Asp Leu
        210             215             220

Ser Glu Ser Tyr Phe Val Asn Pro Glu Asn Phe Glu Asp Val Thr Asn
225             230             235             240

Ser Val Asn Ile Thr Phe Pro Asn Pro Asn Gln Tyr Lys Val Glu Phe
            245             250             255

Asn Thr Pro Asp Asp Gln Ile Thr Thr Pro Tyr Ile Val Val Val Asn
            260             265             270

Gly His Ile Asp Pro Asn Ser Lys Gly Asp Leu Ala Leu Arg Ser Thr
        275             280             285

Leu Tyr Gly Tyr Asn Ser Asn Ile Ile Trp Arg Ser Met Ser Trp Asp
        290             295             300

76

```
Asn Glu Val Ala Phe Asn Asn Gly Ser Gly Ser Gly Asp Gly Ile Asp
305             310             315             320


Lys Pro Val Val Pro Glu Gln Pro Asp Glu Pro Gly Glu Ile Glu Pro
                325             330             335


Ile Pro Glu Asp
            340



<210>  13
<211>  293
<212>  PRT
<213>  Staphylococcus aureus

<400>  13

Ala Asp Ser Asp Ile Asn Ile Lys Thr Gly Thr Thr Asp Ile Gly Ser
1               5               10              15


Asn Thr Thr Val Lys Thr Gly Asp Leu Val Thr Tyr Asp Lys Glu Asn
            20              25              30


Gly Met His Lys Lys Val Phe Tyr Ser Phe Ile Asp Asp Lys Asn His
        35              40              45


Asn Lys Lys Leu Leu Val Ile Arg Thr Lys Gly Thr Ile Ala Gly Gln
    50              55              60


Tyr Arg Val Tyr Ser Glu Glu Gly Ala Asn Lys Ser Gly Leu Ala Trp
65              70              75              80


Pro Ser Ala Phe Lys Val Gln Leu Gln Leu Pro Asp Asn Glu Val Ala
            85              90              95


Gln Ile Ser Asp Tyr Tyr Pro Arg Asn Ser Ile Asp Thr Lys Glu Tyr
            100             105             110


Met Ser Thr Leu Thr Tyr Gly Phe Asn Gly Asn Val Thr Gly Asp Asp
        115             120             125


Thr Gly Lys Ile Gly Gly Leu Ile Gly Ala Asn Val Ser Ile Gly His
    130             135             140


Thr Leu Lys Tyr Val Gln Pro Asp Phe Lys Thr Ile Leu Glu Ser Pro
145             150             155             160


Thr Asp Lys Lys Val Gly Trp Lys Val Ile Phe Asn Asn Met Val Asn
                165             170             175
```

```
Gln Asn Trp Gly Pro Tyr Asp Arg Asp Ser Trp Asn Pro Val Tyr Gly
            180                 185                 190

Asn Gln Leu Phe Met Lys Thr Arg Asn Gly Ser Met Lys Ala Ala Asp
            195                 200                 205

Asn Phe Leu Asp Pro Asn Lys Ala Ser Ser Leu Leu Ser Ser Gly Phe
    210                 215                 220

Ser Pro Asp Phe Ala Thr Val Ile Thr Met Asp Arg Lys Ala Ser Lys
225                 230                 235                 240

Gln Gln Thr Asn Ile Asp Val Ile Tyr Glu Arg Val Arg Asp Asp Tyr
            245                 250                 255

Gln Leu His Trp Thr Ser Thr Asn Trp Lys Gly Thr Asn Thr Lys Asp
            260                 265                 270

Lys Trp Ile Asp Arg Ser Ser Glu Arg Tyr Lys Ile Asp Trp Glu Lys
            275                 280                 285

Glu Glu Met Thr Asn
        290


<210>  14
<211>  293
<212>  PRT
<213>  Artificial Sequence

<220>
<223>  Amino acid sequence of mature HlaH35L

<400>  14

Ala Asp Ser Asp Ile Asn Ile Lys Thr Gly Thr Thr Asp Ile Gly Ser
1               5               10                  15

Asn Thr Thr Val Lys Thr Gly Asp Leu Val Thr Tyr Asp Lys Glu Asn
            20                  25                  30

Gly Met Leu Lys Lys Val Phe Tyr Ser Phe Ile Asp Asp Lys Asn His
            35                  40                  45

Asn Lys Lys Leu Leu Val Ile Arg Thr Lys Gly Thr Ile Ala Gly Gln
    50                  55                  60

Tyr Arg Val Tyr Ser Glu Glu Gly Ala Asn Lys Ser Gly Leu Ala Trp
65                  70                  75                  80
```

```
Pro Ser Ala Phe Lys Val Gln Leu Gln Leu Pro Asp Asn Glu Val Ala
            85                  90                  95

Gln Ile Ser Asp Tyr Tyr Pro Arg Asn Ser Ile Asp Thr Lys Glu Tyr
            100                 105                 110

Met Ser Thr Leu Thr Tyr Gly Phe Asn Gly Asn Val Thr Gly Asp Asp
            115                 120                 125

Thr Gly Lys Ile Gly Gly Leu Ile Gly Ala Asn Val Ser Ile Gly His
    130                 135                 140

Thr Leu Lys Tyr Val Gln Pro Asp Phe Lys Thr Ile Leu Glu Ser Pro
145                 150                 155                 160

Thr Asp Lys Lys Val Gly Trp Lys Val Ile Phe Asn Asn Met Val Asn
            165                 170                 175

Gln Asn Trp Gly Pro Tyr Asp Arg Asp Ser Trp Asn Pro Val Tyr Gly
            180                 185                 190

Asn Gln Leu Phe Met Lys Thr Arg Asn Gly Ser Met Lys Ala Ala Asp
            195                 200                 205

Asn Phe Leu Asp Pro Asn Lys Ala Ser Ser Leu Leu Ser Ser Gly Phe
    210                 215                 220

Ser Pro Asp Phe Ala Thr Val Ile Thr Met Asp Arg Lys Ala Ser Lys
225                 230                 235                 240

Gln Gln Thr Asn Ile Asp Val Ile Tyr Glu Arg Val Arg Asp Asp Tyr
            245                 250                 255

Gln Leu His Trp Thr Ser Thr Asn Trp Lys Gly Thr Asn Thr Lys Asp
            260                 265                 270

Lys Trp Ile Asp Arg Ser Ser Glu Arg Tyr Lys Ile Asp Trp Glu Lys
            275                 280                 285

Glu Glu Met Thr Asn
    290
```

```
<210>  15
<211>  293
<212>  PRT
<213>  Artificial Sequence

<220>
<223>  Amino acid sequence of mature Hla H35L/H48C/G122C
```

<400> 15

```
Ala Asp Ser Asp Ile Asn Ile Lys Thr Gly Thr Thr Asp Ile Gly Ser
1               5                   10              15

Asn Thr Thr Val Lys Thr Gly Asp Leu Val Thr Tyr Asp Lys Glu Asn
            20                  25              30

Gly Met Leu Lys Lys Val Phe Tyr Ser Phe Ile Asp Asp Lys Asn Cys
        35                  40              45

Asn Lys Lys Leu Leu Val Ile Arg Thr Lys Gly Thr Ile Ala Gly Gln
    50                  55                  60

Tyr Arg Val Tyr Ser Glu Glu Gly Ala Asn Lys Ser Gly Leu Ala Trp
65                  70                  75                  80

Pro Ser Ala Phe Lys Val Gln Leu Gln Leu Pro Asp Asn Glu Val Ala
            85                  90                  95

Gln Ile Ser Asp Tyr Tyr Pro Arg Asn Ser Ile Asp Thr Lys Glu Tyr
            100                 105                 110

Met Ser Thr Leu Thr Tyr Gly Phe Asn Cys Asn Val Thr Gly Asp Asp
        115                 120                 125

Thr Gly Lys Ile Gly Gly Leu Ile Gly Ala Asn Val Ser Ile Gly His
    130                 135                 140

Thr Leu Lys Tyr Val Gln Pro Asp Phe Lys Thr Ile Leu Glu Ser Pro
145                 150                 155                 160

Thr Asp Lys Lys Val Gly Trp Lys Val Ile Phe Asn Asn Met Val Asn
            165                 170                 175

Gln Asn Trp Gly Pro Tyr Asp Arg Asp Ser Trp Asn Pro Val Tyr Gly
            180                 185                 190

Asn Gln Leu Phe Met Lys Thr Arg Asn Gly Ser Met Lys Ala Ala Asp
        195                 200                 205

Asn Phe Leu Asp Pro Asn Lys Ala Ser Ser Leu Leu Ser Ser Gly Phe
    210                 215                 220

Ser Pro Asp Phe Ala Thr Val Ile Thr Met Asp Arg Lys Ala Ser Lys
225                 230                 235                 240
```

```
Gln Gln Thr Asn Ile Asp Val Ile Tyr Glu Arg Val Arg Asp Asp Tyr
            245             250             255

Gln Leu His Trp Thr Ser Thr Asn Trp Lys Gly Thr Asn Thr Lys Asp
            260             265             270

Lys Trp Ile Asp Arg Ser Ser Glu Arg Tyr Lys Ile Asp Trp Glu Lys
            275             280             285

Glu Glu Met Thr Asn
            290


<210>  16
<211>  258
<212>  PRT
<213>  Artificial Sequence

<220>
<223>  HlaPSGS

<400>  16

Ala Asp Ser Asp Ile Asn Ile Lys Thr Gly Thr Thr Asp Ile Gly Ser
1               5               10              15

Asn Thr Thr Val Lys Thr Gly Asp Leu Val Thr Tyr Asp Lys Glu Asn
            20              25              30

Gly Met His Lys Lys Val Phe Tyr Ser Phe Ile Asp Asp Lys Asn His
            35              40              45

Asn Lys Lys Leu Leu Val Ile Arg Thr Lys Gly Thr Ile Ala Gly Gln
            50              55              60

Tyr Arg Val Tyr Ser Glu Glu Gly Ala Asn Lys Ser Gly Leu Ala Trp
65              70              75              80

Pro Ser Ala Phe Lys Val Gln Leu Gln Leu Pro Asp Asn Glu Val Ala
            85              90              95

Gln Ile Ser Asp Tyr Tyr Pro Arg Asn Ser Ile Asp Thr Pro Ser Gly
            100             105             110

Ser Val Gln Pro Asp Phe Lys Thr Ile Leu Glu Ser Pro Thr Asp Lys
            115             120             125

Lys Val Gly Trp Lys Val Ile Phe Asn Asn Met Val Asn Gln Asn Trp
            130             135             140

Gly Pro Tyr Asp Arg Asp Ser Trp Asn Pro Val Tyr Gly Asn Gln Leu
```

145                     150                     155                     160


Phe Met Lys Thr Arg Asn Gly Ser Met Lys Ala Ala Asp Asn Phe Leu
                165                     170                     175


Asp Pro Asn Lys Ala Ser Ser Leu Leu Ser Ser Gly Phe Ser Pro Asp
            180                     185                     190


Phe Ala Thr Val Ile Thr Met Asp Arg Lys Ala Ser Lys Gln Gln Thr
        195                     200                     205


Asn Ile Asp Val Ile Tyr Glu Arg Val Arg Asp Asp Tyr Gln Leu His
    210                     215                     220


Trp Thr Ser Thr Asn Trp Lys Gly Thr Asn Thr Lys Asp Lys Trp Ile
225                     230                     235                     240


Asp Arg Ser Ser Glu Arg Tyr Lys Ile Asp Trp Glu Lys Glu Glu Met
                245                     250                     255


Thr Asn


<210> 17
<211> 5
<212> PRT
<213> Artificial Sequence

<220>
<223> consensus sequence


<220>
<221> MISC_FEATURE
<222> (1)..(1)
<223> Xaa can be Asp or Glu

<220>
<221> MISC_FEATURE
<222> (2)..(2)
<223> Xaa is any amino acid apart from proline

<220>
<221> MISC_FEATURE
<222> (4)..(4)
<223> Xaa is any amino acid apart from proline

<220>
<221> MISC_FEATURE
<222> (5)..(5)
<223> Xaa can be Ser or Thr

<400> 17

Xaa Xaa Asn Xaa Xaa

```
                1                    5


                <210>  18
                <211>  7
                <212>  PRT
                <213>  Artificial Sequence

                <220>
                <223>  consensus sequence


                <220>
                <221>  MISC_FEATURE
                <222>  (2)..(2)
                <223>  Xaa can be Asp or Glu

                <220>
                <221>  MISC_FEATURE
                <222>  (3)..(3)
                <223>  Xaa is any amino acid apart from proline

                <220>
                <221>  MISC_FEATURE
                <222>  (5)..(5)
                <223>  Xaa is any amino acid apart from proline

                <220>
                <221>  MISC_FEATURE
                <222>  (6)..(6)
                <223>  Xaa can be Ser or Thr

                <400>  18

                Lys Xaa Xaa Asn Xaa Xaa Lys
                1                    5



                <210>  19
                <211>  9
                <212>  PRT
                <213>  Artificial Sequence

                <220>
                <223>  consensus sequence


                <220>
                <221>  MISC_FEATURE
                <222>  (1)..(1)
                <223>  Xaa can be Lys or Arg

                <220>
                <221>  MISC_FEATURE
                <222>  (2)..(2)
                <223>  Xaa can be Asp or Glu

                <220>
                <221>  MISC_FEATURE
                <222>  (3)..(3)
                <223>  Xaa is any amino acid apart from proline

                <220>
```

```
<221>  MISC_FEATURE
<222>  (5)..(5)
<223>  Xaa is any amino acid apart from proline

<220>
<221>  MISC_FEATURE
<222>  (6)..(6)
<223>  Xaa can be Ser or Thr

<220>
<221>  MISC_FEATURE
<222>  (7)..(7)
<223>  Xaa is any amino acid except lysine or arginine

<220>
<221>  MISC_FEATURE
<222>  (8)..(8)
<223>  Xaa is any amino acid except lysine or arginine

<220>
<221>  MISC_FEATURE
<222>  (9)..(9)
<223>  Xaa can be Lys or Arg

<400>  19

Xaa Xaa Xaa Asn Xaa Xaa Xaa Xaa Xaa
1                   5


<210>  20
<211>  9
<212>  PRT
<213>  Artificial Sequence

<220>
<223>  consensus sequence


<220>
<221>  MISC_FEATURE
<222>  (2)..(2)
<223>  Xaa can be Asp or Glu

<220>
<221>  MISC_FEATURE
<222>  (3)..(3)
<223>  Xaa is any amino acid apart from proline

<220>
<221>  MISC_FEATURE
<222>  (5)..(5)
<223>  Xaa is any amino acid apart from proline

<220>
<221>  MISC_FEATURE
<222>  (6)..(6)
<223>  Xaa can be Ser or Thr

<400>  20

Lys Xaa Xaa Asn Xaa Xaa Ser Ala Arg
1                   5
```

```
<210>  21
<211>  19
<212>  PRT
<213>  Escherichia coli

<400>  21

Met Ile Lys Phe Leu Ser Ala Leu Ile Leu Leu Leu Val Thr Thr Ala
1               5                   10                  15

Ala Gln Ala


<210>  22
<211>  21
<212>  PRT
<213>  Escherichia coli

<400>  22

Met Lys Lys Thr Ala Ile Ala Ile Ala Val Ala Leu Ala Gly Phe Ala
1               5                   10                  15

Thr Val Ala Gln Ala
            20


<210>  23
<211>  26
<212>  PRT
<213>  Escherichia coli

<400>  23

Met Lys Ile Lys Thr Gly Ala Arg Ile Leu Ala Leu Ser Ala Leu Thr
1               5                   10                  15

Thr Met Met Phe Ser Ala Ser Ala Leu Ala
            20                  25


<210>  24
<211>  22
<212>  PRT
<213>  Erwinia carotovora

<400>  24

Met Lys Tyr Leu Leu Pro Thr Ala Ala Ala Gly Leu Leu Leu Leu Ala
1               5                   10                  15

Ala Gln Pro Ala Met Ala
            20


<210>  25
```

```
<211>  23
<212>  PRT
<213>  Escherichia coli

<400>  25

Met Ser Phe Lys Lys Ile Ile Lys Ala Phe Val Ile Met Ala Ala Leu
1               5                   10                  15

Val Ser Val Gln Ala His Ala
                20


<210>  26
<211>  28
<212>  PRT
<213>  Escherichia coli

<400>  26

Met Phe Lys Phe Lys Lys Lys Phe Leu Val Gly Leu Thr Ala Ala Phe
1               5                   10                  15

Met Ser Ile Ser Met Phe Ser Ala Thr Ala Ser Ala
                20                  25


<210>  27
<211>  19
<212>  PRT
<213>  Escherichia coli

<400>  27

Met Lys Lys Ile Trp Leu Ala Leu Ala Gly Leu Val Leu Ala Phe Ser
1               5                   10                  15

Ala Ser Ala


<210>  28
<211>  21
<212>  PRT
<213>  Escherichia coli

<400>  28

Met Lys Gln Ala Leu Arg Val Ala Phe Gly Phe Leu Ile Leu Trp Ala
1               5                   10                  15

Ser Val Leu His Ala
                20


<210>  29
<211>  26
<212>  PRT
<213>  Artificial Sequence
```

```
<220>
<223>  SipA signal sequence

<400>  29

Met Lys Met Asn Lys Lys Val Leu Leu Thr Ser Thr Met Ala Ala Ser
1               5                   10                  15


Leu Leu Ser Val Ala Ser Val Gln Ala Ser
            20                  25



<210>  30
<211>  325
<212>  PRT
<213>  Artificial Sequence

<220>
<223>  Amino acid sequence of Hla H35L/H48C/G122C with N-terminal S,
       Flgl signal sequence, C-terminal GSHRHR, and KDQNRTK substitution
       for residue K131

<400>  30

Met Ile Lys Phe Leu Ser Ala Leu Ile Leu Leu Leu Val Thr Thr Ala
1               5                   10                  15


Ala Gln Ala Ser Ala Asp Ser Asp Ile Asn Ile Lys Thr Gly Thr Thr
            20                  25                  30


Asp Ile Gly Ser Asn Thr Thr Val Lys Thr Gly Asp Leu Val Thr Tyr
            35                  40                  45


Asp Lys Glu Asn Gly Met Leu Lys Lys Val Phe Tyr Ser Phe Ile Asp
        50                  55                  60


Asp Lys Asn Cys Asn Lys Lys Leu Leu Val Ile Arg Thr Lys Gly Thr
65                  70                  75                  80


Ile Ala Gly Gln Tyr Arg Val Tyr Ser Glu Glu Gly Ala Asn Lys Ser
                85                  90                  95


Gly Leu Ala Trp Pro Ser Ala Phe Lys Val Gln Leu Gln Leu Pro Asp
                100                 105                 110


Asn Glu Val Ala Gln Ile Ser Asp Tyr Tyr Pro Arg Asn Ser Ile Asp
                115                 120                 125


Thr Lys Glu Tyr Met Ser Thr Leu Thr Tyr Gly Phe Asn Cys Asn Val
        130                 135                 140


Thr Gly Asp Asp Thr Gly Lys Asp Gln Asn Arg Thr Lys Ile Gly Gly
```

145      150      155      160

Leu Ile Gly Ala Asn Val Ser Ile Gly His Thr Leu Lys Tyr Val Gln
         165          170          175

Pro Asp Phe Lys Thr Ile Leu Glu Ser Pro Thr Asp Lys Lys Val Gly
         180          185          190

Trp Lys Val Ile Phe Asn Asn Met Val Asn Gln Asn Trp Gly Pro Tyr
         195          200          205

Asp Arg Asp Ser Trp Asn Pro Val Tyr Gly Asn Gln Leu Phe Met Lys
         210          215          220

Thr Arg Asn Gly Ser Met Lys Ala Ala Asp Asn Phe Leu Asp Pro Asn
225          230          235          240

Lys Ala Ser Ser Leu Leu Ser Ser Gly Phe Ser Pro Asp Phe Ala Thr
         245          250          255

Val Ile Thr Met Asp Arg Lys Ala Ser Lys Gln Gln Thr Asn Ile Asp
         260          265          270

Val Ile Tyr Glu Arg Val Arg Asp Asp Tyr Gln Leu His Trp Thr Ser
         275          280          285

Thr Asn Trp Lys Gly Thr Asn Thr Lys Asp Lys Trp Ile Asp Arg Ser
         290          295          300

Ser Glu Arg Tyr Lys Ile Asp Trp Glu Lys Glu Glu Met Thr Asn Gly
305          310          315          320

Ser His Arg His Arg
         325

<210> 31
<211> 327
<212> PRT
<213> Artificial Sequence

<220>
<223> Amino acid sequence of mature Hla H35L/G122C/H48C with N-terminal
       S, Flgl signal sequence, C-terminal GSHRHR and KDSNITSAR
       substitution for residue K131

<400> 31

Met Ile Lys Phe Leu Ser Ala Leu Ile Leu Leu Leu Val Thr Thr Ala
1          5          10          15

```
Ala  Gln  Ala  Ser  Ala  Asp  Ser  Asp  Ile  Asn  Ile  Lys  Thr  Gly  Thr  Thr
               20                  25                  30

Asp  Ile  Gly  Ser  Asn  Thr  Thr  Val  Lys  Thr  Gly  Asp  Leu  Val  Thr  Tyr
               35                  40                  45

Asp  Lys  Glu  Asn  Gly  Met  Leu  Lys  Lys  Val  Phe  Tyr  Ser  Phe  Ile  Asp
               50                  55                  60

Asp  Lys  Asn  Cys  Asn  Lys  Lys  Leu  Leu  Val  Ile  Arg  Thr  Lys  Gly  Thr
65                  70                  75                  80

Ile  Ala  Gly  Gln  Tyr  Arg  Val  Tyr  Ser  Glu  Glu  Gly  Ala  Asn  Lys  Ser
               85                  90                  95

Gly  Leu  Ala  Trp  Pro  Ser  Ala  Phe  Lys  Val  Gln  Leu  Gln  Leu  Pro  Asp
               100                 105                 110

Asn  Glu  Val  Ala  Gln  Ile  Ser  Asp  Tyr  Tyr  Pro  Arg  Asn  Ser  Ile  Asp
               115                 120                 125

Thr  Lys  Glu  Tyr  Met  Ser  Thr  Leu  Thr  Tyr  Gly  Phe  Asn  Cys  Asn  Val
               130                 135                 140

Thr  Gly  Asp  Asp  Thr  Gly  Lys  Asp  Ser  Asn  Ile  Thr  Ser  Ala  Arg  Ile
145                 150                 155                 160

Gly  Gly  Leu  Ile  Gly  Ala  Asn  Val  Ser  Ile  Gly  His  Thr  Leu  Lys  Tyr
               165                 170                 175

Val  Gln  Pro  Asp  Phe  Lys  Thr  Ile  Leu  Glu  Ser  Pro  Thr  Asp  Lys  Lys
               180                 185                 190

Val  Gly  Trp  Lys  Val  Ile  Phe  Asn  Asn  Met  Val  Asn  Gln  Asn  Trp  Gly
               195                 200                 205

Pro  Tyr  Asp  Arg  Asp  Ser  Trp  Asn  Pro  Val  Tyr  Gly  Asn  Gln  Leu  Phe
               210                 215                 220

Met  Lys  Thr  Arg  Asn  Gly  Ser  Met  Lys  Ala  Ala  Asp  Asn  Phe  Leu  Asp
225                 230                 235                 240

Pro  Asn  Lys  Ala  Ser  Ser  Leu  Leu  Ser  Ser  Gly  Phe  Ser  Pro  Asp  Phe
               245                 250                 255

Ala  Thr  Val  Ile  Thr  Met  Asp  Arg  Lys  Ala  Ser  Lys  Gln  Gln  Thr  Asn
               260                 265                 270
```

89

```
Ile Asp Val Ile Tyr Glu Arg Val Arg Asp Asp Tyr Gln Leu His Trp
        275                 280                 285

Thr Ser Thr Asn Trp Lys Gly Thr Asn Thr Lys Asp Lys Trp Ile Asp
        290                 295                 300

Arg Ser Ser Glu Arg Tyr Lys Ile Asp Trp Glu Lys Glu Glu Met Thr
305                 310                 315                 320

Asn Gly Ser His Arg His Arg
                325
```

<210> 32
<211> 327
<212> PRT
<213> Artificial Sequence

<220>
<223> Amino acid sequence of mature Hla H35L/G122C/H48C with N-terminal
      S, Flgl signal sequence, C-terminal GSHRHR and KDSNSTSAR
      substitution for residue K131

<400> 32

```
Met Ile Lys Phe Leu Ser Ala Leu Ile Leu Leu Leu Val Thr Thr Ala
1               5               10                  15

Ala Gln Ala Ser Ala Asp Ser Asp Ile Asn Ile Lys Thr Gly Thr Thr
            20                  25                  30

Asp Ile Gly Ser Asn Thr Thr Val Lys Thr Gly Asp Leu Val Thr Tyr
        35                  40                  45

Asp Lys Glu Asn Gly Met Leu Lys Lys Val Phe Tyr Ser Phe Ile Asp
        50                  55                  60

Asp Lys Asn Cys Asn Lys Lys Leu Leu Val Ile Arg Thr Lys Gly Thr
65                  70                  75                  80

Ile Ala Gly Gln Tyr Arg Val Tyr Ser Glu Glu Gly Ala Asn Lys Ser
                85                  90                  95

Gly Leu Ala Trp Pro Ser Ala Phe Lys Val Gln Leu Gln Leu Pro Asp
                100                 105                 110

Asn Glu Val Ala Gln Ile Ser Asp Tyr Tyr Pro Arg Asn Ser Ile Asp
            115                 120                 125

Thr Lys Glu Tyr Met Ser Thr Leu Thr Tyr Gly Phe Asn Cys Asn Val
        130                 135                 140
```

```
Thr Gly Asp Asp Thr Gly Lys Asp Ser Asn Ser Thr Ser Ala Arg Ile
145             150             155             160

Gly Gly Leu Ile Gly Ala Asn Val Ser Ile Gly His Thr Leu Lys Tyr
                165             170             175

Val Gln Pro Asp Phe Lys Thr Ile Leu Glu Ser Pro Thr Asp Lys Lys
            180             185             190

Val Gly Trp Lys Val Ile Phe Asn Asn Met Val Asn Gln Asn Trp Gly
            195             200             205

Pro Tyr Asp Arg Asp Ser Trp Asn Pro Val Tyr Gly Asn Gln Leu Phe
        210             215             220

Met Lys Thr Arg Asn Gly Ser Met Lys Ala Ala Asp Asn Phe Leu Asp
225             230             235             240

Pro Asn Lys Ala Ser Ser Leu Leu Ser Ser Gly Phe Ser Pro Asp Phe
            245             250             255

Ala Thr Val Ile Thr Met Asp Arg Lys Ala Ser Lys Gln Gln Thr Asn
            260             265             270

Ile Asp Val Ile Tyr Glu Arg Val Arg Asp Asp Tyr Gln Leu His Trp
            275             280             285

Thr Ser Thr Asn Trp Lys Gly Thr Asn Thr Lys Asp Lys Trp Ile Asp
        290             295             300

Arg Ser Ser Glu Arg Tyr Lys Ile Asp Trp Glu Lys Glu Glu Met Thr
305             310             315             320

Asn Gly Ser His Arg His Arg
                325
```

```
<210>  33
<211>  327
<212>  PRT
<213>  Artificial Sequence

<220>
<223>  Amino acid sequence of mature Hla H35L/G122C/H48C with N-terminal
       S, Flgl signal sequence, C-terminal GSHRHR and KDSNVTSAR
       substitution for residue K131,

<400>  33
```

```
Met Ile Lys Phe Leu Ser Ala Leu Ile Leu Leu Leu Val Thr Thr Ala
```

|     |     | 1   |     |     |     | 5   |     |     |     |     | 10  |     |     |     |     | 15  |
| --- | --- | --- | --- | --- | --- | --- | --- | --- | --- | --- | --- | --- | --- | --- | --- | --- |

Ala Gln Ala Ser Ala Asp Ser Asp Ile Asn Ile Lys Thr Gly Thr Thr
        20              25              30

Asp Ile Gly Ser Asn Thr Thr Val Lys Thr Gly Asp Leu Val Thr Tyr
        35              40              45

Asp Lys Glu Asn Gly Met Leu Lys Lys Val Phe Tyr Ser Phe Ile Asp
    50              55              60

Asp Lys Asn Cys Asn Lys Lys Leu Leu Val Ile Arg Thr Lys Gly Thr
65              70              75              80

Ile Ala Gly Gln Tyr Arg Val Tyr Ser Glu Glu Gly Ala Asn Lys Ser
            85              90              95

Gly Leu Ala Trp Pro Ser Ala Phe Lys Val Gln Leu Gln Leu Pro Asp
            100             105             110

Asn Glu Val Ala Gln Ile Ser Asp Tyr Tyr Pro Arg Asn Ser Ile Asp
        115             120             125

Thr Lys Glu Tyr Met Ser Thr Leu Thr Tyr Gly Phe Asn Cys Asn Val
    130             135             140

Thr Gly Asp Asp Thr Gly Lys Asp Ser Asn Val Thr Ser Ala Arg Ile
145             150             155             160

Gly Gly Leu Ile Gly Ala Asn Val Ser Ile Gly His Thr Leu Lys Tyr
            165             170             175

Val Gln Pro Asp Phe Lys Thr Ile Leu Glu Ser Pro Thr Asp Lys Lys
        180             185             190

Val Gly Trp Lys Val Ile Phe Asn Asn Met Val Asn Gln Asn Trp Gly
        195             200             205

Pro Tyr Asp Arg Asp Ser Trp Asn Pro Val Tyr Gly Asn Gln Leu Phe
    210             215             220

Met Lys Thr Arg Asn Gly Ser Met Lys Ala Ala Asp Asn Phe Leu Asp
225             230             235             240

Pro Asn Lys Ala Ser Ser Leu Leu Ser Ser Gly Phe Ser Pro Asp Phe
            245             250             255

```
Ala Thr Val Ile Thr Met Asp Arg Lys Ala Ser Lys Gln Gln Thr Asn
            260                 265                 270

Ile Asp Val Ile Tyr Glu Arg Val Arg Asp Asp Tyr Gln Leu His Trp
            275                 280                 285

Thr Ser Thr Asn Trp Lys Gly Thr Asn Thr Lys Asp Lys Trp Ile Asp
            290                 295                 300

Arg Ser Ser Glu Arg Tyr Lys Ile Asp Trp Glu Lys Glu Glu Met Thr
305                 310                 315                 320

Asn Gly Ser His Arg His Arg
                325


<210>  34
<211>  327
<212>  PRT
<213>  Artificial Sequence

<220>
<223>  Amino acid sequence of mature Hla H35L/G122C/H48C with N-terminal
       S, Flgl signal sequence, C-terminal GSHRHR and KDSNATSAR
       substitution for residue K131

<400>  34

Met Ile Lys Phe Leu Ser Ala Leu Ile Leu Leu Leu Val Thr Thr Ala
1                5                   10                  15

Ala Gln Ala Ser Ala Asp Ser Asp Ile Asn Ile Lys Thr Gly Thr Thr
            20                  25                  30

Asp Ile Gly Ser Asn Thr Thr Val Lys Thr Gly Asp Leu Val Thr Tyr
            35                  40                  45

Asp Lys Glu Asn Gly Met Leu Lys Lys Val Phe Tyr Ser Phe Ile Asp
            50                  55                  60

Asp Lys Asn Cys Asn Lys Lys Leu Leu Val Ile Arg Thr Lys Gly Thr
65                  70                  75                  80

Ile Ala Gly Gln Tyr Arg Val Tyr Ser Glu Glu Gly Ala Asn Lys Ser
                85                  90                  95

Gly Leu Ala Trp Pro Ser Ala Phe Lys Val Gln Leu Gln Leu Pro Asp
            100                 105                 110

Asn Glu Val Ala Gln Ile Ser Asp Tyr Tyr Pro Arg Asn Ser Ile Asp
            115                 120                 125
```

```
Thr Lys Glu Tyr Met Ser Thr Leu Thr Tyr Gly Phe Asn Cys Asn Val
    130                 135             140

Thr Gly Asp Asp Thr Gly Lys Asp Ser Asn Val Thr Ser Ala Arg Ile
    145             150             155             160

Gly Gly Leu Ile Gly Ala Asn Val Ser Ile Gly His Thr Leu Lys Tyr
            165             170             175

Val Gln Pro Asp Phe Lys Thr Ile Leu Glu Ser Pro Thr Asp Lys Lys
            180             185             190

Val Gly Trp Lys Val Ile Phe Asn Asn Met Val Asn Gln Asn Trp Gly
            195             200             205

Pro Tyr Asp Arg Asp Ser Trp Asn Pro Val Tyr Gly Asn Gln Leu Phe
    210             215             220

Met Lys Thr Arg Asn Gly Ser Met Lys Ala Ala Asp Asn Phe Leu Asp
    225             230             235             240

Pro Asn Lys Ala Ser Ser Leu Leu Ser Ser Gly Phe Ser Pro Asp Phe
            245             250             255

Ala Thr Val Ile Thr Met Asp Arg Lys Ala Ser Lys Gln Gln Thr Asn
            260             265             270

Ile Asp Val Ile Tyr Glu Arg Val Arg Asp Asp Tyr Gln Leu His Trp
            275             280             285

Thr Ser Thr Asn Trp Lys Gly Thr Asn Thr Lys Asp Lys Trp Ile Asp
    290             295             300

Arg Ser Ser Glu Arg Tyr Lys Ile Asp Trp Glu Lys Glu Glu Met Thr
    305             310             315             320

Asn Gly Ser His Arg His Arg
            325
```

<210> 35
<211> 299
<212> PRT
<213> Artificial Sequence

<220>
<223> Amino acid sequence of mature Hla H35L/H48C/G122C with KDQNRTK
      substitution for residue K131

<400> 35

```
Ala Asp Ser Asp Ile Asn Ile Lys Thr Gly Thr Thr Asp Ile Gly Ser
1               5               10              15

Asn Thr Thr Val Lys Thr Gly Asp Leu Val Thr Tyr Asp Lys Glu Asn
            20              25              30

Gly Met Leu Lys Lys Val Phe Tyr Ser Phe Ile Asp Asp Lys Asn Cys
        35              40              45

Asn Lys Lys Leu Leu Val Ile Arg Thr Lys Gly Thr Ile Ala Gly Gln
    50              55              60

Tyr Arg Val Tyr Ser Glu Glu Gly Ala Asn Lys Ser Gly Leu Ala Trp
65              70              75              80

Pro Ser Ala Phe Lys Val Gln Leu Gln Leu Pro Asp Asn Glu Val Ala
            85              90              95

Gln Ile Ser Asp Tyr Tyr Pro Arg Asn Ser Ile Asp Thr Lys Glu Tyr
            100             105             110

Met Ser Thr Leu Thr Tyr Gly Phe Asn Cys Asn Val Thr Gly Asp Asp
        115             120             125

Thr Gly Lys Asp Gln Asn Arg Thr Lys Ile Gly Gly Leu Ile Gly Ala
    130             135             140

Asn Val Ser Ile Gly His Thr Leu Lys Tyr Val Gln Pro Asp Phe Lys
145             150             155             160

Thr Ile Leu Glu Ser Pro Thr Asp Lys Lys Val Gly Trp Lys Val Ile
            165             170             175

Phe Asn Asn Met Val Asn Gln Asn Trp Gly Pro Tyr Asp Arg Asp Ser
        180             185             190

Trp Asn Pro Val Tyr Gly Asn Gln Leu Phe Met Lys Thr Arg Asn Gly
        195             200             205

Ser Met Lys Ala Ala Asp Asn Phe Leu Asp Pro Asn Lys Ala Ser Ser
    210             215             220

Leu Leu Ser Ser Gly Phe Ser Pro Asp Phe Ala Thr Val Ile Thr Met
225             230             235             240

Asp Arg Lys Ala Ser Lys Gln Gln Thr Asn Ile Asp Val Ile Tyr Glu
            245             250             255
```

Arg Val Arg Asp Asp Tyr Gln Leu His Trp Thr Ser Thr Asn Trp Lys
        260              265              270

Gly Thr Asn Thr Lys Asp Lys Trp Ile Asp Arg Ser Ser Glu Arg Tyr
        275              280              285

Lys Ile Asp Trp Glu Lys Glu Glu Met Thr Asn
        290              295

<210> 36
<211> 301
<212> PRT
<213> Artificial Sequence

<220>
<223> Amino acid sequence of mature Hla H35L/G122C/H48C with KDSNITSAR
      substitution for residue K131

<400> 36

Ala Asp Ser Asp Ile Asn Ile Lys Thr Gly Thr Thr Asp Ile Gly Ser
1             5              10              15

Asn Thr Thr Val Lys Thr Gly Asp Leu Val Thr Tyr Asp Lys Glu Asn
        20              25              30

Gly Met Leu Lys Lys Val Phe Tyr Ser Phe Ile Asp Asp Lys Asn Cys
        35              40              45

Asn Lys Lys Leu Leu Val Ile Arg Thr Lys Gly Thr Ile Ala Gly Gln
        50              55              60

Tyr Arg Val Tyr Ser Glu Glu Gly Ala Asn Lys Ser Gly Leu Ala Trp
65              70            75              80

Pro Ser Ala Phe Lys Val Gln Leu Gln Leu Pro Asp Asn Glu Val Ala
           85             90              95

Gln Ile Ser Asp Tyr Tyr Pro Arg Asn Ser Ile Asp Thr Lys Glu Tyr
        100             105           110

Met Ser Thr Leu Thr Tyr Gly Phe Asn Cys Asn Val Thr Gly Asp Asp
        115             120           125

Thr Gly Lys Asp Ser Asn Ile Thr Ser Ala Arg Ile Gly Gly Leu Ile
        130             135           140

Gly Ala Asn Val Ser Ile Gly His Thr Leu Lys Tyr Val Gln Pro Asp
145              150            155           160

```
Phe Lys Thr Ile Leu Glu Ser Pro Thr Asp Lys Lys Val Gly Trp Lys
            165                 170                 175

Val Ile Phe Asn Asn Met Val Asn Gln Asn Trp Gly Pro Tyr Asp Arg
            180                 185                 190

Asp Ser Trp Asn Pro Val Tyr Gly Asn Gln Leu Phe Met Lys Thr Arg
            195                 200                 205

Asn Gly Ser Met Lys Ala Ala Asp Asn Phe Leu Asp Pro Asn Lys Ala
        210                 215                 220

Ser Ser Leu Leu Ser Ser Gly Phe Ser Pro Asp Phe Ala Thr Val Ile
225                 230                 235                 240

Thr Met Asp Arg Lys Ala Ser Lys Gln Gln Thr Asn Ile Asp Val Ile
            245                 250                 255

Tyr Glu Arg Val Arg Asp Asp Tyr Gln Leu His Trp Thr Ser Thr Asn
            260                 265                 270

Trp Lys Gly Thr Asn Thr Lys Asp Lys Trp Ile Asp Arg Ser Ser Glu
            275                 280                 285

Arg Tyr Lys Ile Asp Trp Glu Lys Glu Glu Met Thr Asn
        290                 295                 300
```

```
Ala Asp Ser Asp Ile Asn Ile Lys Thr Gly Thr Thr Asp Ile Gly Ser
1               5                   10                  15

Asn Thr Thr Val Lys Thr Gly Asp Leu Val Thr Tyr Asp Lys Glu Asn
            20                  25                  30

Gly Met Leu Lys Lys Val Phe Tyr Ser Phe Ile Asp Asp Lys Asn Cys
            35                  40                  45

Asn Lys Lys Leu Leu Val Ile Arg Thr Lys Gly Thr Ile Ala Gly Gln
        50                  55                  60
```

Tyr Arg Val Tyr Ser Glu Glu Gly Ala Asn Lys Ser Gly Leu Ala Trp
65              70              75              80

Pro Ser Ala Phe Lys Val Gln Leu Gln Leu Pro Asp Asn Glu Val Ala
                85              90              95

Gln Ile Ser Asp Tyr Tyr Pro Arg Asn Ser Ile Asp Thr Lys Glu Tyr
            100             105             110

Met Ser Thr Leu Thr Tyr Gly Phe Asn Cys Asn Val Thr Gly Asp Asp
            115             120             125

Thr Gly Lys Asp Ser Asn Ser Thr Ser Ala Arg Ile Gly Gly Leu Ile
        130             135             140

Gly Ala Asn Val Ser Ile Gly His Thr Leu Lys Tyr Val Gln Pro Asp
145             150             155             160

Phe Lys Thr Ile Leu Glu Ser Pro Thr Asp Lys Lys Val Gly Trp Lys
                165             170             175

Val Ile Phe Asn Asn Met Val Asn Gln Asn Trp Gly Pro Tyr Asp Arg
            180             185             190

Asp Ser Trp Asn Pro Val Tyr Gly Asn Gln Leu Phe Met Lys Thr Arg
        195             200             205

Asn Gly Ser Met Lys Ala Ala Asp Asn Phe Leu Asp Pro Asn Lys Ala
    210             215             220

Ser Ser Leu Leu Ser Ser Gly Phe Ser Pro Asp Phe Ala Thr Val Ile
225             230             235             240

Thr Met Asp Arg Lys Ala Ser Lys Gln Gln Thr Asn Ile Asp Val Ile
            245             250             255

Tyr Glu Arg Val Arg Asp Asp Tyr Gln Leu His Trp Thr Ser Thr Asn
            260             265             270

Trp Lys Gly Thr Asn Thr Lys Asp Lys Trp Ile Asp Arg Ser Ser Glu
        275             280             285

Arg Tyr Lys Ile Asp Trp Glu Lys Glu Glu Met Thr Asn
    290             295             300

<210> 38

<211> 301
<212> PRT
<213> Artificial Sequence

<220>
<223> Amino acid sequence of mature Hla H35L/G122C/H48C with KDSNVTSAR
substitution for residue K131

<400> 38

Ala Asp Ser Asp Ile Asn Ile Lys Thr Gly Thr Thr Asp Ile Gly Ser
1               5                   10                  15

Asn Thr Thr Val Lys Thr Gly Asp Leu Val Thr Tyr Asp Lys Glu Asn
            20                  25                  30

Gly Met Leu Lys Lys Val Phe Tyr Ser Phe Ile Asp Asp Lys Asn Cys
        35                  40                  45

Asn Lys Lys Leu Leu Val Ile Arg Thr Lys Gly Thr Ile Ala Gly Gln
        50                  55                  60

Tyr Arg Val Tyr Ser Glu Glu Gly Ala Asn Lys Ser Gly Leu Ala Trp
65                  70                  75                  80

Pro Ser Ala Phe Lys Val Gln Leu Gln Leu Pro Asp Asn Glu Val Ala
                85                  90                  95

Gln Ile Ser Asp Tyr Tyr Pro Arg Asn Ser Ile Asp Thr Lys Glu Tyr
            100                 105                 110

Met Ser Thr Leu Thr Tyr Gly Phe Asn Cys Asn Val Thr Gly Asp Asp
        115                 120                 125

Thr Gly Lys Asp Ser Asn Val Thr Ser Ala Arg Ile Gly Gly Leu Ile
        130                 135                 140

Gly Ala Asn Val Ser Ile Gly His Thr Leu Lys Tyr Val Gln Pro Asp
145                 150                 155                 160

Phe Lys Thr Ile Leu Glu Ser Pro Thr Asp Lys Lys Val Gly Trp Lys
                165                 170                 175

Val Ile Phe Asn Asn Met Val Asn Gln Asn Trp Gly Pro Tyr Asp Arg
                180                 185                 190

Asp Ser Trp Asn Pro Val Tyr Gly Asn Gln Leu Phe Met Lys Thr Arg
        195                 200                 205

Asn Gly Ser Met Lys Ala Ala Asp Asn Phe Leu Asp Pro Asn Lys Ala

```
                210                    215                      220


        Ser Ser Leu Leu Ser Ser Gly Phe Ser Pro Asp Phe Ala Thr Val Ile
        225                 230                 235                     240


        Thr Met Asp Arg Lys Ala Ser Lys Gln Gln Thr Asn Ile Asp Val Ile
                        245                 250                     255


        Tyr Glu Arg Val Arg Asp Asp Tyr Gln Leu His Trp Thr Ser Thr Asn
                    260                 265                 270


        Trp Lys Gly Thr Asn Thr Lys Asp Lys Trp Ile Asp Arg Ser Ser Glu
                275                 280                 285


        Arg Tyr Lys Ile Asp Trp Glu Lys Glu Glu Met Thr Asn
            290                 295                 300
```

<210> 39
<211> 301
<212> PRT
<213> Artificial Sequence

<220>
<223> Amino acid sequence of mature Hla H35L/G122C/H48C with KDSNATSAR
      substitution for residue K131

<400> 39

```
        Ala Asp Ser Asp Ile Asn Ile Lys Thr Gly Thr Thr Asp Ile Gly Ser
        1                   5                   10                  15


        Asn Thr Thr Val Lys Thr Gly Asp Leu Val Thr Tyr Asp Lys Glu Asn
                    20                  25                  30


        Gly Met Leu Lys Lys Val Phe Tyr Ser Phe Ile Asp Asp Lys Asn Cys
                35                  40                  45


        Asn Lys Lys Leu Leu Val Ile Arg Thr Lys Gly Thr Ile Ala Gly Gln
            50                  55                  60


        Tyr Arg Val Tyr Ser Glu Glu Gly Ala Asn Lys Ser Gly Leu Ala Trp
        65                  70                  75                  80


        Pro Ser Ala Phe Lys Val Gln Leu Gln Leu Pro Asp Asn Glu Val Ala
                        85                  90                  95


        Gln Ile Ser Asp Tyr Tyr Pro Arg Asn Ser Ile Asp Thr Lys Glu Tyr
                    100                 105                 110


        Met Ser Thr Leu Thr Tyr Gly Phe Asn Cys Asn Val Thr Gly Asp Asp
```

115                          120                          125

Thr Gly Lys Asp Ser Asn Val Thr Ser Ala Arg Ile Gly Gly Leu Ile
    130                 135                 140

Gly Ala Asn Val Ser Ile Gly His Thr Leu Lys Tyr Val Gln Pro Asp
145                 150                 155                 160

Phe Lys Thr Ile Leu Glu Ser Pro Thr Asp Lys Lys Val Gly Trp Lys
                165                 170                 175

Val Ile Phe Asn Asn Met Val Asn Gln Asn Trp Gly Pro Tyr Asp Arg
                180                 185                 190

Asp Ser Trp Asn Pro Val Tyr Gly Asn Gln Leu Phe Met Lys Thr Arg
    195                 200                 205

Asn Gly Ser Met Lys Ala Ala Asp Asn Phe Leu Asp Pro Asn Lys Ala
    210                 215                 220

Ser Ser Leu Leu Ser Ser Gly Phe Ser Pro Asp Phe Ala Thr Val Ile
225                 230                 235                 240

Thr Met Asp Arg Lys Ala Ser Lys Gln Gln Thr Asn Ile Asp Val Ile
                245                 250                 255

Tyr Glu Arg Val Arg Asp Asp Tyr Gln Leu His Trp Thr Ser Thr Asn
                260                 265                 270

Trp Lys Gly Thr Asn Thr Lys Asp Lys Trp Ile Asp Arg Ser Ser Glu
                275                 280                 285

Arg Tyr Lys Ile Asp Trp Glu Lys Glu Glu Met Thr Asn
    290                 295                 300

<210>  40
<211>  7
<212>  PRT
<213>  Artificial Sequence

<220>
<223>  KDQNRTK glycosite

<400>  40

Lys Asp Gln Asn Arg Thr Lys
1                   5

<210>  41
<211>  7

```
<212>   PRT
<213>   Artificial Sequence

<220>
<223>   KDQNATK glycosite

<400>   41

Lys Asp Gln Asn Ala Thr Lys
1               5


<210>   42
<211>   9
<212>   PRT
<213>   Artificial Sequence

<220>
<223>   KDSNITSAR glycosite

<400>   42

Lys Asp Ser Asn Ile Thr Ser Ala Arg
1               5


<210>   43
<211>   9
<212>   PRT
<213>   Artificial Sequence

<220>
<223>   KDSNSTSAR glycosite

<400>   43

Lys Asp Ser Asn Ser Thr Ser Ala Arg
1               5


<210>   44
<211>   9
<212>   PRT
<213>   Artificial Sequence

<220>
<223>   KDSNVTSAR glycosite

<400>   44

Lys Asp Ser Asn Val Thr Ser Ala Arg
1               5


<210>   45
<211>   9
<212>   PRT
<213>   Artificial Sequence

<220>
<223>   KDSNATSAR glycosite

<400>   45
```

```
Lys Asp Ser Asn Ala Thr Ser Ala Arg
1               5


<210>  46
<211>  9
<212>  PRT
<213>  Artificial Sequence

<220>
<223>  consensus sequence


<220>
<221>  MISC_FEATURE
<222>  (1)..(1)
<223>  Xaa can be Lys or Arg

<220>
<221>  MISC_FEATURE
<222>  (2)..(2)
<223>  Xaa represent 0-9 amino acids which are any amino acid except
       cysteine, methionine, asparagine, glutamine, lysine or arginine.

<220>
<221>  MISC_FEATURE
<222>  (3)..(3)
<223>  Xaa can be Asp or Glu

<220>
<221>  MISC_FEATURE
<222>  (4)..(4)
<223>  Xaa is any amino acid except proline, lysine or arginine

<220>
<221>  MISC_FEATURE
<222>  (6)..(6)
<223>  Xaa is any amino acid except proline, lysine or arginine

<220>
<221>  MISC_FEATURE
<222>  (7)..(7)
<223>  Xaa can be Ser or Thr

<220>
<221>  MISC_FEATURE
<222>  (8)..(8)
<223>  Xaa represent 0-9 amino acids which are any amino acid except
       cysteine, methionine, asparagine, glutamine, lysine or arginine.

<220>
<221>  MISC_FEATURE
<222>  (9)..(9)
<223>  Xaa can be Lys or Arg

<400>  46

Xaa Xaa Xaa Xaa Asn Xaa Xaa Xaa Xaa
1               5


<210>  47
```

```
<211>  9
<212>  PRT
<213>  Artificial Sequence

<220>
<223>  consensus sequence


<220>
<221>  MISC_FEATURE
<222>  (1)..(1)
<223>  Xaa can be Lys or Arg

<220>
<221>  MISC_FEATURE
<222>  (2)..(2)
<223>  Xaa represent 0-9 amino acids which are any amino acid except
       cysteine, methionine, asparagine, glutamine, lysine or arginine.

<220>
<221>  MISC_FEATURE
<222>  (3)..(3)
<223>  Xaa can be Asp or Glu

<220>
<221>  MISC_FEATURE
<222>  (4)..(4)
<223>  Xaa is any amino acid except proline, cysteine, methionine,
       asparagine, glutamine, lysine or arginine

<220>
<221>  MISC_FEATURE
<222>  (6)..(6)
<223>  Xaa is any amino acid except proline, cysteine, methionine,
       asparagine, glutamine, lysine or arginine

<220>
<221>  MISC_FEATURE
<222>  (7)..(7)
<223>  Xaa can be Ser or Thr

<220>
<221>  MISC_FEATURE
<222>  (8)..(8)
<223>  Xaa represent 0-9 amino acids which are any amino acid except
       cysteine, methionine, asparagine, glutamine, lysine or arginine.

<220>
<221>  MISC_FEATURE
<222>  (9)..(9)
<223>  Xaa can be Lys or Arg

<400>  47

Xaa Xaa Xaa Xaa Asn Xaa Xaa Xaa Xaa
1                   5
```

**Description**

**FIELD OF THE INVENTION**

The present invention relates to analytical tools for the characterisation of bioconjugates, in particular the measurement of glycosylation levels.

**BACKGROUND TO THE INVENTION**

Glycoconjugate vaccines have been proven to be efficacious and cost effective in the prevention of infectious diseases caused by encapsulated bacteria. In the last decade, new approaches have been taken for glycoconjugate vaccine production, including techniques exploiting bacterial N-glycosylation. The most well-developed of these 'bioconjugation' technologies is based on the production of glycoproteins in *Escherichia coli* in which the *Campylobacter jejuni* glycosylation machinery PgIB is co-expressed with a pathogen polysaccharide chain and a target carrier protein, acceptor of the polysaccharide (Wacker et al, 2002, Science 298:1790-3), which is engineered to contain a consensus sequence for PgIB.

The main strength of the bioconjugation technology is the selectivity of the site of glycosylation on the carrier protein sequence. This is achieved by selectively introducing into the carrier protein sequence specific amino acids, creating one or more effective consensus sequences for selective conjugation to the polysaccharide chain. The core consensus sequence of PgIB is D/E-X-N-Z-S/T wherein X and Z are independently any amino acid apart from proline (see Wacker et al, 2002, Science 298:1790-3), but an extended consensus sequence of K-D/E-X-N-Z-S/T-K is glycosylated with higher efficiency and is more widely used (see for example WO2019/121924 and WO20119/121926).

This technology is of particular interest for vaccine development especially when carrier proteins are selected to have a dual role as carrier and antigen, as it can preserve key protective epitopes. Furthermore, bioconjugation shows a higher suitability to large scale production in manufacturing of vaccines in comparison to the chemical conjugation, as it decreases the need for pathogen handling, permits a reduction in production process steps, and is less time and resource-consuming.

Bioconjugate vaccine candidates have been recently proposed for the prevention of Gram-negative (*Salmonella enterica, Shigella* spp, pathogenic *E. coli*) and Gram-positive pathogen infections (*Streptococcus pneumoniae* and *Staphylococcus aureus*) (e.g. Wetter et al, 2013, Glycoconj J. 30:511-22. Engineering, conjugation, and immunogenicity assessment of Escherichia coli 0121 O antigen for its potential use as a typhoid vaccine component; Wacker et al., 2014, J. Infect. Dis. 209:1551-1561; Van den Dobbelsteen et al., 2016, Vaccine 34:4152-60). Among them, *S. aureus* alpha toxin (Hla) bioconjugated with *S. aureus* type 5 CP (Hla-CP5) was shown to induce rabbit or mice protective antibodies recognizing both the glycan and the protein moieties, demonstrating the dual role of Hla protein as carrier and as protective antigen (Wacker et al., 2014, J. Infect. Dis. 209:1551-1561). This data is particularly relevant for the development of a vaccine preventing the diffusion of *S. aureus,* which is becoming challenging to fight due to the increase of the multi-drug resistant strains spreading around the world, including hospital and community-related infections strains.

Despite the increasing relevance of bioconjugates in vaccine development field, robust analytical tools needed to evaluate efficacy of carrier glycosylation are still lacking (Micoli, F.et al, 2018, Molecules 23:1451). In particular, precise quantification of the extent of glycosylation remains a challenging task, although this information is fundamental to fulfil potential regulatory requirements and to monitor antigen production and characterisation. There is thus a need in the art for robust and reliable methods of accurately quantifying absolute levels of glycosylation site occupancy in bioconjugates.

**SUMMARY OF THE INVENTION**

The inventors have designed universal consensus sequences for protein N-glycosylation which are suitable for the absolute quantification of glycosylation site occupancy. Specifically, the use of these consensus sequences allows the overall protein concentration and the unglycosylated portion of the protein to be quantified simultaneously by using heavy isotope-labeled internal standards in an LC-MS/MS analysis, and the extent of site occupancy to be accurately determined (Zhu et al 2015, J Am Soc Mass Spectrom. 25:1012-7).

The inventors devised a method based on that of Zhu et al for quantification of glycosylation using as a model a Hla carrier protein containing the glycosylation consensus site KDQNRTK (described in WO2019/121924). The strategy is based on the quantification of the natively unglycosylated form of the glycopeptide, using isotopically labeled internal standards. In brief, two sets of heavy isotope labeled peptide standards are spiked into the sample before trypsin digestion, and the digested sample is analyzed by LC-MS. One set of peptide standards is employed to determine the total glycoprotein amount, while the other standard monitors the unglycosylated amount of the glycoprotein. In this way, the abundance of the glycosylated portion of the protein is calculated by subtracting the unglycosylated protein amount from the total protein amount, and the site occupancy is then determined.

However, the KDQNRTK consensus sequence was found to generate a tryptic peptide which was too short and too hydrophilic to allow a LC-MS quantification. The same problem would be encountered for other commonly used consensus sequences such as KDQNATK.

The inventors thus set out to design universal consensus sites that would be compatible with the method, i.e. which would be glycosylated with at least the same efficiency as the previously used sites and would also generate tryptic peptides detectable by LC-MS. Using Hla as a proof of principle, they were able to successfully design consensus sequences suitable for the quantification of the extent of conjugation by mass spectrometry.

The present invention invention will permit the amount of unglycosylated carrier in the final product to be quantified; the rate of bioconjugation to be followed in-process; and the extent of glycosylation on single and multiple consensus sites to be quantified. Moreover, the selectivity of detection reduces the necessity for extensive sample purification supporting the characterisation of in-process and final product.

In a first aspect, therefore, the invention provides a consensus sequence comprising or consisting of the following amino acid sequence:

K/R-$Z_{0-9}$-D/E-X-N-Y-S/T-$Z_{0-9}$-K/R

wherein X and Y are independently any amino acid except proline, and Z represents any amino acid. In a preferred embodiment, X and Y are independently any amino acid except proline, lysine or arginine. In an embodiment, Z represents any amino acid except lysine or arginine. In an embodiment, X, Y and/or Z are not aromatic or hydrophobic amino acids. In a preferred embodiment, Z represents any amino acid except cysteine, methionine, asparagine, glutamine, lysine or arginine (eg SEQ ID NO: 47).

In a specific embodiment, the invention provides a consensus sequence(s) comprising or consisting of the amino acid sequence K/R-D/E-$X_1$-N-$X_2$-S/T-$Z_1$-$Z_2$-K/R (SEQ ID NO 19), wherein $X_1$ and $X_2$ are independently any amino acid apart from proline, lysine or arginine and wherein $Z_1$ and $Z_2$.are not lysine or arginine or cysteine. In an embodiment, the consensus sequence comprises or consists of the amino acid sequence of SEQ ID NO: 20. In an embodiment, the consensus sequence comprises or consists of the amino acid sequence of any one of SEQ ID No: 42, SEQ ID No: 43; SEQ ID No: 44 or SEQ ID No 45, preferably SEQ ID Nos: 42-44.

In one aspect, the invention provides a modified carrier protein, modified in that it comprises one or more consensus sequence(s) of the invention. Thus, the invention provides a modified carrier protein modified in that it comprises one or more consensus sequence(s) comprising or consisting of the amino acid sequence K/R-$(Z)_{0-9}$-D/E-X-N-Y-S/T-$(Z)_{0-9}$-K/R (SEQ ID NO : 46) as defined above; for example the amino acid sequence K/R-D/E-$X_1$-N-$X_2$-S/T-$Z_1$-$Z_2$-K/R (SEQ ID NO 19), wherein $X_1$ and $X_2$ are independently any amino acid apart from proline, lysine or arginine and wherein $Z_1$ and $Z_2$.are not lysine or arginine or cysteine. In an embodiment, the consensus sequence comprises or consists of the amino acid sequence of SEQ ID NO: 20. In an embodiment, the consensus sequence comprises or consists of the amino acid sequence of any one of SEQ ID No: 42, SEQ ID No: 43; SEQ ID No: 44 or SEQ ID No 45, preferably SEQ ID Nos: 42-44.

In an embodiment, said consensus sequence has been substituted for one or more amino acids of the carrier protein sequence. In another embodiment said consensus sequence has been inserted into the carrier protein sequence.

The modified carrier protein may comprise more than one said consensus sequence, optionally at least 2, 3, 4 or 5 consensus sequences. In a preferred embodiment, where a modified carrier protein contains more than one consensus sequence, all of said consensus sequences are different i.e. have different amino acid sequences.

The carrier protein may be any protein, preferably a protein able to elicit a T-dependent immune response. In specific embodiments, the carrier protein is CRM197, TT from *Clostridium tetani,* EPA from *P. aeruginosa,* Hcp1 from *P. aeruginosa,* Hla from *S. aureus,* ClfA from *S. aureus,* MBP from *E.*, PspA from *E. coli,* or MtrE from *N. gonorrhoeae.*

In an embodiment, the carrier protein comprises or consists of an amino acid sequence of any one of SEQ ID Nos: 1 to 16 or an amino acid sequence at least 80%, 85%, 90%, 92%, 95%, 96%, 97%, 98% or 99% identical to any one of SEQ ID NOs. 1 to 16.

In an embodiment, the modified carrier protein comprises or consists of an amino acid sequence at least 80%, 85%, 90%, 92%, 95%, 96% or 97% identical to any one of SEQ ID NOs. 1 to 16.

The modified carrier protein may be glycosylated. The invention also provides a glycosylated carrier protein of the invention, and conjugates (e.g. bioconjugates) comprising a modified carrier protein of the invention linked to a polysaccharide. The polysaccharide is linked to an amino acid on the modified carrier protein selected from asparagine, aspartic acid, glutamic acid, lysine, cysteine, tyrosine, histidine, arginine or tryptophan (preferably asparagine). In an embodiment, the capsular polysaccharide is from the same organism as the carrier protein. In an embodiment, the capsular polysaccharide is from a different organism to the carrier protein.

In an embodiment, the polysaccharide is a bacterial capsular polysaccharide, for example *Staphylococcus aureus* type 5 capsular saccharide, *Staphylococcus aureus* type 8 capsular saccharide, *N. meningitidis* serogroup A capsular saccharide (MenA), *N. meningitidis* serogroup C capsular saccharide (MenC), *N. meningitidis* serogroup Y capsular saccharide (MenY), *N. meningitidis* serogroup W capsular saccharide (MenW), *H. influenzae* type b capsular saccharide (Hib), Group B Streptococcus group I capsular saccharide, Group B Streptococcus group II capsular saccharide, Group B Streptococcus group III capsular saccharide, Group B Streptococcus group IV capsular saccharide, Group B Streptococcus group V capsular saccharide, Vi saccharide from *Salmonella typhi, N. meningitidis* LPS (such as L3 and/or L2), *M. catarrhalis* LPS, *H. influenzae* LPS, Shigella O-antigens, *P.aeruginosa* O-antigens, *E. coli* O-antigens or *S. pneumoniae* a *S. aureus* capsular polysaccharide.

According to a further aspect of the invention, there is provided a polynucleotide encoding a modified carrier protein or bioconjugate of the invention.

According to a further aspect of the invention, there is provided a vector comprising a polynucleotide encoding a modified carrier protein or bioconjugate of the invention.

According to a further aspect of the invention, there is provided a host cell comprising:

i) one or more nucleic acids that encode glycosyltransferase(s);
ii) a nucleic acid that encodes an oligosaccharyl transferase;
iii) a nucleic acid that encodes a modified Carrier protein of the invention; and optionally
iv) a nucleic acid that encodes a polymerase (*e.g. wzy*).

The nucleic acid that encodes the modified carrier protein may be carried on a plasmid in the host cell, or may be integrated into the genome of the host cell. The host cell is preferably *E. coli.*

According to a further aspect of the invention, there is provided a process for producing a bioconjugate that comprises (or consists of) a modified carrier protein linked to a saccharide, said method comprising: (i) culturing a host cell of the invention under conditions suitable for the production of proteins and (ii) isolating the bioconjugate produced by said host cell. Also provided is a bioconjugate produced by said process, wherein said bioconjugate comprises a polysaccharide linked to a modified carrier protein.

According to a further aspect of the invention, there is provided an immunogenic composition comprising the modified carrier protein of the invention, or a conjugate of the invention, or a bioconjugate of the invention and a pharmaceutically acceptable excipient or carrier.

According to a further aspect of the invention, there is provided a method of making a immunogenic composition of the invention comprising the step of mixing a modified carrier protein or the conjugate or the bioconjugate of the invention with a pharmaceutically acceptable excipient or carrier.

An immunogenic composition comprising the modified carrier protein, conjugate or bioconjugate of the invention.

A method of making the immunogenic composition of paragraph 32 comprising the step of mixing the modified carrier protein or the conjugate or the bioconjugate of the invention with a pharmaceutically acceptable excipient or carrier.

A vaccine comprising the immunogenic composition of the invention and a pharmaceutically acceptable excipient or carrier.

According to a further aspect of the invention, there is provided a method for the treatment or prevention of a bacterial infection in a subject in need thereof comprising administering to said subject a therapeutically effective amount of the modified carrier protein, conjugate or bioconjugate of the invention.

According to a further aspect of the invention, there is provided a method of immunising a human host against a bacterial infection comprising administering to the host an immunoprotective dose of the modified carrier protein, conjugate or bioconjugate of the invention.

According to a further aspect of the invention, there is provided a method of inducing an immune response to a bacterium in a subject, the method comprising administering a therapeutically or prophylactically effective amount of the modified carrier protein, conjugate or bioconjugate of the invention.

According to a further aspect of the invention, there is provided a modified carrier protein, conjugate or bioconjugate of the invention for use in the treatment or prevention of a disease caused by bacterial infection.

According to a further aspect of the invention, there is provided use of the the modified carrier protein, conjugate or bioconjugate of the invention in the manufacture of a medicament for the treatment or prevention of a disease caused by bacterial infection.

In specific embodiments, said bacterium or bacterial infection is selected from the group consisting of *Staphylococcus aureus, N. meningitidis, H. influenzae, H. influenzae* type b, Group B Streptococcus, *S. typhi, M. catarrhalis* LPS, *S. flexneri, P.aeruginosa, E. coli* or *S. pneumoniae.*

According to a further aspect of the invention, there is provided a method of measuring the level of glycosylation site occupancy of a carrier protein of the invention, said method comprisingdigesting the glycosylated carrier protein with a protease, e.g. trypsin; subjecting the digested protein to LC-MS; determining the concentration U of unmodified carrier protein; determining the concentration T of total carrier protein; and calculating glycosylation site occupancy according to the following equation:

$$Site\ Occupancy\ (\%) = \frac{(Total - unmodified)\ carrier\ concentration}{Total\ carrier\ concentration}x100$$

The concentration U of unmodified carrier protein is determined by determining the concentration of a peptide fragment corresponding to the consensus sequence of the invention. The concentration T of total carrier protein may suitably be determined by determining the concentration of one or more peptide fragments which are unique to said carrier protein.

## DESCRIPTION OF THE FIGURES

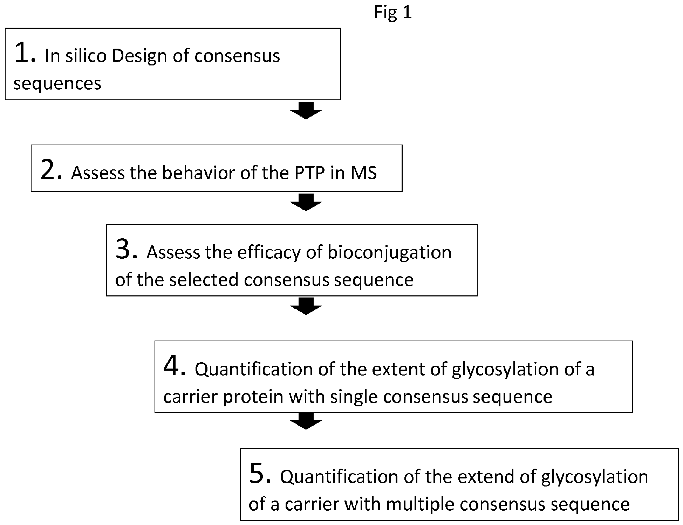

**Figure 1: Workflow of the strategy undertaken.**

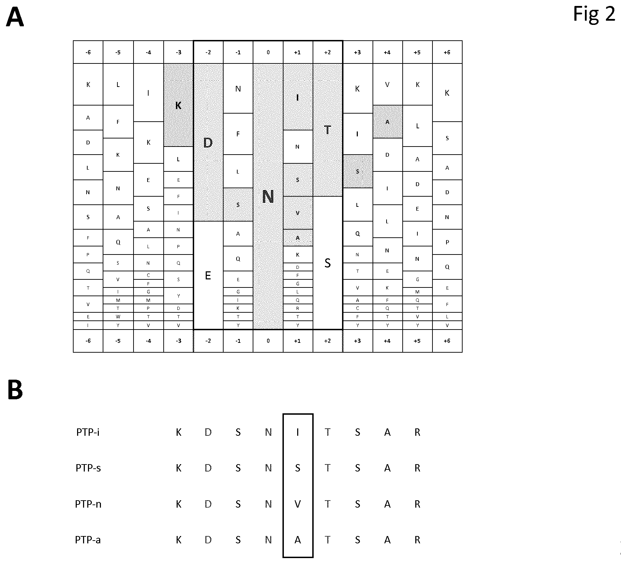

**Figure 2: In silico design of consensus sequences.** (A) Statistical analysis of the occurrence of amino acids in the region from -6 to +6 of the glycosylated Asn residue found in 32 native *C. jejuni* glycoproteins. The analysis is reported in Kowarik et al. EMBO J. 2006; 25(9): 1957-66. The height of the box reflects the frequency of the amino acid residues in the naturally occurring consensus sequences. The Asn residue, site of glycosylation and the Asp and Thr residues in position -2 and +2 respectively, demonstrated crucial for an efficient glycosylation, are reported in bold red in a grey box. The amino acid residues in position -3, -1, +1, +3 and +4, respectively, represented in bold black in grey boxes, were selected for the design of the four consensus sequences (B).

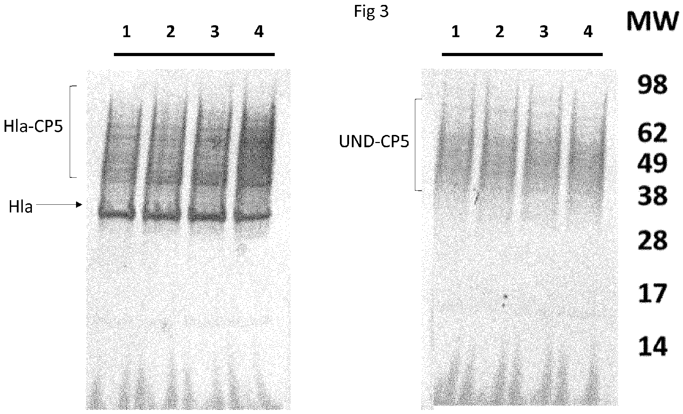

**Figure 3: Efficacy of bioconjugation of the newly designed carriers assessed by Western blot.** The periplasmic fractions prepared from *E. coli* engineered for the expression of Hla-i-CP5, Hla-v-CP5 and Hla-s-CP5 (lanes 1-3, left panel) were analyzed by Western blot using a rabbit anti-Hla-CP5 serum. The levels of expression were compared to the optimized Hla bearing the consensus sequence KDQNRTK which is not compatible with the MS analysis (lane 4, left panel). As negative control the Western blot analysis of the periplasmic fractions prepared from the respective strains that do not express Hla are reported (lanes 1-4, right panel). The positive signal observed might be related to the reaction intermediate undecaprenyl-linked CP5 molecules, produced and assembled during the process.

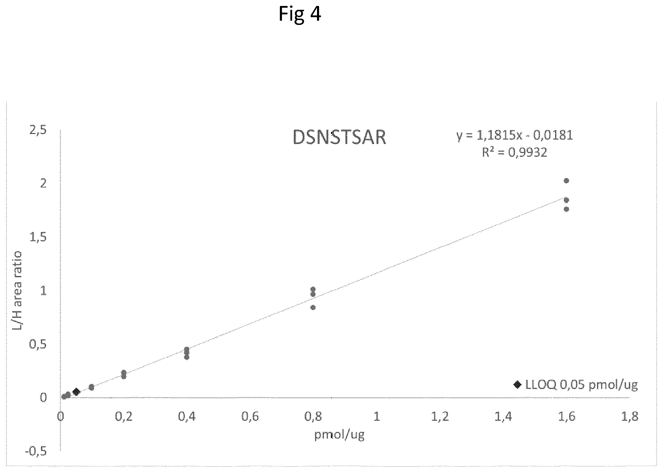

**Figure 4: Dose-response linearity curve of PTP-s.** To build up the calibration curve, on y axes are plotted the L/H area ratios responses determined by spiking in 50 $\mu$g of *E. coli* periplasmic fraction a fixed amount of heavy forms of PTPs (0.1 pmol/$\mu$g) and scalar concentration of light PTPs (ranging from 0.0125 to 1.6 pmol/$\mu$g, x axes), before the trypsin digestion. The LLOQ for each PTP was set as the lowest concentration point on the fitted curve with an accuracy deviation $\leq$20%, and was shown to be 0.05 pmoles/ug of periplasmic proteins for each peptide.

## DETAILED DESCRIPTION OF THE INVENTION

## DEFINITIONS

As used herein, the term "carrier protein" refers to a protein covalently attached to a polysaccharide antigen (e.g. saccharide antigen) to create a conjugate (e.g. bioconjugate). A carrier protein activates T-cell mediated immunity in relation to the polysaccharide antigen to which it is conjugated.

ClfA: clumping factor A from a staphylococcal bacterium, in particular *S. aureus.*

CRM197: non-toxic mutant of diphtheria toxin.

EPA: exotoxin A of *Pseudomonas aeruginosa.*

Hla: Haemolysin A, also known as alpha toxin, from a staphylococcal bacterium, in particular *S. aureus.*

Hcp1: Protein Hcp1 from *Pseudomonas aeruginosa*

MBP: Maltose/maltodextrin binding protein from *Escherichia coli.*

MtrE: Membrane Transporter E from *Neisseria gonorrhoeae.*

PspA, phage shock protein A from *Escherichia coli.*

CP: Capsular polysaccharide.

As used herein, the term "bioconjugate" refers to conjugate between a protein (*e.g.* a carrier protein) and an antigen (e.g. a saccharide) prepared in a host cell background, wherein host cell machinery links the antigen to the protein *(e.g.* N-links). Usually, in a bioconjugate the polysaccharide is linked to asparagine via N-acetylglucosamine.

As used herein, the term "glycosite" refers to an amino acid sequence recognized by a bacterial oligosaccharyltransferase, e.g. PgIB of *C. jejuni.* The minimal consensus sequence for PgIB is D/E-X-N-Z-S/T (SEQ ID NO: 17), while an extended consensus sequence K-D/E-X-N-Z-S/T-K (SEQ ID NO: 18) has also been defined. Exemplary and alternative glycosite sequences are described herein.

Any amino acid apart from proline (pro, P): refers to an amino acid selected from the group consisting of alanine (ala, A), arginine (arg, R), asparagine (asn, N), aspartic acid (asp,D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), serine (ser, S), threonine (thr, T), tryptophan (trp, W), tyrosine (tyr, Y), valine (val, V).

As used herein, the term "effective amount," in the context of administering a therapy (*e.g.* an immunogenic composition or vaccine of the invention) to a subject refers to the amount of a therapy which has a prophylactic and/or therapeutic effect(s).

As used herein, the term "subject" refers to an animal, in particular a mammal such as a primate (e.g. human).

As used herein, reference to a percentage sequence identity between two amino or nucleic acid sequences means that, when aligned, that percentage of amino acids or bases are the same in comparing the two sequences. This alignment and the percent homology or sequence identity can be determined using software programs known in the art, for example those described in section 7.7.18 of Current Protocols in Molecular Biology (F.M. Ausubel et al., eds., 1987, Supplement 30). A preferred alignment is determined by the Smith-Waterman homology search algorithm using an affine gap search with a gap open penalty of 12 and a gap extension penalty of 2, BLOSUM matrix of 62. The Smith-Waterman homology search algorithm is disclosed in Smith & Waterman (1981) Adv. Appl. Math. 2: 482-489. Percentage identity to any particular sequence (*e.g.* to a particular SEQ ID) is ideally calculated over the entire length of that sequence. The percentage sequence identity between two sequences of different lengths is preferably calculated over the length of the longer sequence. Global or local alignments may be used. Preferably, a global alignment is used.

As used herein, the term "purifying" or "purification" of a fusion protein or protein of interest, or conjugate (eg bioconjugate) thereof, means separating it from one or more contaminants. A contaminant is any material that is different from said fusion protein or protein of interest, or conjugate (eg bioconjugate) thereof. Contaminants may be, for example, cell debris, nucleic acid, lipids, proteins other than the fusion protein or protein of interest, polysaccharides and other cellular components.

A "recombinant" polypeptide is one which has been produced in a host cell which has been transformed or transfected with nucleic acid encoding the polypeptide, or produces the polypeptide as a result of homologous recombination.

As used herein, the term "conservative amino acid substitution" involves substitution of a native amino acid residue with a non-native residue such that there is little or no effect on the size, polarity, charge, hydrophobicity, or hydrophilicity of the amino acid residue at that position, and without resulting in decreased immunogenicity. For example, these may be substitutions within the following groups: valine, glycine; glycine, alanine; valine, isoleucine, leucine; aspartic acid, glutamic acid; asparagine, glutamine; serine, threonine; lysine, arginine; and phenylalanine, tyrosine. Conservative amino acid modifications to the sequence of a polypeptide (and the corresponding modifications to the encoding nucleotides) may produce polypeptides having functional and chemical characteristics similar to those of a parental polypeptide.

As used herein, the term "deletion" is the removal of one or more amino acid residues from the protein sequence. Typically, no more than about from 1 to 6 residues (e.g. 1 to 4 residues) are deleted at any one site within the protein molecule.

As used herein, the term "insertion" is the addition of one or more non-native amino acid residues in the protein sequence. Typically, no more than about from 1 to 6 residues (e.g. 1 to 4 residues) are inserted at any one site within the protein molecule.

As used herein, the term 'comprising' indicates that other components in addition to those named may be present, whereas the term 'consisting of' indicates that other components are not present, or not present in detectable amounts. The term 'comprising' naturally includes the term consisting of'.

Carrier proteins

Conjugation of T-independent antigens such as saccharides to carrier proteins has long been established as a way of enabling T-cell help to become part of the immune response for a normally T-independent antigen. In this way, an immune response can be enhanced by allowing the development of immune memory and boostability of the response. The carrier protein turns the T-independent saccharide antigen into a T-dependent antigen capable of triggering an immune memory response. Successful conjugate vaccines which have been developed by conjugating bacterial capsular saccharides to carrier proteins are known in the art; carrier proteins which have been widely used in commercialised vaccines include tetanus toxoid, diphtheria toxoid, CRM197 and protein D from *Haemophilus influenzae.* CRM197 is currently used in the *Streptococcus pneumoniae* capsular polysaccharide conjugate vaccine PREVENAR™ (Pfizer) and protein D, tetanus toxoid and diphtheria toxoid are currently used as carriers for capsular polysaccharides in the *Strep-*

*tococcus pneumoniae* capsular polysaccharide conjugate vaccine SYNFLORIX™ (GlaxoSmithKline). Other carrier proteins known in the art include EPA (exotoxin A of *P. aeruginosa*) for *Staphlyococcus aureus* serotype 5 and 8 capsular polysaccharides (Wacker et al., 2014, J Infect. Dis. 209:1551-1561).

It is also possible to use as carrier proteins which represent protein antigens from the same organism as the conjugates polysaccharide, in order to increase the protective capacity of the conjugate. For example, the *S. aureus* protein antigens Hla have successfully been used as a carrier protein for *S. aureus* capsular polysaccharide. Vaccination with Hla-CP5 and ClfA-CP8 bioconjugates was able to induce functional antibodies to both the capsular polysaccharide and protein antigens, and confer protection from S. aureus infection in animal models, as described in in WO2019/121924, WO2019/121926 and PCT/EP2019/053463. Thus, any protein antigen could be a candidate for use as a carrier protein in a polysaccharide conjugate vaccine. Preferably, said protein antigen would be from the same organism as the polysaccharide. However, it would also be possible to use a protein antigen from a different organism, for example to confer protection against multiple pathogens.

Exemplary carrier proteins which may be used with the present invention are described below.

*EPA: exotoxin A of Pseudomonas aeruginosa.*

In an embodiment, the carrier protein is exotoxin A from *Pseudomonas aeruginosa* (EPA). Said EPA may comprise the amino acid sequence of SEQ ID NO: 1 or an amino acid sequence at least 80%, 85%, 90%, 92%, 95%, 96%, 97%, 98% or 99% identical to SEQ ID NO: 1.

Accordingly, there is provided in one aspect of the present invention, a modified EPA protein comprising an amino acid sequence of SEQ ID NO: 1 or an amino acid sequence at least 80%, 85%, 90%, 92%, 95%, 96%, 97%, 98% or 99% identical to SEQ ID NO: 1, modified in that it comprises one or more consensus sequence(s) comprising or consisting of the amino acid sequence $K/R-(Z)_{0-9}-D/EX-N-Y-S/T-(Z)_{0-9}-K/R$ (SEQ ID NO : 46) as defined above; for example one or more consensus sequence(s) having the amino acid sequence $K/R-D/E-X_1-N-X_2-S/T-Z_1-Z_2-K/R$ (SEQ ID NO 19), wherein $X_1$ and $X_2$ are independently any amino acid apart from proline, and wherein $Z_1$ and $Z_2$ are not lysine or arginine. In an embodiment, said consensus sequence comprises or consists of the amino acid sequence of SEQ ID NO: 20. In an embodiment, said consensus sequence comprises or consists of the amino acid sequence of any one of SEQ ID Nos: 42-45. In a preferred embodiment, said consensus sequence comprises or consists of the amino acid sequence of any one of SEQ ID Nos: 42-44.

The EPA protein may be further modified in that it comprises a detoxifying mutation, for example L to V substitution at the amino acid position corresponding to position L552 of SEQ ID NO: 1, and/or deletion of E553 of SEQ ID NO: 1, or at equivalent positions within an amino acid sequence at least 80%, 85%, 90%, 92%, 95%, 96%, 97%, 98% or 99% identical to SEQ ID NO: 1 (e.g. SEQ ID NO: 2); and/or one or more amino acids have been substituted by one or more consensus sequence(s) $K/R-D/E-X_1-N-X_2-S/T-Z_1-Z_2-K/R$ (SEQ ID NO 19). In an embodiment, said substitution is substitution of A375, A376 or K240 of SEQ ID NO: 1. Hence, the protein of interest may comprise the amino acid sequence of SEQ ID NO: 2 or an amino acid sequence at least 80%, 85%, 90%, 92%, 95%, 96%, 97%, 98% or 99% identical to SEQ ID NO: 2, with insertion or substitution of one or more amino acids with a consensus sequence having an amino acid sequence of SEQ ID NO: 19, 20 or 42-47.

In an embodiment, said modified EPA protein comprises more than one said consensus sequence, for example 2, 3, 4 or 5 consensus sequences. In a preferred embodiment, wherein multiple consensus sequences are present, the consensus sequences have different sequences in order that glycosylation at each individual site may be distinguished.

In an embodiment, the modified EPA protein of the invention may be derived from an amino acid sequence at least 80%, 85%, 90%, 92%, 95%, 96%, 97%, 98% or 99% identical to SEQ ID NO: 1 which is an immunogenic fragment and/or a variant of SEQ ID NO: 1. In an embodiment, the modified EPA protein of the invention may be derived from an immunogenic fragment of SEQ ID NO: 1 or 2 comprising at least about 15, at least about 20, at least about 40, or at least about 60 contiguous amino acid residues of the full length sequence, wherein said polypeptide is capable of eliciting an immune response specific for said amino acid sequence.

In an embodiment, the modified EPA protein of the invention may be derived from an amino acid sequence at least 80%, 85%, 90%, 92%, 95%, 96%, 97%, 98% or 99% identical to SEQ ID NO: 1 which is a variant of SEQ ID NO: 1 which has been modified by the deletion and/or addition and/or substitution of one or more amino acids (e.g. 1, 2, 3, 4, 5, 6, 7, 8, 9, 10, 11 or 12 amino acids). Amino acid substitution may be conservative or non-conservative. In one aspect, amino acid substitution is conservative. Substitutions, deletions, additions or any combination thereof may be combined in a single variant so long as the variant is an immunogenic polypeptide. In an embodiment, the modified EPA protein of the present invention may be derived from a variant in which 1 to 10, 5 to 10, 1 to 5, 1 to 3, 1 to 2 or 1 amino acids are substituted, deleted, or added in any combination.

In an embodiment, the present invention includes fragments and/or variants which comprise a B-cell or T-cell epitope. Such epitopes may be predicted using a combination of 2D-structure prediction, e.g. using the PSIPRED program (from David Jones, Brunel Bioinformatics Group, Dept. Biological Sciences, Brunel University, Uxbridge UB8 3PH, UK) and antigenic index calculated on the basis of the method described by Jameson and Wolf (CABIOS 4:181-186 [1988]).

The term "modified EPA protein" refers to a EPA acid sequence (for example, having a EPA amino acid sequence of SEQ ID NO: 1 or an amino acid sequence at least 80%, 85%, 90%, 91%, 92%, 93%, 94%, 95%, 96%, 97%, 98% or 99% identical to SEQ ID NO: 1), which EPA amino acid sequence may be a wild-type mature EPA amino acid sequence (for example, a wild-type amino acid sequence of SEQ ID NO: 1), which has been modified by the addition, substitution or deletion of one or more amino acids (for example, addition (insertion) of a consensus sequence(s) with amino acid sequence SEQ ID NO:19 or 46; or by substitution of one or more amino acids by a consensus sequence(s) with amino acid sequence SEQ ID NO:19 or 46. The modified EPA protein may also comprise further modifications (additions, substitutions, deletions) as well as the addition or substitution of one or more consensus sequence(s). For example, a signal sequence and/or peptide tag may be added. Additional amino acids at the N and/or C-terminal may be included to aid in cloning (for example, after the signal sequence or before the peptide tag, where present). In an embodiment, the modified EPA protein of the invention may be a non-naturally occurring EPA protein.

In an embodiment of the invention, one or more amino acids (e.g. 1-7 amino acids, e.g. one amino acid) of the modified EPA amino acid sequence (for example, having an amino acid sequence of SEQ ID NO: 1 or a EPA amino acid sequence at least 80%, 85%, 90%, 91%, 92%, 93%, 94%, 95%, 96%, 97%, 98% or 99% identical to SEQ ID NO: 1, e.g. SEQ ID No 2) have been substituted by a consensus sequence(s) with amino acid sequence SEQ ID NO: 46 or SEQ ID NO: 19, for example SEQ ID NO 20 or 42-45 or 47, in particular SEQ ID Nos: 42-44.. For example, a single amino acid in the EPA amino acid sequence (e.g. SEQ ID NO: 1) may be replaced with a said consensus sequence. Alternatively, 2, 3, 4, 5, 6 or 7 amino acids in the EPA amino acid sequence (e.g. SEQ ID NO: 1 or a EPA amino acid sequence at least 80%, 85%, 90%, 92%, 95%, 96%, 97%, 98% or 99% identical to SEQ ID NO: 1) may be replaced with said consensus sequence.

Introduction of a consensus sequence(s) enables the modified EPA protein to be glycosylated. Thus, the present invention also provides a modified EPA protein of the invention wherein the modified EPA protein is glycosylated. In specific embodiments, the consensus sequences are introduced into specific regions of the EPA amino acid sequence, e.g. surface structures of the protein, at the N or C termini of the protein, and/or in loops that are stabilized by disulfide bridges.

A person skilled in the art will understand that when the EPA amino acid sequence is a variant and/or fragment of an amino acid sequence of SEQ ID NO: 2, such as an amino acid sequence at least 80%, 85%, 90%, 92%, 95%, 96%, 97%, 98% or 99% identical to SEQ ID NO: 2, the reference to "between amino acids ..." refers to a the position that would be equivalent to the defined position, if this sequence was lined up with an amino acid sequence of SEQ ID NO: 1 in order to maximise the sequence identity between the two sequences Sequence alignment tools are described above. The addition or deletion of amino acids from the variant and/or fragment of SEQ ID NO: 1 could lead to a difference in the actual amino acid position of the consensus sequence in the mutated sequence, however, by lining the mutated sequence up with the reference sequence, the amino acid in in an equivalent position to the corresponding amino acid in the reference sequence can be identified and hence the appropriate position for addition or substitution of the consensus sequence can be established.

Introduction of such glycosylation sites can be accomplished by, e.g. adding new amino acids to the primary structure of the protein (i.e. the glycosylation sites are added, in full or in part), or by mutating existing amino acids in the protein in order to generate the glycosylation sites (i.e. amino acids are not added to the protein, but selected amino acids of the protein are mutated so as to form glycosylation sites). Those of skill in the art will recognize that the amino acid sequence of a protein can be readily modified using approaches known in the art, e.g. recombinant approaches that include modification of the nucleic acid sequence encoding the protein.

In an embodiment, the modified EPA protein of the invention further comprises a "peptide tag" or "tag", i.e. a sequence of amino acids that allows for the isolation and/or identification of the modified EPA protein. For example, adding a tag to a modified EPA protein of the invention can be useful in the purification of that protein and, hence, the purification of conjugate vaccines comprising the tagged modified EPA protein. Exemplary tags that can be used herein include, without limitation, histidine (HIS) tags. I one embodiment, the tag is a hexa-histidine tag. In certain embodiments, the tags used herein are removable, e.g. removal by chemical agents or by enzymatic means, once they are no longer needed, e.g. after the protein has been purified. Optionally the peptide tag is located at the C-terminus of the amino acid sequence. Optionally the peptide tag comprises six histidine residues at the C-terminus of the amino acid sequence. The peptide tag may be comprise or be preceded by one, two or more additional amino acid residues, for example alanine, serine and/or glycine residues, e.g. GS.

*Hla.*

In an embodiment, the carrier protein is Hla (haemolysin A of *S. aureus,* also known as alpha toxin). Hla has successfully been used as a carrier protein for *S. aureus* capsular polysaccharide, as described above. The mature wild-type amino acid sequence of Hla is given in SEQ ID NO 13.

Accordingly, there is provided in one aspect of the present invention, a modified Hla protein comprising an amino acid sequence of SEQ ID NO: 13 or an amino acid sequence at least 80%, 85%, 90%, 92%, 95%, 96%, 97%, 98% or 99% identical to SEQ ID NO: 13, modified in that it comprises one or more consensus sequence(s) comprising or consisting of the amino acid sequence K/R-(Z)$_{0-9}$-D/E-X-N-Y-S/T-(Z)$_{0-9}$-K/R (SEQ ID NO : 46) as defined above; for example one or more consensus sequence(s) selected from: K/R-D/E-X$_1$-N-X$_2$-S/T-Z$_1$-Z$_2$-K/R (SEQ ID NO 19), wherein X$_1$ and X$_2$ are independently any amino acid apart from proline, and wherein Z$_1$ and Z$_2$ are not lysine or arginine. In an embodiment, said consensus sequence comprises or consists of the amino acid sequence of SEQ ID NO: 20. In an embodiment, said consensus sequence comprises or consists of the amino acid sequence of any one of SEQ ID Nos: 42-45. In a preferred embodiment, said consensus sequence comprises or consists of the amino acid sequence of any one of SEQ ID Nos: 42-44.

In an embodiment, said modified Hla protein comprises more than one said consensus sequence, for example 2, 3, 4 or 5 consensus sequences. In a preferred embodiment, wherein multiple consensus sequences are present, the consensus sequences have different sequences in order that glycosylation at each individual site may be distinguished.

Because Hla is a toxin, it needs to be detoxified (i.e. rendered non-toxic to a mammal, e.g. human, when provided at a dosage suitable for protection) before it can be administered *in vivo.* A modified Hla protein of the invention may be genetically detoxified (i.e. by mutation). The genetically detoxified sequences may remove undesirable activities such as the ability to form a lipid-bilayer penetrating pore, membrane permeation, cell lysis, and cytolytic activity against human erythrocytes and other cells, in order to reduce toxicity, whilst retaining the ability to induce anti-Hla protective and/or neutralizing antibodies following administration to a human. For example, as described herein, a Hla protein may be altered so that it is biologically inactive whilst still maintaining its immunogenic epitopes. The modified Hla proteins of the invention may be genetically detoxified by one or more point mutations. For example, residues involved in pore formation been implicated in the lytic activity of Hla. In one aspect, the modified Hla proteins of the invention may be detoxified by amino acid substitutions as described in Menzies and Kernodle (Menzies and Kernodle, 1994, Infect Immun 62, 1843-1847), for example substitution of H35, H48, H114 and/or H259 with another amino acid such as lysine. For example, the modified Hla proteins of the invention may comprise at least one amino acid substitution selected from H35L, H114L or H259L, with reference to the amino acid sequence of SEQ ID NO: 13 (or an equivalent position in an amino acid sequence at least 80%, 85%, 90%, 92%, 95%, 96%, 97%, 98% or 99% identical to SEQ ID NO: 13). Preferably, the modified Hla protein comprises the substitution H35L (e.g. SEQ ID NO: 14).

Said modified Hla protein may thus be further modified in that the amino acid sequence comprises a detoxifying mutation, for example an amino acid substitution at position H35 (e.g. H35L) of SEQ ID NO: 13 or at an equivalent position within an amino acid sequence at least 80%, 85%, 90%, 92%, 95%, 96%, 97%, 98% or 99% identical to SEQ ID NO: 13 (e.g. SEQ ID NO: 14 and 15). An alternative detoxifying mutation is replacement of the stem region of the Hla monomer with PSGS, as for example in SEQ ID NO: 16. Exemplary modified sequences are those of SEQ ID NO: 31-34 and 36-39, in particular 31-33 and 36-38.

In an embodiment, said Hla sequence may be alternatively or additionally modified in that the amino acid sequence comprises amino acid substitutions at positions H48 and G122 of SEQ ID NO: 13 or at equivalent positions within an amino acid sequence at least 80%, 85%, 90%, 92%, 95%, 96%, 97%, 98% or 99% identical to SEQ ID NO: 1, wherein said substitutions are respectively H to C and G to C (e.g. SEQ ID NO: 15).

Accordingly, there is provided a modified Hla protein comprising an amino acid sequence of SEQ ID NO: 15 or an amino acid sequence at least 80%, 85%, 90%, 92%, 95%, 96%, 97%, 98% or 99% identical to SEQ ID NO: 15, wherein said modified Hla protein contains the following mutations: H35L, H48C and G122C modified in that the amino acid sequence comprises one or more consensus sequence(s) selected from SEQ ID Nos 19, 20 and 42-45, in particular 42-44, wherein said modified Hla protein contains the following mutations: H35L, H48C and G122C. Exemplary sequence are those of SEQ ID NO: 31-34 and 36-39, in particular 31-33 and 36-38.

These sequences may be modified by addition of a signal sequence and optionally insertion of an N-terminal serine and/or alanine for cloning purposes, as described herein. The sequences may further be modified to contain detoxifying mutations, such as any one or all of the detoxifying mutations described herein. A preferred detoxifying mutation is H35L of SEQ ID No 1 or 2.

In an embodiment, the modified Hla protein of the invention may be derived from an amino acid sequence at least 80%, 85%, 90%, 92%, 95%, 96%, 97%, 98% or 99% identical to SEQ ID NO: 13 which is an immunogenic fragment and/or a variant of SEQ ID NO: 13. In an embodiment, the modified Hla protein of the invention may be derived from an immunogenic fragment of SEQ ID NO: 13, 14 or 15 comprising at least about 15, at least about 20, at least about 40, or at least about 60 contiguous amino acid residues of the full length sequence, wherein said polypeptide is capable of eliciting an immune response specific for said amino acid sequence.

In an embodiment, the modified Hla protein of the invention may be derived from an amino acid sequence at least 80%, 85%, 90%, 92%, 95%, 96%, 97%, 98% or 99% identical to SEQ ID NO: 13 which is a variant of SEQ ID NO: 13 which has been modified by the deletion and/or addition and/or substitution of one or more amino acids (e.g. 1, 2, 3, 4, 5, 6, 7, 8, 9, 10, 11 or 12 amino acids). Amino acid substitution may be conservative or non-conservative. In one aspect, amino acid substitution is conservative. Substitutions, deletions, additions or any combination thereof may be combined in a single variant so long as the variant is an immunogenic polypeptide. In an embodiment, the modified Hla protein of the present invention may be derived from a variant in which 1 to 10, 5 to 10, 1 to 5, 1 to 3, 1 to 2 or 1 amino acids are substituted, deleted, or added in any combination. For example, the modified Hla protein of the invention may be derived from an amino acid sequence which is a variant of any one of SEQ ID NOs. 13-16 in that it has one or two additional amino acids at the N terminus, for example an initial N-terminal SA (e.g. SEQ ID NO: 36-39). The modified Hla protein may additionally or alternatively have one or more additional amino acids at the C terminus, for example 1, 2, 3, 4, 5, or 6 amino acids. Such additional amino acids may include a peptide tag to assist in purification, and include for example GSHRHR (e.g. SEQ ID NOs 36-39).

In an embodiment, the present invention includes fragments and/or variants which comprise a B-cell or T-cell epitope. Such epitopes may be predicted using a combination of 2D-structure prediction, e.g. using the PSIPRED program (from David Jones, Brunel Bioinformatics Group, Dept. Biological Sciences, Brunel University, Uxbridge UB8 3PH, UK) and antigenic index calculated on the basis of the method described by Jameson and Wolf (CABIOS 4:181-186 [1988]).

The term "modified Hla protein" refers to a Hla acid sequence (for example, having a Hla amino acid sequence of SEQ ID NO: 13 or an amino acid sequence at least 80%, 85%, 90%, 91%, 92%, 93%, 94%, 95%, 96%, 97%, 98% or 99% identical to SEQ ID NO: 13), which Hla amino acid sequence may be a wild-type mature Hla amino acid sequence (for example, a wild-type amino acid sequence of SEQ ID NO: 13), which has been modified by the addition, substitution or deletion of one or more amino acids (for example, substitution of H48 and G122 of SEQ ID NO: 13 with cysteine, substitution of H35 of SEQ ID NO: 1 with lysine, addition (insertion) of a consensus sequence(s) with amino acid sequence SEQ ID NO:19 or 46; or by substitution of one or more amino acids by a consensus sequence(s) a consensus sequence(s) with amino acid sequence SEQ ID NO:19 or 46. The modified Hla protein may also comprise further modifications (additions, substitutions, deletions) as well as the addition or substitution of one or more consensus sequence(s). For example, a signal sequence and/or peptide tag may be added. Additional amino acids at the N and/or C-terminal may be included to aid in cloning (for example, after the signal sequence or before the peptide tag, where present). In an embodiment, the modified Hla protein of the invention may be a non-naturally occurring Hla protein.

In an embodiment of the invention, one or more amino acids (e.g. 1-7 amino acids, e.g. one amino acid) of the modified Hla amino acid sequence (for example, having an amino acid sequence of SEQ ID NO: 13 or a Hla amino acid sequence at least 80%, 85%, 90%, 91%, 92%, 93%, 94%, 95%, 96%, 97%, 98% or 99% identical to SEQ ID NO: 13, e.g. SEQ ID Nos 14-16) have been substituted by a consensus sequence(s) with amino acid sequence SEQ ID NO: 19 or 46, for example SEQ ID NO 20 or 42-44 or 47, in particular SEQ ID Nos: 42-44.. For example, a single amino acid in the Hla amino acid sequence (e.g. SEQ ID NO: 13) may be replaced with a said consensus sequence (e.g. SEQ ID NOs: 30-39). In an embodiment, said substituted amino acid is at the position corresponding to position K131 of SEQ ID NO: 13. Alternatively, 2, 3, 4, 5, 6 or 7 amino acids in the Hla amino acid sequence (e.g. SEQ ID NO: 13 or a Hla amino acid sequence at least 80%, 85%, 90%, 92%, 95%, 96%, 97%, 98% or 99% identical to SEQ ID NO: 13) may be replaced with said consensus sequence.

Introduction of a consensus sequence(s) enables the modified Hla protein to be glycosylated. Thus, the present invention also provides a modified Hla protein of the invention wherein the modified Hla protein is glycosylated. In specific embodiments, the consensus sequences are introduced into specific regions of the Hla amino acid sequence, e.g. surface structures of the protein, at the N or C termini of the protein, and/or in loops that are stabilized by disulfide bridges. In an aspect of the invention, the position of the consensus sequence(s) provides improved glycosylation, for example increased yield. In an embodiment, a consensus sequence has been added or substituted for one or more amino acid residues or in place of amino acid residue K131 of SEQ ID NO: 13 or in an equivalent position in an amino acid sequence at least 80%, 85%, 90%, 92%, 95%, 96%, 97%, 98% or 99% identical to SEQ ID NO: 13 (e.g. in an equivalent position in the amino acid sequence of SEQ ID Nos: 14-16), e.g. SEQ ID Nos: 30-39.

A person skilled in the art will understand that when the Hla amino acid sequence is a variant and/or fragment of an amino acid sequence of SEQ ID NO: 2, such as an amino acid sequence at least 80%, 85%, 90%, 92%, 95%, 96%, 97%, 98% or 99% identical to SEQ ID NO: 2, the reference to "between amino acids ..." refers to a the position that would be equivalent to the defined position, if this sequence was lined up with an amino acid sequence of SEQ ID NO: 1 in order to maximise the sequence identity between the two sequences Sequence alignment tools are described above. The addition or deletion of amino acids from the variant and/or fragment of SEQ ID NO: 13 could lead to a difference in the actual amino acid position of the consensus sequence in the mutated sequence, however, by lining the mutated sequence up with the reference sequence, the amino acid in in an equivalent position to the corresponding amino acid in the reference sequence can be identified and hence the appropriate position for addition or substitution of the consensus sequence can be established.

Introduction of such glycosylation sites can be accomplished by, e.g. adding new amino acids to the primary structure of the protein (i.e. the glycosylation sites are added, in full or in part), or by mutating existing amino acids in the protein in order to generate the glycosylation sites (i.e. amino acids are not added to the protein, but selected amino acids of the protein are mutated so as to form glycosylation sites). Those of skill in the art will recognize that the amino acid sequence of a protein can be readily modified using approaches known in the art, e.g. recombinant approaches that include modification of the nucleic acid sequence encoding the protein. Thus, in an embodiment, the present invention provides a modified Hla protein having an amino acid sequence wherein the amino acids corresponding to H48 and G122 of SEQ ID NO 13 or equivalent positions in an Hla amino acid sequence at least 80%, 85%, 90%, 92%, 95%, 96%, 97%, 98% or 99% identical to SEQ ID NO: 13 have been substituted by cysteine, and wherein a glycosylation site has been recombinantly introduced into the Hla amino acid sequence of SEQ ID NO: 13 or a Hla amino acid sequence at least 80%, 85%, 90%, 92%, 95%, 96%, 97%, 98% or 99% identical to SEQ ID NO: 13.

In an embodiment, the modified Hla protein of the invention further comprises a "peptide tag" or "tag", i.e. a sequence of amino acids that allows for the isolation and/or identification of the modified Hla protein. For example, adding a tag to a modified Hla protein of the invention can be useful in the purification of that protein and, hence, the purification of conjugate vaccines comprising the tagged modified Hla protein. Exemplary tags that can be used herein include, without limitation, histidine (HIS) tags. I one embodiment, the tag is a hexa-histidine tag. In another embodiment, the tag is a HR tag, for example an HRHR tag. In certain embodiments, the tags used herein are removable, e.g. removal by chemical agents or by enzymatic means, once they are no longer needed, e.g. after the protein has been purified. Optionally the peptide tag is located at the C-terminus of the amino acid sequence. Optionally the peptide tag comprises six histidine residues at the C-terminus of the amino acid sequence. Optionally the peptide tag comprises four HR residues (HRHR) at the C-terminus of the amino acid sequence. The peptide tag may be comprise or be preceded by one, two or more additional amino acid residues, for example alanine, serine and/or glycine residues, e.g. GS. Exemplary such sequences are SEQ ID Nos: 36-39.

In an embodiment, the modified Hla protein of the invention comprises a signal sequence which is capable of directing the carrier protein to the periplasm of a host cell (e.g. bacterium). In a specific embodiment, the signal sequence is from S. flexneriflagellin (FlgI) [MIKFLSALILLLVTTAAQA (SEQ ID NO: 21)]. In other embodiments, the signal sequence is from *E. coli* outer membrane porin A (OmpA) [MKKTAIAIAVALAGFATVAQA (SEQ ID NO: 22)], *E. coli* maltose binding protein (MalE) [MKIKTGARILALSALTTMMFSASALA (SEQ ID NO: 23)], *Pectobacterium carotovorum* pectate lyase (PelB) [MKYLLPTAAAGLLLLAAQPAMA (SEQ ID NO: 24], heat labile *E. coli* enterotoxin LTIIb [MSFKKIIKAFVIMAALVSVQAHA (SEQ ID NO: 25)], *Bacillus subtilis* endoxylanase XynA [MFKFKKKFLVGLTAAFMSISMFSATASA (SEQ ID NO: 26)], *E. coli* DsbA [MKKIWLALAGLVLAFSASA (SEQ ID NO: 27)], TolB [MKQALRVAFGFLILWASVLHA (SEQ ID NO: 28)] or *S. agalactiae* SipA [MKMNKKVLLTSTMAASLLSVASVQAS (SEQ ID NO: 29)]. In an embodiment, the signal sequence has an amino acid sequence at least 80%, 85%, 90%, 92%, 95%, 96%, 97%, 98%, 99% or 100% identical to a SEQ ID NO: 21-29. In one aspect, the signal sequence has an amino acid sequence at least 80%, 85%, 90%, 95%, 96%, 97%, 98%, 99% or 100% identical to *E. coli* flagellin signal sequence (FlgI) [MIKFLSALILLLVTTAAQA (SEQ ID NO: 13)]. Exemplary modified Hla sequences comprising a signal sequence are SEQ ID NOs: 35-39.

In an embodiment, a serine and/or alanine residue is added between the signal sequence and the start of the sequence of the mature protein, e.g.SA or S, preferably S. Such a reside or residues have the advantage of leading to more efficient cleavage of the leader sequence.

*ClfA: Clumping factor A from Staphylococcus aureus*

In an embodiment, the carrier protein is clumping factor A (ClfA) from a staphylococcal bacterium, in particular *S. aureus.* ClfA has been used as carrier protein for S aureus capsular polysaccharide (CP8) and the ClfA-CP8 conjugate was able to induce functional antibodies to both ClfA and CP8, and had protective effect in animal models. ClfA contains a 520 amino acid N-terminal A domain (the Fibrinogen Binding Region), which comprises three separately folded subdomains N1, N2 and N3. The A domain is followed by a serine-aspartate dipeptide repeat region and a cell wall-and membrane-spanning region, which contains the LPDTG-motif for sortase-promoted anchoring to the cell wall. When used as an antigen or carrier protein, only the N1-N3 (SEQ ID NO: 10) or N2/N3 (SEQ ID No: 11) domains are used.

Said ClfA may thus comprise the amino acid sequence of SEQ ID NO: 10 or 11 or an amino acid sequence at least 80%, 85%, 90%, 92%, 95%, 96%, 97%, 98% or 99% identical to SEQ ID NO: 10 or 11.

The ClfA protein may be further rmodified to reduce its fibrinogen binding activity. Thus the ClfA protein may further comprise at least one amino acid substitution selected from P116 to S and Y118 to A with reference to the amino acid sequence of SEQ ID NO: 11 (corresponding to positions P336 and Y338 in the sequence of SEQ ID NO: 10) or an equivalent position in an amino acid sequence at least 80%, 85%, 90%, 92%, 95%, 96%, 97%, 98% or 99% identical to SEQ ID NO: 11.

Accordingly, there is provided in one aspect of the present invention, a modified ClfA protein comprising an amino acid sequence of SEQ ID NOs: 10, 11 or 12 or an amino acid sequence at least 80%, 85%, 90%, 92%, 95%, 96%, 97%, 98% or 99% identical to SEQ ID NOs: 10, 11 or 12, modified in that it comprises one or more consensus sequence(s) comprising or consisting of the amino acid sequence K/R-$(Z)_{0-9}$-D/E-X-N-Y-S/T-$(Z)_{0-9}$-K/R (SEQ ID NO : 46) as defined above; for example one or more consensus sequence(s) having the amino acid sequence K/R-D/E-$X_1$-N-$X_2$-S/T-$Z_1$-$Z_2$-K/R (SEQ ID NO 19), wherein $X_1$ and $X_2$ are independently any amino acid apart from proline, and wherein

$Z_1$ and $Z_2$.are not lysine or arginine. In an embodiment, said consensus sequence comprises or consists of the amino acid sequence of SEQ ID NO: 20. In an embodiment, said consensus sequence comprises or consists of the amino acid sequence of any one of SEQ ID Nos: 42-45. In a preferred embodiment, said consensus sequence comprises or consists of the amino acid sequence of any one of SEQ ID Nos: 42-44.

In an embodiment, said modified ClfA protein comprises more than one said consensus sequence, for example 2, 3, 4 or 5 consensus sequences. In a preferred embodiment, wherein multiple consensus sequences are present, the consensus sequences have different sequences in order that glycosylation at each individual site may be distinguished.

In an embodiment, the modified ClfA protein of the invention may be derived from an amino acid sequence at least 80%, 85%, 90%, 92%, 95%, 96%, 97%, 98% or 99% identical to SEQ ID NO:s: 10, 11 or 12 which is an immunogenic fragment and/or a variant of SEQ ID Nos: 10, 11 or 12. In an embodiment, the modified ClfA protein of the invention may be derived from an immunogenic fragment of SEQ ID Nos: 10, 11 or 12 comprising at least about 15, at least about 20, at least about 40, or at least about 60 contiguous amino acid residues of the full length sequence, wherein said polypeptide is capable of eliciting an immune response specific for said amino acid sequence.

In an embodiment, the modified ClfA protein of the invention may be derived from an amino acid sequence at least 80%, 85%, 90%, 92%, 95%, 96%, 97%, 98% or 99% identical to SEQ ID Nos: 10, 11 or 12 which is a variant of SEQ ID Nos: 10, 11 or 12 which has been modified by the deletion and/or addition and/or substitution of one or more amino acids (e.g. 1, 2, 3, 4, 5, 6, 7, 8, 9, 10, 11 or 12 amino acids). Amino acid substitution may be conservative or non-conservative. In one aspect, amino acid substitution is conservative. Substitutions, deletions, additions or any combination thereof may be combined in a single variant so long as the variant is an immunogenic polypeptide. In an embodiment, the modified ClfA protein of the present invention may be derived from a variant in which 1 to 10, 5 to 10, 1 to 5, 1 to 3, 1 to 2 or 1 amino acids are substituted, deleted, or added in any combination.

In an embodiment, the present invention includes fragments and/or variants which comprise a B-cell or T-cell epitope. Such epitopes may be predicted using a combination of 2D-structure prediction, e.g. using the PSIPRED program (from David Jones, Brunel Bioinformatics Group, Dept. Biological Sciences, Brunel University, Uxbridge UB8 3PH, UK) and antigenic index calculated on the basis of the method described by Jameson and Wolf (CABIOS 4:181-186 [1988]).

The term "modified ClfA protein" refers to a ClfA amino acid sequence (for example, having a ClfA amino acid sequence of SEQ ID NO: 10, 11 or 12 or an amino acid sequence at least 80%, 85%, 90%, 91%, 92%, 93%, 94%, 95%, 96%, 97%, 98% or 99% identical to SEQ ID NO: 10, 11 or 12), which ClfA amino acid sequence has been modified by the addition, substitution or deletion of one or more amino acids (for example, addition (insertion) of a consensus sequence(s) with amino acid sequence SEQ ID NO:19 or 46; or by substitution of one or more amino acids by a consensus sequence(s) with amino acid sequence SEQ ID NO:19 or 46). The modified ClfA protein may also comprise further modifications (additions, substitutions, deletions) as well as the addition or substitution of one or more consensus sequence(s). For example, a signal sequence and/or peptide tag may be added. Additional amino acids at the N and/or C-terminal may be included to aid in cloning (for example, after the signal sequence or before the peptide tag, where present). In an embodiment, the modified ClfA protein of the invention may be a non-naturally occurring ClfA protein.

In an embodiment of the invention, one or more amino acids (e.g. 1-7 amino acids, e.g. one amino acid) of the modified ClfA amino acid sequence (for example, having an amino acid sequence of SEQ ID NO: 10, 11 or 12 or a ClfAamino acid sequence at least 80%, 85%, 90%, 91%, 92%, 93%, 94%, 95%, 96%, 97%, 98% or 99% identical to 10, 11 or 12) have been substituted by a consensus sequence(s) with amino acid sequence SEQ ID NO: 19 or 46, for example SEQ ID NO 20 or 42-45 or 47, in particular SEQ ID Nos: 42-44.. For example, a single amino acid in the ClfA amino acid sequence (e.g. SEQ ID NO: 10, 11 or 12) may be replaced with a said consensus sequence. Alternatively, 2, 3, 4, 5, 6 or 7 amino acids in the EPA amino acid sequence (e.g. SEQ ID NO: 10, 11 or 12 or a ClfA amino acid sequence at least 80%, 85%, 90%, 92%, 95%, 96%, 97%, 98% or 99% identical to SEQ ID NO: 10, 11 or 12) may be replaced with said consensus sequence.

Introduction of a consensus sequence(s) enables the modified ClfA protein to be glycosylated. Thus, the present invention also provides a modified ClfA protein of the invention wherein the modified ClfA protein is glycosylated. In specific embodiments, the consensus sequences are introduced into specific regions of the ClfA amino acid sequence, e.g. surface structures of the protein, at the N or C termini of the protein, and/or in loops that are stabilized by disulfide bridges.

A person skilled in the art will understand that when the ClfA amino acid sequence is a variant and/or fragment of an amino acid sequence of SEQ ID NO: 10, 11 or 12, such as an amino acid sequence at least 80%, 85%, 90%, 92%, 95%, 96%, 97%, 98% or 99% identical to SEQ ID NO: 10, 11 or 12, the reference to "between amino acids ..." refers to a the position that would be equivalent to the defined position, if this sequence was lined up with an amino acid sequence of SEQ ID NO: 10, 11 or 12 in order to maximise the sequence identity between the two sequences Sequence alignment tools are described above. The addition or deletion of amino acids from the variant and/or fragment of SEQ ID NO: 10, 11 or 12 could lead to a difference in the actual amino acid position of the consensus sequence in the mutated sequence, however, by lining the mutated sequence up with the reference sequence, the amino acid in in an equivalent position to the corresponding amino acid in the reference sequence can be identified and hence the appropriate position for addition or substitution of the consensus sequence can be established.

Introduction of such glycosylation sites can be accomplished by, e.g. adding new amino acids to the primary structure of the protein (i.e. the glycosylation sites are added, in full or in part), or by mutating existing amino acids in the protein in order to generate the glycosylation sites (i.e. amino acids are not added to the protein, but selected amino acids of the protein are mutated so as to form glycosylation sites). Those of skill in the art will recognize that the amino acid sequence of a protein can be readily modified using approaches known in the art, e.g. recombinant approaches that include modification of the nucleic acid sequence encoding the protein.

In an embodiment, the modified ClfA protein of the invention further comprises a "peptide tag" or "tag", i.e. a sequence of amino acids that allows for the isolation and/or identification of the modified ClfA protein. For example, adding a tag to a modified EPA protein of the invention can be useful in the purification of that protein and, hence, the purification of conjugate vaccines comprising the tagged modified ClfA protein. Exemplary tags that can be used herein include, without limitation, histidine (HIS) tags. I one embodiment, the tag is a hexa-histidine tag. In certain embodiments, the tags used herein are removable, e.g. removal by chemical agents or by enzymatic means, once they are no longer needed, e.g. after the protein has been purified. Optionally the peptide tag is located at the C-terminus of the amino acid sequence. Optionally the peptide tag comprises six histidine residues at the C-terminus of the amino acid sequence. The peptide tag may be comprise or be preceded by one, two or more additional amino acid residues, for example alanine, serine and/or glycine residues, e.g. GS.

*CRM197: non-toxic mutant of diphtheria toxin.*

In an embodiment, the carrier protein is CRM197, a genetically detoxified mutant of diphtheria toxin having a single point mutation G52E compared to diptheria toxin. CRM197 is a widely used and well tested carrier protein which has been used in several commercialised vaccines. The amino acid sequence of DT is shown in SEQ ID NO: 4 and that of CRM197 is shown in SEQ ID NO: 5.

Accordingly, there is provided in one aspect of the present invention, a modified CRM197 protein comprising an amino acid sequence of SEQ ID NO: 5 or an amino acid sequence at least 80%, 85%, 90%, 92%, 95%, 96%, 97%, 98% or 99% identical to SEQ ID NO: 5, modified in that it comprises one or more consensus sequence(s) comprising or consisting of the amino acid sequence K/R-$(Z)_{0-9}$-D/E-X-N-Y-S/T-$(Z)_{0-9}$-K/R (SEQ ID NO : 46) as defined above; for example one or more consensus sequence(s) having the amino acid sequence K/R-D/E-$X_1$-N-$X_2$-S/T-$Z_1$-$Z_2$-K/R (SEQ ID NO 19), wherein $X_1$ and $X_2$ are independently any amino acid apart from proline, and wherein $Z_1$ and $Z_2$ are not lysine or arginine. In an embodiment, said consensus sequence comprises or consists of the amino acid sequence of SEQ ID NO: 20. In an embodiment, said consensus sequence comprises or consists of the amino acid sequence of any one of SEQ ID Nos: 42-45. In a preferred embodiment, said consensus sequence comprises or consists of the amino acid sequence of any one of SEQ ID Nos: 42-44.

In an embodiment, said modified CRM197 protein comprises more than one said consensus sequence, for example 2, 3, 4 or 5 consensus sequences. In a preferred embodiment, wherein multiple consensus sequences are present, the consensus sequences have different sequences in order that glycosylation at each individual site may be distinguished.

In an embodiment, the modified CRM197 protein of the invention may be derived from an amino acid sequence at least 80%, 85%, 90%, 92%, 95%, 96%, 97%, 98% or 99% identical to SEQ ID NO: 5 which is an immunogenic fragment and/or a variant of SEQ ID NO: 5. In an embodiment, the modified CRM197 protein of the invention may be derived from an immunogenic fragment of SEQ ID NO: 5 comprising at least about 15, at least about 20, at least about 40, or at least about 60 contiguous amino acid residues of the full length sequence, wherein said polypeptide is capable of eliciting an immune response specific for said amino acid sequence.

In an embodiment, the modified CRM197 protein of the invention may be derived from an amino acid sequence at least 80%, 85%, 90%, 92%, 95%, 96%, 97%, 98% or 99% identical to SEQ ID NO: 5 which is a variant of SEQ ID NO: 5 which has been modified by the deletion and/or addition and/or substitution of one or more amino acids (e.g. 1, 2, 3, 4, 5, 6, 7, 8, 9, 10, 11 or 12 amino acids). Amino acid substitution may be conservative or non-conservative. In one aspect, amino acid substitution is conservative. Substitutions, deletions, additions or any combination thereof may be combined in a single variant so long as the variant is an immunogenic polypeptide. In an embodiment, the modified CRM197 protein of the present invention may be derived from a variant in which 1 to 10, 5 to 10, 1 to 5, 1 to 3, 1 to 2 or 1 amino acids are substituted, deleted, or added in any combination.

In an embodiment, the present invention includes fragments and/or variants which comprise a B-cell or T-cell epitope. Such epitopes may be predicted using a combination of 2D-structure prediction, e.g. using the PSIPRED program (from David Jones, Brunel Bioinformatics Group, Dept. Biological Sciences, Brunel University, Uxbridge UB8 3PH, UK) and antigenic index calculated on the basis of the method described by Jameson and Wolf (CABIOS 4:181-186 [1988]).

The term "modified CRM197 protein" refers to a CRM197 acid sequence (for example, having a CRM197 amino acid sequence of SEQ ID NO: 5 or an amino acid sequence at least 80%, 85%, 90%, 91%, 92%, 93%, 94%, 95%, 96%, 97%, 98% or 99% identical to SEQ ID NO: 5 which has been modified by the addition, substitution or deletion of one or more amino acids (for example, addition (insertion) of a consensus sequence(s) with amino acid sequence SEQ ID NO:19 or 46; or by substitution of one or more amino acids by a consensus sequence(s) with amino acid sequence SEQ ID NO: 19 or 46. The modified CRM197 protein may also comprise further modifications (additions, substitutions, deletions) as well as the addition or substitution of one or more consensus sequence(s). For example, a signal sequence and/or peptide tag may be added. Additional amino acids at the N and/or C-terminal may be included to aid in cloning (for example, after the signal sequence or before the peptide tag, where present).

In an embodiment of the invention, one or more amino acids (e.g. 1-7 amino acids, e.g. one amino acid) of the modified CRM197 amino acid sequence (for example, having an amino acid sequence of SEQ ID NO: 5) have been substituted by a consensus sequence(s) with amino acid sequence SEQ ID NO:19 or 46, for example SEQ ID NO 20 or 42-45 or 47, in particular SEQ ID Nos: 42-44.. For example, a single amino acid in the CRM197 amino acid sequence (e.g. SEQ ID NO: 5) may be replaced with a said consensus sequence. Alternatively, 2, 3, 4, 5, 6 or 7 amino acids in the CRM197 amino acid sequence (e.g. SEQ ID NO: 5) may be replaced with said consensus sequence.

Introduction of a consensus sequence(s) enables the modified CRM197 protein to be glycosylated. Thus, the present invention also provides a modified CRM197 protein of the invention wherein the modified CRM197 protein is glycosylated. In specific embodiments, the consensus sequences are introduced into specific regions of the CRM197 amino acid sequence, e.g. surface structures of the protein, at the N or C termini of the protein, and/or in loops that are stabilized by disulfide bridges.

A person skilled in the art will understand that when the CRM197 amino acid sequence is a variant and/or fragment of an amino acid sequence of SEQ ID NO: 5, such as an amino acid sequence at least 80%, 85%, 90%, 92%, 95%, 96%, 97%, 98% or 99% identical to SEQ ID NO: 5, the reference to "between amino acids ..." refers to a the position that would be equivalent to the defined position, if this sequence was lined up with an amino acid sequence of SEQ ID NO: 5 in order to maximise the sequence identity between the two sequences Sequence alignment tools are described above. The addition or deletion of amino acids from the variant and/or fragment of SEQ ID NO: 5 could lead to a difference in the actual amino acid position of the consensus sequence in the mutated sequence, however, by lining the mutated sequence up with the reference sequence, the amino acid in in an equivalent position to the corresponding amino acid in the reference sequence can be identified and hence the appropriate position for addition or substitution of the consensus sequence can be established.

Introduction of such glycosylation sites can be accomplished by, e.g. adding new amino acids to the primary structure of the protein (i.e. the glycosylation sites are added, in full or in part), or by mutating existing amino acids in the protein in order to generate the glycosylation sites (i.e. amino acids are not added to the protein, but selected amino acids of the protein are mutated so as to form glycosylation sites). Those of skill in the art will recognize that the amino acid sequence of a protein can be readily modified using approaches known in the art, e.g. recombinant approaches that include modification of the nucleic acid sequence encoding the protein.

In an embodiment, the modified CRM197 protein of the invention further comprises a "peptide tag" or "tag", i.e. a sequence of amino acids that allows for the isolation and/or identification of the modified CRM197 protein. For example, adding a tag to a modified CRM197 protein of the invention can be useful in the purification of that protein and, hence, the purification of conjugate vaccines comprising the tagged modified CRM197 protein. Exemplary tags that can be used herein include, without limitation, histidine (HIS) tags. In one embodiment, the tag is a hexa-histidine tag. In certain embodiments, the tags used herein are removable, e.g. removal by chemical agents or by enzymatic means, once they are no longer needed, e.g. after the protein has been purified. Optionally the peptide tag is located at the C-terminus of the amino acid sequence. Optionally the peptide tag comprises six histidine residues at the C-terminus of the amino acid sequence. The peptide tag may be comprise or be preceded by one, two or more additional amino acid residues, for example alanine, serine and/or glycine residues, e.g. GS.

*Tetanus toxin*

Tetanus toxin (TT) produced by *C. tetani* cultures is widely used as a carrier after detoxification by formaldehyde inactivation. Fragments of TT which show lower toxicity have also been produced recombinant means.

Accordingly, there is provided in one aspect of the present invention, a modified TT protein comprising an amino acid sequence of SEQ ID NO: 3 or an amino acid sequence at least 80%, 85%, 90%, 92%, 95%, 96%, 97%, 98% or 99% identical to SEQ ID NO: 3, modified in that it comprises one or more consensus sequence(s) comprising or consisting of the amino acid sequence K/R-$(Z)_{0-9}$-D/EX-N-Y-S/T-$(Z)_{0-9}$-K/R (SEQ ID NO : 46) as defined above; for example one or more consensus sequence(s) having the amino acid sequence K/R-D/E-$X_1$-N-$X_2$-S/T-$Z_1$-$Z_2$-K/R (SEQ ID NO 19), wherein $X_1$ and $X_2$ are independently any amino acid apart from proline, and wherein $Z_1$ and $Z_2$ are not lysine or arginine. In an embodiment, said consensus sequence comprises or consists of the amino acid sequence of SEQ ID NO: 20. In an embodiment, said consensus sequence comprises or consists of the amino acid sequence of any one of SEQ ID Nos: 42-45. In a preferred embodiment, said consensus sequence comprises or consists of the amino acid sequence of any one of SEQ ID Nos: 42-44.

In an embodiment, said modified TT protein comprises more than one said consensus sequence, for example 2, 3, 4 or 5 consensus sequences. In a preferred embodiment, wherein multiple consensus sequences are present, the consensus sequences have different sequences in order that glycosylation at each individual site may be distinguished.

In an embodiment, the modified TT protein of the invention may be derived from an amino acid sequence at least 80%, 85%, 90%, 92%, 95%, 96%, 97%, 98% or 99% identical to SEQ ID NO: 3 which is an immunogenic fragment and/or a variant of SEQ ID NO: 3. In an embodiment, the modified TT protein of the invention may be derived from an immunogenic fragment of SEQ ID NO: 3 or 2 comprising at least about 15, at least about 20, at least about 40, or at least about 60 contiguous amino acid residues of the full length sequence, wherein said polypeptide is capable of eliciting an immune response specific for said amino acid sequence.

In an embodiment, the modified TT protein of the invention may be derived from an amino acid sequence at least 80%, 85%, 90%, 92%, 95%, 96%, 97%, 98% or 99% identical to SEQ ID NO: 3 which is a variant of SEQ ID NO: 3 which has been modified by the deletion and/or addition and/or substitution of one or more amino acids (e.g. 1, 2, 3, 4, 5, 6, 7, 8, 9, 10, 11 or 12 amino acids). Amino acid substitution may be conservative or non-conservative. In one aspect, amino acid substitution is conservative. Substitutions, deletions, additions or any combination thereof may be combined in a single variant so long as the variant is an immunogenic polypeptide. In an embodiment, the modified TT protein of the present invention may be derived from a variant in which 1 to 10, 5 to 10, 1 to 5, 1 to 3, 1 to 2 or 1 amino acids are substituted, deleted, or added in any combination.

In an embodiment, the present invention includes fragments and/or variants which comprise a B-cell or T-cell epitope. Such epitopes may be predicted using a combination of 2D-structure prediction, e.g. using the PSIPRED program (from David Jones, Brunel Bioinformatics Group, Dept. Biological Sciences, Brunel University, Uxbridge UB8 3PH, UK) and antigenic index calculated on the basis of the method described by Jameson and Wolf (CABIOS 4:181-186 [1988]).

The term "modified TT protein" refers to a TT acid sequence (for example, having a TT amino acid sequence of SEQ ID NO: 3 or an amino acid sequence at least 80%, 85%, 90%, 91%, 92%, 93%, 94%, 95%, 96%, 97%, 98% or 99% identical to SEQ ID NO: 3), which TT amino acid sequence may be a wild-type mature TT amino acid sequence (for example, a wild-type amino acid sequence of SEQ ID NO: 3), which has been modified by the addition, substitution or deletion of one or more amino acids (for example, addition (insertion) of a consensus sequence(s) with amino acid sequence SEQ ID NO:19 or 46; or by substitution of one or more amino acids by a consensus sequence(s) with amino acid sequence SEQ ID NO:19 or 46. The modified TT protein may also comprise further modifications (additions, substitutions, deletions) as well as the addition or substitution of one or more consensus sequence(s). For example, a signal sequence and/or peptide tag may be added. Additional amino acids at the N and/or C-terminal may be included to aid in cloning (for example, after the signal sequence or before the peptide tag, where present). In an embodiment, the modified TT protein of the invention may be a non-naturally occurring TT protein.

In an embodiment of the invention, one or more amino acids (e.g. 1-7 amino acids, e.g. one amino acid) of the modified TT amino acid sequence (for example, having an amino acid sequence of SEQ ID NO: 3 or a TT amino acid sequence at least 80%, 85%, 90%, 91%, 92%, 93%, 94%, 95%, 96%, 97%, 98% or 99% identical to SEQ ID NO: 3) have been substituted by a consensus sequence(s) with amino acid sequence SEQ ID NO: 19 or 46, for example SEQ ID NO 20 or 42-45 or 47, in particular SEQ ID Nos: 42-44.. For example, a single amino acid in the TT amino acid sequence (e.g. SEQ ID NO: 3) may be replaced with a said consensus sequence. Alternatively, 2, 3, 4, 5, 6 or 7 amino acids in the TT amino acid sequence (e.g. SEQ ID NO: 3 or a TT amino acid sequence at least 80%, 85%, 90%, 92%, 95%, 96%, 97%, 98% or 99% identical to SEQ ID NO: 3) may be replaced with said consensus sequence.

Introduction of a consensus sequence(s) enables the modified TT protein to be glycosylated. Thus, the present invention also provides a modified TT protein of the invention wherein the modified TT protein is glycosylated. In specific embodiments, the consensus sequences are introduced into specific regions of the TT amino acid sequence, e.g. surface structures of the protein, at the N or C termini of the protein, and/or in loops that are stabilized by disulfide bridges.

A person skilled in the art will understand that when the TT amino acid sequence is a variant and/or fragment of an amino acid sequence of SEQ ID NO: 3, such as an amino acid sequence at least 80%, 85%, 90%, 92%, 95%, 96%, 97%, 98% or 99% identical to SEQ ID NO: 3, the reference to "between amino acids ..." refers to a the position that would be equivalent to the defined position, if this sequence was lined up with an amino acid sequence of SEQ ID NO: 3 in order to maximise the sequence identity between the two sequences Sequence alignment tools are described above. The addition or deletion of amino acids from the variant and/or fragment of SEQ ID NO: 3 could lead to a difference in the actual amino acid position of the consensus sequence in the mutated sequence, however, by lining the mutated sequence up with the reference sequence, the amino acid in in an equivalent position to the corresponding amino acid in the reference sequence can be identified and hence the appropriate position for addition or substitution of the consensus sequence can be established.

Introduction of such glycosylation sites can be accomplished by, e.g. adding new amino acids to the primary structure of the protein (i.e. the glycosylation sites are added, in full or in part), or by mutating existing amino acids in the protein in order to generate the glycosylation sites (i.e. amino acids are not added to the protein, but selected amino acids of the protein are mutated so as to form glycosylation sites). Those of skill in the art will recognize that the amino acid sequence of a protein can be readily modified using approaches known in the art, e.g. recombinant approaches that include modification of the nucleic acid sequence encoding the protein.

In an embodiment, the modified TT protein of the invention further comprises a "peptide tag" or "tag", i.e. a sequence of amino acids that allows for the isolation and/or identification of the modified TT protein. For example, adding a tag to a modified TT protein of the invention can be useful in the purification of that protein and, hence, the purification of conjugate vaccines comprising the tagged modified TT protein. Exemplary tags that can be used herein include, without limitation, histidine (HIS) tags. In one embodiment, the tag is a hexa-histidine tag. In certain embodiments, the tags used herein are removable, e.g. removal by chemical agents or by enzymatic means, once they are no longer needed, e.g. after the protein has been purified. Optionally the peptide tag is located at the C-terminus of the amino acid sequence. Optionally the peptide tag comprises six histidine residues at the C-terminus of the amino acid sequence. The peptide tag may be comprise or be preceded by one, two or more additional amino acid residues, for example alanine, serine and/or glycine residues, e.g. GS.

*Hcp1: Protein Hcp1 from Pseudomonas aeruginosa*

In an embodiment, the carrier protein is Hcp1 from *Pseudomonas aeruginosa* (Hcp1). Said Hcp1 may comprise the amino acid sequence of SEQ ID NO: 6 or an amino acid sequence at least 80%, 85%, 90%, 92%, 95%, 96%, 97%, 98% or 99% identical to SEQ ID NO: 6.

Accordingly, there is provided in one aspect of the present invention, a modified Hcp1 protein comprising an amino acid sequence of SEQ ID NO: 6 or an amino acid sequence at least 80%, 85%, 90%, 92%, 95%, 96%, 97%, 98% or 99% identical to SEQ ID NO: 6, modified in that it comprises one or more consensus sequence(s) comprising or consisting of the amino acid sequence K/R-(Z)$_{0-9}$-D/EX-N-Y-S/T-(Z)$_{0-9}$-K/R (SEQ ID NO : 46) as defined above; for example one or more consensus sequence(s) having the amino acid sequence K/R-D/E-X$_1$-N-X$_2$-S/T-Z$_1$-Z$_2$-K/R (SEQ ID NO 19), wherein X$_1$ and X$_2$ are independently any amino acid apart from proline, and wherein Z$_1$ and Z$_2$.are not lysine or arginine. In an embodiment, said consensus sequence comprises or consists of the amino acid sequence of SEQ ID NO: 20. In an embodiment, said consensus sequence comprises or consists of the amino acid sequence of any one of SEQ ID Nos: 42-45. In a preferred embodiment, said consensus sequence comprises or consists of the amino acid sequence of any one of SEQ ID Nos: 42-44.

In an embodiment, said modified Hcp1 protein comprises more than one said consensus sequence, for example 2, 3, 4 or 5 consensus sequences. In a preferred embodiment, wherein multiple consensus sequences are present, the consensus sequences have different sequences in order that glycosylation at each individual site may be distinguished.

In an embodiment, the modified Hcp1 protein of the invention may be derived from an amino acid sequence at least 80%, 85%, 90%, 92%, 95%, 96%, 97%, 98% or 99% identical to SEQ ID NO: 6 which is an immunogenic fragment and/or a variant of SEQ ID NO: 6. In an embodiment, the modified Hcp1 protein of the invention may be derived from an immunogenic fragment of SEQ ID NO: 6 comprising at least about 15, at least about 20, at least about 40, or at least about 60 contiguous amino acid residues of the full length sequence, wherein said polypeptide is capable of eliciting an immune response specific for said amino acid sequence.

In an embodiment, the modified Hcp1 protein of the invention may be derived from an amino acid sequence at least 80%, 85%, 90%, 92%, 95%, 96%, 97%, 98% or 99% identical to SEQ ID NO: 6 which is a variant of SEQ ID NO: 6 which has been modified by the deletion and/or addition and/or substitution of one or more amino acids (e.g. 1, 2, 3, 4, 5, 6, 7, 8, 9, 10, 11 or 12 amino acids). Amino acid substitution may be conservative or non-conservative. In one aspect, amino acid substitution is conservative. Substitutions, deletions, additions or any combination thereof may be combined in a single variant so long as the variant is an immunogenic polypeptide. In an embodiment, the modified Hcp1 protein of the present invention may be derived from a variant in which 1 to 10, 5 to 10, 1 to 5, 1 to 3, 1 to 2 or 1 amino acids are substituted, deleted, or added in any combination.

In an embodiment, the present invention includes fragments and/or variants which comprise a B-cell or T-cell epitope. Such epitopes may be predicted using a combination of 2D-structure prediction, e.g. using the PSIPRED program (from David Jones, Brunel Bioinformatics Group, Dept. Biological Sciences, Brunel University, Uxbridge UB8 3PH, UK) and antigenic index calculated on the basis of the method described by Jameson and Wolf (CABIOS 4:181-186 [1988]).

The term "modified Hcp1 protein" refers to a Hcp1 acid sequence (for example, having a Hcp1 amino acid sequence of SEQ ID NO: 6 or an amino acid sequence at least 80%, 85%, 90%, 91%, 92%, 93%, 94%, 95%, 96%, 97%, 98% or 99% identical to SEQ ID NO: 6), which Hcp1 amino acid has been modified by the addition, substitution or deletion of one or more amino acids (for example, addition (insertion) of a consensus sequence(s) with amino acid sequence SEQ ID NO:19 or 46; or by substitution of one or more amino acids by a consensus sequence(s) with amino acid sequence SEQ ID NO:19 or 46. The modified Hcp1 protein may also comprise further modifications (additions, substitutions, deletions) as well as the addition or substitution of one or more consensus sequence(s). For example, a signal sequence and/or peptide tag may be added. Additional amino acids at the N and/or C-terminal may be included to aid in cloning (for example, after the signal sequence or before the peptide tag, where present). In an embodiment, the modified Hcp1 protein of the invention may be a non-naturally occurring Hcp1 protein.

In an embodiment of the invention, one or more amino acids (e.g. 1-7 amino acids, e.g. one amino acid) of the modified Hcp1 amino acid sequence (for example, having an amino acid sequence of SEQ ID NO: 6 or a Hcp1 amino acid sequence at least 80%, 85%, 90%, 91%, 92%, 93%, 94%, 95%, 96%, 97%, 98% or 99% identical to SEQ ID NO: 6) have been substituted by a consensus sequence(s) with amino acid sequence SEQ ID NO: 19 or 46, for example SEQ ID NO 20 or 42-45 or 47, in particular SEQ ID Nos: 42-44.. For example, a single amino acid in the Hcp1 amino acid sequence (e.g. SEQ ID NO: 6) may be replaced with a said consensus sequence. Alternatively, 2, 3, 4, 5, 6 or 7 amino acids in the HCP6 amino acid sequence (e.g. SEQ ID NO: 6 or a Hcp1 amino acid sequence at least 80%, 85%, 90%, 92%, 95%, 96%, 97%, 98% or 99% identical to SEQ ID NO: 6) may be replaced with said consensus sequence.

Introduction of a consensus sequence(s) enables the modified Hcp1 protein to be glycosylated. Thus, the present invention also provides a modified Hcp1 protein of the invention wherein the modified Hcp1 protein is glycosylated. In specific embodiments, the consensus sequences are introduced into specific regions of the Hcp1 amino acid sequence, e.g. surface structures of the protein, at the N or C termini of the protein, and/or in loops that are stabilized by disulfide bridges.

A person skilled in the art will understand that when the Hcp1 amino acid sequence is a variant and/or fragment of an amino acid sequence of SEQ ID NO: 6, such as an amino acid sequence at least 80%, 85%, 90%, 92%, 95%, 96%, 97%, 98% or 99% identical to SEQ ID NO: 6, the reference to "between amino acids ..." refers to a the position that would be equivalent to the defined position, if this sequence was lined up with an amino acid sequence of SEQ ID NO: 6 in order to maximise the sequence identity between the two sequences Sequence alignment tools are described above. The addition or deletion of amino acids from the variant and/or fragment of SEQ ID NO: 6 could lead to a difference in the actual amino acid position of the consensus sequence in the mutated sequence, however, by lining the mutated sequence up with the reference sequence, the amino acid in in an equivalent position to the corresponding amino acid in the reference sequence can be identified and hence the appropriate position for addition or substitution of the consensus sequence can be established.

Introduction of such glycosylation sites can be accomplished by, e.g. adding new amino acids to the primary structure of the protein (i.e. the glycosylation sites are added, in full or in part), or by mutating existing amino acids in the protein in order to generate the glycosylation sites (i.e. amino acids are not added to the protein, but selected amino acids of the protein are mutated so as to form glycosylation sites). Those of skill in the art will recognize that the amino acid sequence of a protein can be readily modified using approaches known in the art, e.g. recombinant approaches that include modification of the nucleic acid sequence encoding the protein.

In an embodiment, the modified Hcp1 protein of the invention further comprises a "peptide tag" or "tag", i.e. a sequence of amino acids that allows for the isolation and/or identification of the modified Hcp1 protein. For example, adding a tag to a modified Hcp1 protein of the invention can be useful in the purification of that protein and, hence, the purification of conjugate vaccines comprising the tagged modified Hcp1 protein. Exemplary tags that can be used herein include, without limitation, histidine (HIS) tags. In one embodiment, the tag is a hexa-histidine tag. In certain embodiments, the tags used herein are removable, e.g. removal by chemical agents or by enzymatic means, once they are no longer needed, e.g. after the protein has been purified. Optionally the peptide tag is located at the C-terminus of the amino acid sequence. Optionally the peptide tag comprises six histidine residues at the C-terminus of the amino acid sequence. The peptide tag may be comprise or be preceded by one, two or more additional amino acid residues, for example alanine, serine and/or glycine residues, e.g. GS.

*MBP: Maltose/maltodextrin binding protein from Escherichia coli.*

In an embodiment, the carrier protein is exotoxin A from *Pseudomonas aeruginosa* (MBP). Said MBP may comprise the amino acid sequence of SEQ ID NO: 8 or an amino acid sequence at least 80%, 85%, 90%, 92%, 95%, 96%, 97%, 98% or 99% identical to SEQ ID NO: 8.

Accordingly, there is provided in one aspect of the present invention, a modified MBP protein comprising an amino acid sequence of SEQ ID NO: 8 or an amino acid sequence at least 80%, 85%, 90%, 92%, 95%, 96%, 97%, 98% or 99% identical to SEQ ID NO: 8, modified in that it comprises one or more consensus sequence(s) comprising or consisting of the amino acid sequence K/R-$(Z)_{0-9}$-D/EX-N-Y-S/T-$(Z)_{0-9}$-K/R (SEQ ID NO : 46) as defined above; for example one or more consensus sequence(s) having the amino acid sequence K/R-D/E-$X_1$-N-$X_2$-S/T-$Z_1$-$Z_2$-K/R (SEQ ID NO 19), wherein $X_1$ and $X_2$ are independently any amino acid apart from proline, and wherein $Z_1$ and $Z_2$ are not lysine or arginine. In an embodiment, said consensus sequence comprises or consists of the amino acid sequence of SEQ ID NO: 20. In an embodiment, said consensus sequence comprises or consists of the amino acid sequence of any one of SEQ ID Nos: 42-45. In a preferred embodiment, said consensus sequence comprises or consists of the amino acid sequence of any one of SEQ ID Nos: 42-44.

In an embodiment, said modified MBP protein comprises more than one said consensus sequence, for example 2, 3, 4 or 5 consensus sequences. In a preferred embodiment, wherein multiple consensus sequences are present, the consensus sequences have different sequences in order that glycosylation at each individual site may be distinguished.

In an embodiment, the modified MBP protein of the invention may be derived from an amino acid sequence at least 80%, 85%, 90%, 92%, 95%, 96%, 97%, 98% or 99% identical to SEQ ID NO: 8 which is an immunogenic fragment and/or a variant of SEQ ID NO: 8. In an embodiment, the modified MBP protein of the invention may be derived from an immunogenic fragment of SEQ ID NO: 8 comprising at least about 15, at least about 20, at least about 40, or at least about 60 contiguous amino acid residues of the full length sequence, wherein said polypeptide is capable of eliciting an immune response specific for said amino acid sequence.

In an embodiment, the modified MBP protein of the invention may be derived from an amino acid sequence at least 80%, 85%, 90%, 92%, 95%, 96%, 97%, 98% or 99% identical to SEQ ID NO: 8 which is a variant of SEQ ID NO: 8 which has been modified by the deletion and/or addition and/or substitution of one or more amino acids (e.g. 1, 2, 3, 4, 5, 6, 7, 8, 9, 10, 11 or 12 amino acids). Amino acid substitution may be conservative or non-conservative. In one aspect, amino acid substitution is conservative. Substitutions, deletions, additions or any combination thereof may be combined in a single variant so long as the variant is an immunogenic polypeptide. In an embodiment, the modified MBP protein of the present invention may be derived from a variant in which 1 to 10, 5 to 10, 1 to 5, 1 to 3, 1 to 2 or 1 amino acids are substituted, deleted, or added in any combination.

In an embodiment, the present invention includes fragments and/or variants which comprise a B-cell or T-cell epitope. Such epitopes may be predicted using a combination of 2D-structure prediction, e.g. using the PSIPRED program (from David Jones, Brunel Bioinformatics Group, Dept. Biological Sciences, Brunel University, Uxbridge UB8 3PH, UK) and antigenic index calculated on the basis of the method described by Jameson and Wolf (CABIOS 4:181-186 [1988]).

The term "modified MBP protein" refers to a MBP acid sequence (for example, having a MBP amino acid sequence of SEQ ID NO: 8 or an amino acid sequence at least 80%, 85%, 90%, 91%, 92%, 93%, 94%, 95%, 96%, 97%, 98% or 99% identical to SEQ ID NO: 8), which MBP amino acid sequence may be a wild-type mature MBP amino acid sequence (for example, a wild-type amino acid sequence of SEQ ID NO: 8), which has been modified by the addition, substitution or deletion of one or more amino acids (for example, addition (insertion) of a consensus sequence(s) with amino acid sequence SEQ ID NO:19 or 46; or by substitution of one or more amino acids by a consensus sequence(s) with amino acid sequence SEQ ID NO:19 or 46. The modified MBP protein may also comprise further modifications (additions, substitutions, deletions) as well as the addition or substitution of one or more consensus sequence(s). For example, a signal sequence and/or peptide tag may be added. Additional amino acids at the N and/or C-terminal may be included to aid in cloning (for example, after the signal sequence or before the peptide tag, where present). In an embodiment, the modified MBP protein of the invention may be a non-naturally occurring MBP protein.

In an embodiment of the invention, one or more amino acids (e.g. 1-7 amino acids, e.g. one amino acid) of the modified MBP amino acid sequence (for example, having an amino acid sequence of SEQ ID NO: 8 or a MBP amino acid sequence at least 80%, 85%, 90%, 91%, 92%, 93%, 94%, 95%, 96%, 97%, 98% or 99% identical to SEQ ID NO: 8) have been substituted by a consensus sequence(s) with amino acid sequence SEQ ID NO: 19 or 46, for example SEQ ID NO 20 or 42-45 or 47, in particular SEQ ID Nos: 42-44.. For example, a single amino acid in the MBP amino acid sequence (e.g. SEQ ID NO: 8) may be replaced with a said consensus sequence. Alternatively, 2, 3, 4, 5, 6 or 7 amino acids in the MBP amino acid sequence (e.g. SEQ ID NO: 8 or a MBP amino acid sequence at least 80%, 85%, 90%, 92%, 95%, 96%, 97%, 98% or 99% identical to SEQ ID NO: 8) may be replaced with said consensus sequence.

Introduction of a consensus sequence(s) enables the modified MBP protein to be glycosylated. Thus, the present invention also provides a modified MBP protein of the invention wherein the modified MBP protein is glycosylated. In specific embodiments, the consensus sequences are introduced into specific regions of the MBP amino acid sequence, e.g. surface structures of the protein, at the N or C termini of the protein, and/or in loops that are stabilized by disulfide bridges.

A person skilled in the art will understand that when the MBP amino acid sequence is a variant and/or fragment of an amino acid sequence of SEQ ID NO: 8, such as an amino acid sequence at least 80%, 85%, 90%, 92%, 95%, 96%, 97%, 98% or 99% identical to SEQ ID NO: 8, the reference to "between amino acids ..." refers to a the position that would be equivalent to the defined position, if this sequence was lined up with an amino acid sequence of SEQ ID NO: 8 in order to maximise the sequence identity between the two sequences Sequence alignment tools are described above. The addition or deletion of amino acids from the variant and/or fragment of SEQ ID NO: 8 could lead to a difference in the actual amino acid position of the consensus sequence in the mutated sequence, however, by lining the mutated sequence up with the reference sequence, the amino acid in in an equivalent position to the corresponding amino acid in the reference sequence can be identified and hence the appropriate position for addition or substitution of the consensus sequence can be established.

Introduction of such glycosylation sites can be accomplished by, e.g. adding new amino acids to the primary structure of the protein (i.e. the glycosylation sites are added, in full or in part), or by mutating existing amino acids in the protein in order to generate the glycosylation sites (i.e. amino acids are not added to the protein, but selected amino acids of the protein are mutated so as to form glycosylation sites). Those of skill in the art will recognize that the amino acid sequence of a protein can be readily modified using approaches known in the art, e.g. recombinant approaches that include modification of the nucleic acid sequence encoding the protein.

In an embodiment, the modified MBP protein of the invention further comprises a "peptide tag" or "tag", i.e. a sequence of amino acids that allows for the isolation and/or identification of the modified MBP protein. For example, adding a tag to a modified MBP protein of the invention can be useful in the purification of that protein and, hence, the purification of conjugate vaccines comprising the tagged modified MBP protein. Exemplary tags that can be used herein include, without limitation, histidine (HIS) tags. I one embodiment, the tag is a hexa-histidine tag. In certain embodiments, the tags used herein are removable, e.g. removal by chemical agents or by enzymatic means, once they are no longer needed, e.g. after the protein has been purified. Optionally the peptide tag is located at the C-terminus of the amino acid sequence. Optionally the peptide tag comprises six histidine residues at the C-terminus of the amino acid sequence. The peptide tag may be comprise or be preceded by one, two or more additional amino acid residues, for example alanine, serine and/or glycine residues, e.g. GS.

*MtrE: Membrane Transporter E from Neisseria gonorrhoeae.*

In an embodiment, the carrier protein is Membrane Transporter E from *Neisseria gonorrhoeae* (MtrE). Said MtrE may comprise the amino acid sequence of SEQ ID NO: 9 or an amino acid sequence at least 80%, 85%, 90%, 92%, 95%, 96%, 97%, 98% or 99% identical to SEQ ID NO: 9.

Accordingly, there is provided in one aspect of the present invention, a modified MtrE protein comprising an amino acid sequence of SEQ ID NO: 9 or an amino acid sequence at least 80%, 85%, 90%, 92%, 95%, 96%, 97%, 98% or 99% identical to SEQ ID NO: 9, modified in that it comprises one or more consensus sequence(s) comprising or consisting of the amino acid sequence K/R-$(Z)_{0-9}$-D/EX-N-Y-S/T-$(Z)_{0-9}$-K/R (SEQ ID NO : 46) as defined above; for example one or more consensus sequence(s) having the amino acid sequence K/R-D/E-$X_1$-N-$X_2$-S/T-$Z_1$-$Z_2$-K/R (SEQ ID NO 19), wherein $X_1$ and $X_2$ are independently any amino acid apart from proline, and wherein $Z_1$ and $Z_2$ are not lysine or arginine. In an embodiment, said consensus sequence comprises or consists of the amino acid sequence of SEQ ID NO: 20. In an embodiment, said consensus sequence comprises or consists of the amino acid sequence of any one of SEQ ID Nos: 42-45. In a preferred embodiment, said consensus sequence comprises or consists of the amino acid sequence of any one of SEQ ID Nos: 42-44.

In an embodiment, said modified MtrE protein comprises more than one said consensus sequence, for example 2, 3, 4 or 5 consensus sequences. In a preferred embodiment, wherein multiple consensus sequences are present, the consensus sequences have different sequences in order that glycosylation at each individual site may be distinguished.

In an embodiment, the modified MtrE protein of the invention may be derived from an amino acid sequence at least 80%, 85%, 90%, 92%, 95%, 96%, 97%, 98% or 99% identical to SEQ ID NO: 9 which is an immunogenic fragment and/or a variant of SEQ ID NO: 9. In an embodiment, the modified MtrE protein of the invention may be derived from an immunogenic fragment of SEQ ID NO: 9 comprising at least about 15, at least about 20, at least about 40, or at least about 60 contiguous amino acid residues of the full length sequence, wherein said polypeptide is capable of eliciting an immune response specific for said amino acid sequence.

In an embodiment, the modified MtrE protein of the invention may be derived from an amino acid sequence at least 80%, 85%, 90%, 92%, 95%, 96%, 97%, 98% or 99% identical to SEQ ID NO: 9 which is a variant of SEQ ID NO: 9 which has been modified by the deletion and/or addition and/or substitution of one or more amino acids (e.g. 1, 2, 3, 4, 5, 6, 7, 8, 9, 10, 11 or 12 amino acids). Amino acid substitution may be conservative or non-conservative. In one aspect, amino acid substitution is conservative. Substitutions, deletions, additions or any combination thereof may be combined in a single variant so long as the variant is an immunogenic polypeptide. In an embodiment, the modified MtrE protein of the present invention may be derived from a variant in which 1 to 10, 5 to 10, 1 to 5, 1 to 3, 1 to 2 or 1 amino acids are substituted, deleted, or added in any combination.

In an embodiment, the present invention includes fragments and/or variants which comprise a B-cell or T-cell epitope. Such epitopes may be predicted using a combination of 2D-structure prediction, e.g. using the PSIPRED program (from David Jones, Brunel Bioinformatics Group, Dept. Biological Sciences, Brunel University, Uxbridge UB8 3PH, UK) and antigenic index calculated on the basis of the method described by Jameson and Wolf (CABIOS 4:181-186 [1988]).

The term "modified MtrE protein" refers to a MtrE amino acid sequence (for example, having a MtrE amino acid sequence of SEQ ID NO: 9 or an amino acid sequence at least 80%, 85%, 90%, 91%, 92%, 93%, 94%, 95%, 96%, 97%, 98% or 99% identical to SEQ ID NO: 9), which MtrE amino acid sequence may be a wild-type mature MtrE amino acid sequence (for example, a wild-type amino acid sequence of SEQ ID NO: 9), which has been modified by the addition, substitution or deletion of one or more amino acids (for example, addition (insertion) of a consensus sequence(s) with amino acid sequence SEQ ID NO:19 or 46; or by substitution of one or more amino acids by a consensus sequence(s) with amino acid sequence SEQ ID NO:19 or 46. The modified MtrE protein may also comprise further modifications (additions, substitutions, deletions) as well as the addition or substitution of one or more consensus sequence(s). For example, a signal sequence and/or peptide tag may be added. Additional amino acids at the N and/or C-terminal may be included to aid in cloning (for example, after the signal sequence or before the peptide tag, where present). In an embodiment, the modified MtrE protein of the invention may be a non-naturally occurring MtrE protein.

In an embodiment of the invention, one or more amino acids (e.g. 1-7 amino acids, e.g. one amino acid) of the modified MtrE amino acid sequence (for example, having an amino acid sequence of SEQ ID NO: 9 or a MtrE amino acid sequence at least 80%, 85%, 90%, 91%, 92%, 93%, 94%, 95%, 96%, 97%, 98% or 99% identical to SEQ ID NO: 9, e.g. SEQ ID No 9) have been substituted by a consensus sequence(s) with amino acid sequence SEQ ID NO:19 or 46, for example SEQ ID NO 20 or 42-45 or 47, in particular SEQ ID Nos: 42-44. For example, a single amino acid in the MtrE amino acid sequence (e.g. SEQ ID NO: 9) may be replaced with a said consensus sequence. Alternatively, 2, 3, 4, 5, 6 or 7 amino acids in the MtrE amino acid sequence (e.g. SEQ ID NO: 9 or a MtrE amino acid sequence at least 80%, 85%, 90%, 92%, 95%, 96%, 97%, 98% or 99% identical to SEQ ID NO: 9) may be replaced with said consensus sequence.

Introduction of a consensus sequence(s) enables the modified MtrE protein to be glycosylated. Thus, the present invention also provides a modified MtrE protein of the invention wherein the modified MtrE protein is glycosylated. In specific embodiments, the consensus sequences are introduced into specific regions of the MtrE amino acid sequence, e.g. surface structures of the protein, at the N or C termini of the protein, and/or in loops that are stabilized by disulfide bridges.

A person skilled in the art will understand that when the MtrE amino acid sequence is a variant and/or fragment of an amino acid sequence of SEQ ID NO: 9, such as an amino acid sequence at least 80%, 85%, 90%, 92%, 95%, 96%, 97%, 98% or 99% identical to SEQ ID NO: 9, the reference to "between amino acids ..." refers to a the position that would be equivalent to the defined position, if this sequence was lined up with an amino acid sequence of SEQ ID NO: 9 in order to maximise the sequence identity between the two sequences Sequence alignment tools are described above. The addition or deletion of amino acids from the variant and/or fragment of SEQ ID NO: 9 could lead to a difference in the actual amino acid position of the consensus sequence in the mutated sequence, however, by lining the mutated sequence up with the reference sequence, the amino acid in in an equivalent position to the corresponding amino acid in the reference sequence can be identified and hence the appropriate position for addition or substitution of the consensus sequence can be established.

Introduction of such glycosylation sites can be accomplished by, e.g. adding new amino acids to the primary structure of the protein (i.e. the glycosylation sites are added, in full or in part), or by mutating existing amino acids in the protein in order to generate the glycosylation sites (i.e. amino acids are not added to the protein, but selected amino acids of the protein are mutated so as to form glycosylation sites). Those of skill in the art will recognize that the amino acid sequence of a protein can be readily modified using approaches known in the art, e.g. recombinant approaches that include modification of the nucleic acid sequence encoding the protein.

In an embodiment, the modified MtrE protein of the invention further comprises a "peptide tag" or "tag", i.e. a sequence of amino acids that allows for the isolation and/or identification of the modified MtrE protein. For example, adding a tag to a modified MtrE protein of the invention can be useful in the purification of that protein and, hence, the purification of conjugate vaccines comprising the tagged modified MtrE protein. Exemplary tags that can be used herein include, without limitation, histidine (HIS) tags. I one embodiment, the tag is a hexa-histidine tag. In certain embodiments, the tags used herein are removable, e.g. removal by chemical agents or by enzymatic means, once they are no longer needed, e.g. after the protein has been purified. Optionally the peptide tag is located at the C-terminus of the amino acid sequence. Optionally the peptide tag comprises six histidine residues at the C-terminus of the amino acid sequence. The peptide tag may be comprise or be preceded by one, two or more additional amino acid residues, for example alanine, serine and/or glycine residues, e.g. GS.

*PspA, phage shock protein A from Escherichia coli.*

In an embodiment, the carrier protein is phage shock protein A from *Pseudomonas aeruginosa* (PspA). Said PspA may comprise the amino acid sequence of SEQ ID NO: 7 or an amino acid sequence at least 80%, 85%, 90%, 92%, 95%, 96%, 97%, 98% or 99% identical to SEQ ID NO: 7.

Accordingly, there is provided in one aspect of the present invention, a modified PspA protein comprising an amino acid sequence of SEQ ID NO: 7 or an amino acid sequence at least 80%, 85%, 90%, 92%, 95%, 96%, 97%, 98% or 99% identical to SEQ ID NO: 7, modified in that it comprises one or more consensus sequence(s) comprising or consisting of the amino acid sequence K/R-(Z)$_{0-9}$-D/EX-N-Y-S/T-(Z)$_{0-9}$-K/R (SEQ ID NO : 46) as defined above; for example one or more consensus sequence(s) having the amino acid sequence K/R-D/E-X$_1$-N-X$_2$-S/T-Z$_1$-Z$_2$-K/R (SEQ ID NO 19), wherein X$_1$ and X$_2$ are independently any amino acid apart from proline, and wherein Z$_1$ and Z$_2$ are not lysine or arginine. In an embodiment, said consensus sequence comprises or consists of the amino acid sequence of SEQ ID NO: 20. In an embodiment, said consensus sequence comprises or consists of the amino acid sequence of any one of SEQ ID Nos: 42-45. In a preferred embodiment, said consensus sequence comprises or consists of the amino acid sequence of any one of SEQ ID Nos: 42-44.

In an embodiment, said modified PspA protein comprises more than one said consensus sequence, for example 2, 3, 4 or 5 consensus sequences. In a preferred embodiment, wherein multiple consensus sequences are present, the consensus sequences have different sequences in order that glycosylation at each individual site may be distinguished.

In an embodiment, the modified PspA protein of the invention may be derived from an amino acid sequence at least 80%, 85%, 90%, 92%, 95%, 96%, 97%, 98% or 99% identical to SEQ ID NO: 7 which is an immunogenic fragment and/or a variant of SEQ ID NO: 7. In an embodiment, the modified PspA protein of the invention may be derived from an immunogenic fragment of SEQ ID NO: 7 comprising at least about 15, at least about 20, at least about 40, or at least about 60 contiguous amino acid residues of the full length sequence, wherein said polypeptide is capable of eliciting an immune response specific for said amino acid sequence.

In an embodiment, the modified PspA protein of the invention may be derived from an amino acid sequence at least 80%, 85%, 90%, 92%, 95%, 96%, 97%, 98% or 99% identical to SEQ ID NO: 7 which is a variant of SEQ ID NO: 7 which has been modified by the deletion and/or addition and/or substitution of one or more amino acids (e.g. 1, 2, 3, 4, 5, 6, 7, 8, 9, 10, 11 or 12 amino acids). Amino acid substitution may be conservative or non-conservative. In one aspect, amino acid substitution is conservative. Substitutions, deletions, additions or any combination thereof may be combined in a single variant so long as the variant is an immunogenic polypeptide. In an embodiment, the modified PspA protein of the present invention may be derived from a variant in which 1 to 10, 5 to 10, 1 to 5, 1 to 3, 1 to 2 or 1 amino acids are substituted, deleted, or added in any combination.

In an embodiment, the present invention includes fragments and/or variants which comprise a B-cell or T-cell epitope. Such epitopes may be predicted using a combination of 2D-structure prediction, e.g. using the PSIPRED program (from David Jones, Brunel Bioinformatics Group, Dept. Biological Sciences, Brunel University, Uxbridge UB8 3PH, UK) and antigenic index calculated on the basis of the method described by Jameson and Wolf (CABIOS 4:181-186 [1988]).

The term "modified PspA protein" refers to a PspA amino acid sequence (for example, having a PspA amino acid sequence of SEQ ID NO: 7 or an amino acid sequence at least 80%, 85%, 90%, 91%, 92%, 93%, 94%, 95%, 96%, 97%, 98% or 99% identical to SEQ ID NO: 7), which PspA amino acid sequence may be a wild-type mature PspA amino acid sequence (for example, a wild-type amino acid sequence of SEQ ID NO: 7), which has been modified by the addition, substitution or deletion of one or more amino acids (for example, addition (insertion) of a consensus sequence(s) with amino acid sequence SEQ ID NO:19 or 46; or by substitution of one or more amino acids by a consensus sequence(s) with amino acid sequence SEQ ID NO:19 or 46. The modified PspA protein may also comprise further modifications (additions, substitutions, deletions) as well as the addition or substitution of one or more consensus sequence(s). For example, a signal sequence and/or peptide tag may be added. Additional amino acids at the N and/or C-terminal may be included to aid in cloning (for example, after the signal sequence or before the peptide tag, where present). In an embodiment, the modified PspA protein of the invention may be a non-naturally occurring PSPA protein.

In an embodiment of the invention, one or more amino acids (e.g. 1-7 amino acids, e.g. one amino acid) of the modified PspA amino acid sequence (for example, having an amino acid sequence of SEQ ID NO: 7 or a PSPA amino acid sequence at least 80%, 85%, 90%, 91%, 92%, 93%, 94%, 95%, 96%, 97%, 98% or 99% identical to SEQ ID NO: 7) have been substituted by a consensus sequence(s) with amino acid sequence SEQ ID NO:19, for example SEQ ID NO 20 or 42-45, in particular SEQ ID Nos: 42-44.. For example, a single amino acid in the PspA amino acid sequence (e.g. SEQ ID NO: 7) may be replaced with a said consensus sequence. Alternatively, 2, 3, 4, 5, 6 or 7 amino acids in the PspA amino acid sequence (e.g. SEQ ID NO: 7 or a PspA amino acid sequence at least 80%, 85%, 90%, 92%, 95%, 96%, 97%, 98% or 99% identical to SEQ ID NO: 7) may be replaced with said consensus sequence.

Introduction of a consensus sequence(s) enables the modified PspA protein to be glycosylated. Thus, the present invention also provides a modified PspA protein of the invention wherein the modified PspA protein is glycosylated. In specific embodiments, the consensus sequences are introduced into specific regions of the PspA amino acid sequence, e.g. surface structures of the protein, at the N or C termini of the protein, and/or in loops that are stabilized by disulfide bridges.

A person skilled in the art will understand that when the PspA amino acid sequence is a variant and/or fragment of an amino acid sequence of SEQ ID NO: 7, such as an amino acid sequence at least 80%, 85%, 90%, 92%, 95%, 96%, 97%, 98% or 99% identical to SEQ ID NO: 7, the reference to "between amino acids ..." refers to a the position that would be equivalent to the defined position, if this sequence was lined up with an amino acid sequence of SEQ ID NO: 7 in order to maximise the sequence identity between the two sequences. Sequence alignment tools are described above. The addition or deletion of amino acids from the variant and/or fragment of SEQ ID NO: 7 could lead to a difference in the actual amino acid position of the consensus sequence in the mutated sequence, however, by lining the mutated sequence up with the reference sequence, the amino acid in in an equivalent position to the corresponding amino acid in the reference sequence can be identified and hence the appropriate position for addition or substitution of the consensus sequence can be established.

Introduction of such glycosylation sites can be accomplished by, e.g. adding new amino acids to the primary structure of the protein (i.e. the glycosylation sites are added, in full or in part), or by mutating existing amino acids in the protein in order to generate the glycosylation sites (i.e. amino acids are not added to the protein, but selected amino acids of the protein are mutated so as to form glycosylation sites). Those of skill in the art will recognize that the amino acid sequence of a protein can be readily modified using approaches known in the art, e.g. recombinant approaches that include modification of the nucleic acid sequence encoding the protein.

In an embodiment, the modified PspA protein of the invention further comprises a "peptide tag" or "tag", i.e. a sequence of amino acids that allows for the isolation and/or identification of the modified PspA protein. For example, adding a tag to a modified PspA protein of the invention can be useful in the purification of that protein and, hence, the purification of conjugate vaccines comprising the tagged modified PspA protein. Exemplary tags that can be used herein include, without limitation, histidine (HIS) tags. I one embodiment, the tag is a hexa-histidine tag. In certain embodiments, the tags used herein are removable, e.g. removal by chemical agents or by enzymatic means, once they are no longer needed, e.g. after the protein has been purified. Optionally the peptide tag is located at the C-terminus of the amino acid sequence. Optionally the peptide tag comprises six histidine residues at the C-terminus of the amino acid sequence. The peptide tag may be comprise or be preceded by one, two or more additional amino acid residues, for example alanine, serine and/or glycine residues, e.g. GS.

*Signal sequences and other modifications*

In an embodiment, the modified carrier protein of the invention comprises a signal sequence which is capable of directing the protein to the periplasm of a host cell (e.g. bacterium). In a specific embodiment, the signal sequence is from *S. flexneri* flagellin (FlgI) [MIKFLSALILLLVTTAAQA (SEQ ID NO: 21)]. In other embodiments, the signal sequence is from *E. coli* outer membrane porin A (OmpA) [MKKTAIAIAVALAGFATVAQA (SEQ ID NO: 22)], *E. coli* maltose binding protein (MalE) [MKIKTGARILALSALTTMMFSASALA (SEQ ID NO: 23)], *Pectobacterium carotovorum* pectate lyase (PelB) [MKYLLPTAAAGLLLLAAQPAMA (SEQ ID NO: 24], heat labile *E. coli* enterotoxin LTIIb [MSFKKIIKAFVIMAALVSVQAHA (SEQ ID NO: 25)], *Bacillus subtilis* endoxylanase XynA [MFKFKKKFLVGLTAAFMSISMFSATASA (SEQ ID NO: 26)], *E. coli* DsbA [MKKIWLALAGLVLAFSASA (SEQ ID NO: 27)], TolB [MKQALRVAFGFLILWASVLHA (SEQ ID NO: 28)] or *S. agalactiae* SipA [MKMNKKVLLTSTMAASLLSVASVQAS (SEQ ID NO: 29)]. In an embodiment, the signal sequence has an amino acid sequence at least 80%, 85%, 90%, 92%, 95%, 96%, 97%, 98%, 99% or 100% identical to a SEQ ID NO: 21-29. In one aspect, the signal sequence has an amino acid sequence at least 80%, 85%, 90%, 95%, 96%, 97%, 98%, 99% or 100% identical to *E. coli* flagellin signal sequence (FlgI) [MIKFLSALILLLVTTAAQA (SEQ ID NO: 21)]..

In an embodiment, a serine and/or alanine residue is added between the signal sequence and the start of the sequence of the mature protein, e.g.SA or S, preferably S. Such a reside or residues have the advantage of leading to more efficient cleavage of the leader sequence.

Glycosylation sites

The invention provides novel universal PgIB specific consensus sequences for glycosylation sites compatible with the quantification of glycosylation site occupancy by LC-MS. They determined several features that would be shared by such sequences and thus by the consensus sequences of the invention:
Generate tryptic peptides that are between 8 and 16 amino acids in length, e.g. 8, 9, 10, 11, 12, 13, 14 15 or 16.

Show a strong and reproducible signal in mass spectrometry analysis (parental and transition ions);
Commence and terminate with an arginine amino or lysine acid residue (for trypsin cleavage) and do not contain a cysteine amino acid residue (to increase the ionization capability of the tryptic peptide);
Preferably does not contain amino acids susceptible to modification (asparagine and glutamine amino acid residues which are susceptible to deamination, or methionine, cysteine and tryptophan amino acid residues which are susceptible to oxidation, or hydrophobic or aromatic amino acids);

Be localized on well exposed loops on the protein surface in order to be accessible to oligosaccharyltransferase enzyme (PgIB) also in an at least partially folded molecule and do not interfere with normal process of folding.

Thus, the invention provides a consensus sequence comprising or consisting of the following amino acid sequence:

$K/R-Z_{0-9}-D/E-X-N-Y-S/T-Z_{0-9}-K/R$

wherein X and Y are independently any amino acid except proline, and Z represents any amino acid. In a preferred embodiment, X and Y are independently any amino acid except proline, lysine or arginine. In an embodiment, Z represents any amino acid except lysine or arginine. In an embodiment, X, Y and/or Z are not aromatic or hydrophobic amino acids. In a preferred embodiment, Z represents any amino acid except cysteine, methionine, asparagine, glutamine, lysine or arginine (eg SEQ ID NO: 47).

Preferably, the total length of said consensus sequence is 16 or fewer amino acids, for example 8, 10, 11, 12, 13, 14, 15 or 16 amino acids. Preferably, the total length of the sequence is 8 or more amino acids, for example 8, 10, 11, 12, 13, 14, 15 or 16 amino acids.

In a specific embodiment, the invention provides a consensus sequence(s) comprising or consisting of the amino acid sequence K/R-D/E-$X_1$-N-$X_2$-S/T-$Z_1$-$Z_2$-K/R (SEQ ID NO 19), wherein $X_1$ and $X_2$ are independently any amino acid apart from proline, lysine or arginine and wherein $Z_1$ and $Z_2$.are not lysine or arginine or cysteine. In an embodiment, the consensus sequence comprises or consists of the amino acid sequence of SEQ ID NO: 20. In an embodiment, the consensus sequence comprises or consists of the amino acid sequence of any one of SEQ ID No: 42, SEQ ID No: 43; SEQ ID No: 44 or SEQ ID No 45, preferably SEQ ID Nos: 42-44.

Polysaccharides

In an embodiment, one of the antigens in a conjugate (e.g. bioconjugate) of the invention is a saccharide such as a bacterial capsular saccharide, a bacterial lipopolysaccharide or a bacterial oligosaccharide. In an embodiment the antigen is a bacterial capsular saccharide.

The saccharides may be selected from a group consisting of: *Staphylococcus aureus* type 5 capsular saccharide, *Staphylococcus aureus* type 8 capsular saccharide, *N. meningitidis* serogroup A capsular saccharide (MenA), *N. meningitidis* serogroup C capsular saccharide (MenC), *N. meningitidis* serogroup Y capsular saccharide (MenY), *N. meningitidis* serogroup W capsular saccharide (MenW), *H. influenzae* type b capsular saccharide (Hib), Group B Streptococcus group I capsular saccharide, Group B Streptococcus group II capsular saccharide, Group B Streptococcus group III capsular saccharide, Group B Streptococcus group IV capsular saccharide, Group B Streptococcus group V capsular saccharide, Vi saccharide from *Salmonella typhi, N. meningitidis* LPS (such as L3 and/or L2), *M. catarrhalis* LPS, *H. influenzae* LPS, Shigella O-antigens, *P.aeruginosa* O-antigens, *E. coli* O-antigens or *S. pneumoniae* capsular polysaccharide.

In an embodiment, the antigen is a polysaccharide or oligosaccharide. In an embodiment, the antigen comprises two or more monosaccharides, for example 2, 3, 4, 5, 6, 7, 8, 9, 10 or more monosaccharides. In an embodiment, the antigen is an oligosaccharide containing no more than 20, 15, 12, 10, 9, or 8 monosaccharides. In an embodiment, the antigen is an oligosaccharide containing no more than no more than 500, 400, 300, 200, 100, 90, 80, 70, 60, 50, 40, 30, 20, 10 or 5 monosaccharides.

Host cell

The present invention also provides a host cell comprising:

    i) one or more nucleic acids that encode glycosyltransferase(s);
    ii) a nucleic acid that encodes an oligosaccharyl transferase;
    iii) a nucleic acid that encodes a modified carrier protein of the invention; and optionally
    iv) a nucleic acid that encodes a polymerase (*e.g. wzy*).

Host cells that can be used to produce the bioconjugates of the invention, include archea, prokaryotic host cells, and eukaryotic host cells. Exemplary prokaryotic host cells for use in production of the bioconjugates of the invention, without limitation, *Escherichia* species, *Shigella* species, *Klebsiella* species, *Xhantomonas* species, *Salmonella* species, *Yersinia* species, *Lactococcus* species, *Lactobacillus* species, *Pseudomonas* species, *Corynebacterium* species, *Streptomyces* species, *Streptococcus* species, *Staphylococcus* species, *Bacillus* species, and *Clostridium* species. In a specific embodiment, the host cell is *E. coli*.

In an embodiment, the host cells used to produce the bioconjugates of the invention are engineered to comprise heterologous nucleic acids, e.g. heterologous nucleic acids that encode one or more carrier proteins and/or heterologous nucleic acids that encode one or more proteins, e.g. genes encoding one or more proteins. In a specific embodiment, heterologous nucleic acids that encode proteins involved in glycosylation pathways (e.g. prokaryotic and/or eukaryotic glycosylation pathways) may be introduced into the host cells of the invention. Such nucleic acids may encode proteins including, without limitation, oligosaccharyl transferases, epimerases, flippases, polymerases, and/or glycosyltransferases. Heterologous nucleic acids (e.g. nucleic acids that encode carrier proteins and/or nucleic acids that encode other proteins, e.g. proteins involved in glycosylation) can be introduced into the host cells of the invention using methods such as electroporation, chemical transformation by heat shock, natural transformation, phage transduction, and conjugation. In specific embodiments, heterologous nucleic acids are introduced into the host cells of the invention using a plasmid, e.g. the heterologous nucleic acids are expressed in the host cells by a plasmid (e.g. an expression vector). In another specific embodiment, heterologous nucleic acids are introduced into the host cells of the invention using the method of insertion described in International Patent application No. PCT/EP2013/068737 (published as WO 14/037585).

Thus, the present invention also provides a host cell comprising:

    i) one or more nucleic acids that encode glycosyltransferase(s);

ii) a nucleic acid that encodes an oligosaccharyl transferase;
iii) a nucleic acid that encodes a modified carrier protein of the invention;

iv) a nucleic acid that encodes a polymerase (*e.g. wzy*); and
vi) a nucleic acid that encodes a flippase (e.g. *wxy*).

In an embodiment, additional modifications may be introduced (e.g. using recombinant techniques) into the host cells of the invention. For example, host cell nucleic acids (e.g. genes) that encode proteins that form part of a possibly competing or interfering glycosylation pathway (e.g. compete or interfere with one or more heterologous genes involved in glycosylation that are recombinantly introduced into the host cell) can be deleted or modified in the host cell background (genome) in a manner that makes them inactive/dysfunctional (i.e. the host cell nucleic acids that are deleted/modified do not encode a functional protein or do not encode a protein whatsoever). In an embodiment, when nucleic acids are deleted from the genome of the host cells of the invention, they are replaced by a desirable sequence, e.g. a sequence that is useful for glycoprotein production.

Exemplary genes that can be deleted in host cells (and, in some cases, replaced with other desired nucleic acid sequences) include genes of host cells involved in glycolipid biosynthesis, such as *waaL* (see, e.g. Feldman et al. 2005, PNAS USA 102:3016-3021), the lipid A core biosynthesis cluster (waa), galactose cluster (*gal*), arabinose cluster (ara), colonic acid cluster (*wc*), capsular polysaccharide cluster, undecaprenol-pyrophosphate biosynthesis genes (e.g. *uppS* (Undecaprenyl pyrophosphate synthase), *uppP* (Undecaprenyl diphosphatase)), Und-P recycling genes, metabolic enzymes involved in nucleotide activated sugar biosynthesis, enterobacterial common antigen cluster, and prophage O antigen modification clusters like the *gtrABS* cluster.

Such a modified prokaryotic host cell comprises nucleic acids encoding enzymes capable of producing a bioconjugate comprising an antigen, for example a saccharide antigen attached to a modified Hla protein of the invention. Such host cells may naturally express nucleic acids specific for production of a saccharide antigen, or the host cells may be made to express such nucleic acids, *i.e.* in certain embodiments said nucleic acids are heterologous to the host cells. In certain embodiments, one or more of said nucleic acids specific for production of a saccharide antigen are heterologous to the host cell and integrated into the genome of the host cell. In certain embodiments, the host cells of the invention comprise nucleic acids encoding additional enzymes active in the N-glycosylation of proteins, *e.g.* the host cells of the invention further comprise a nucleic acid encoding an oligosaccharyl transferase and/or one or more nucleic acids encoding other glycosyltransferases.

Nucleic acid sequences comprising capsular polysaccharide gene clusters can be inserted into the host cells of the invention. In a specific embodiment, the capsular polysaccharide gene cluster inserted into a host cell of the invention is a capsular polysaccharide gene cluster from an *E. coli* strain, a *Staphylococcus* strain (e.g. *S. aureus*), a *Streptococcus* strain (e.g. *S. pneumoniae, S. pyrogenes, S. agalacticae*), or a *Burkholderia* strain (e.g. *B mallei, B. pseudomallei, B. thailandensis*). Disclosures of methods for making such host cells which are capable of producing bioconjugates are found in WO 06/119987, WO 09/104074, WO 11/62615, WO 11/138361, WO 14/57109, WO14/72405 and WO16/20499.

In an embodiment, the host cell comprises a nucleic acid that encodes a modified carrier protein of the invention in a plasmid in the host cell.

Glycosylation Machinery

The host cells of the invention comprise, and/or can be modified to comprise, nucleic acids that encode genetic machinery (e.g. glycosyltransferases, flippases, polymerases, and/or oligosaccharyltransferases) capable of producing hybrid oligosaccharides and/or polysaccharides, as well as genetic machinery capable of linking antigens to the modified Hla protein of the invention.

Capsular polysaccharides are assembled on the bacterial membrane carrier lipid undecaprenyl pyrophosphate by a conserved pathway that shares homology to the polymerase-dependent pathway of O polysaccharide synthesis in Gram-negative bacteria. O antigen assembly is initiated by the transfer of a sugar phosphate from a DP-donor to undecaprenyl phosphate. The lipid linked O antigen is assembled at the cytoplasmic side of the inner membrane by sequential action of different glycosyltransferases. The glycolipid is then flipped to the periplasmic space and polymerised. By replacing the O antigen ligase WaaL with the oligosaccharyltransferase PglB, the polymerised O antigen can be transferred to a protein carrier rather than to the lipid A core.

*Glycosyltransferases*

The host cells of the invention comprise nucleic acids that encode glycosyltransferases that produce an oligosaccharide or polysaccharide repeat unit. In an embodiment, said repeat unit does not comprise a hexose at the reducing end, and said oligosaccharide or polysaccharide repeat unit is derived from a donor oligosaccharide or polysaccharide repeat unit that comprises a hexose at the reducing end.

In an embodiment, the host cells of the invention may comprise a nucleic acid that encodes a glycosyltransferase that assembles a hexose monosaccharide derivative onto undecaprenyl pyrophosphate (Und-PP). In one aspect, the glycosyltransferase that assembles a hexose monosaccharide derivative onto Und-PP is heterologous to the host cell and/or heterologous to one or more of the genes that encode glycosyltransferase(s). Said glycosyltransferase can be derived from, e.g. *Escherichia* species, *Shigella* species, *Klebsiella* species, *Xhantomonas* species, *Salmonella* species, *Yersinia* species, *Aeromonas* species, *Francisella* species, *Helicobacter* species, *Proteus* species, *Lactococcus* species, *Lactobacillus* species, *Pseudomonas* species, *Corynebacterium* species, *Streptomyces* species, *Streptococcus* species, *Enterococcus* species, *Staphylococcus* species, *Bacillus* species, *Clostridium* species, *Listeria* species, or *Campylobacter* species. In a specific embodiment, the glycosyltransferase that assembles a hexose monosaccharide derivative onto Und-PP is *wecA,* optionally from *E. coli* (*wecA* can assemble GlcNAc onto UndP from UDP-GlcNAc). In an embodiment, the hexose monosaccharide is selected from the group consisting of glucose, galactose, rhamnose, arabinotol, fucose and mannose (e.g. galactose).

In an embodiment, the host cells of the invention may comprise nucleic acids that encode one or more glycosyltransferases capable of adding a monosaccharide to the hexose monosaccharide derivative assembled on Und-PP. In a specific embodiment, said one or more glycosyltransferases capable of adding a monosaccharide to the hexose monosaccharide derivative is the galactosyltransferase (*wfeD*) from *Shigella boyedii.* In another specific embodiment, said one or more glycosyltransferases capable of adding a monosaccharide to the hexose monosaccharide derivative is the galactofuranosyltransferase (*wbeY*) from *E. coli* 028. In another specific embodiment, said one or more glycosyltransferases capable of adding a monosaccharide to the hexose monosaccharide derivative is the galactofuranosyltransferase (*wfdK*) from *E. coli* 0167. Galf-transferases, such as *wfdK* and *wbeY,* can transfer Galf (Galactofuranose) from UDP-Galf to -GlcNAc-P-P-Undecaprenyl. In another specific embodiment, said one or more glycosyltransferases capable of adding a monosaccharide to the hexose monosaccharide derivative are the galactofuranosyltransferase (*wbeY*) from *E. coli* 028 and the galactofuranosyltransferase (*wfdK*) from *E. coli* 0167.

In an embodiment, the host cells of the invention comprise nucleic acids that encode glycosyltransferases that assemble the donor oligosaccharide or polysaccharide repeat unit onto the hexose monosaccharide derivative.

In an embodiment, the glycosyltransferases that assemble the donor oligosaccharide or polysaccharide repeat unit onto the hexose monosaccharide derivative comprise a glycosyltransferase that is capable of adding the hexose monosaccharide present at the reducing end of the first repeat unit of the donor oligosaccharide or polysaccharide to the hexose monosaccharide derivative. Exemplary glycosyltransferases include galactosyltransferases (*wciP*)*,* e.g. *wciP* from *E. coli* 021.

In one embodiment, the glycosyltransferases that assemble the donor oligosaccharide or polysaccharide repeat unit onto the hexose monosaccharide derivative comprise a glycosyltransferase that is capable of adding the monosaccharide that is adjacent to the hexose monosaccharide present at the reducing end of the first repeat unit of the donor oligosaccharide or polysaccharide to the hexose monosaccharide present at the reducing end of the first repeat unit of the donor oligosaccharide or polysaccharide. Exemplary glycosyltransferases include glucosyltransferase (*wciQ*)*,* e.g. *wciQ* from *E. coli* O21.

In an embodiment, a host cell of the invention comprises glycosyltransferases for synthesis of the repeat units of an oligosaccharide or polysaccharide selected from the *Staphylococcus aureus* CP5 or CP8 gene cluster. In a specific embodiment, the glycosyltransferases for synthesis of the repeat units of an oligosaccharide or polysaccharide are from the *Staphylococcus aureus* CP5 gene cluster. S. *aureus* CP5 and CP8 have a similar structure to *P. aeruginosa* O11 antigen synthetic genes, so these genes may be combined with *E. coli* monosaccharide synthesis genes to synthesise an undecaprenyl pyrophosphate-linked CP5 or CP8 polymer consisting of repeating trisaccharide units.

In an embodiment, a host cell of the invention comprises glycosyltransferases that assemble the donor oligosaccharide or polysaccharide repeat unit onto the hexose monosaccharide derivative comprise a glycosyltransferase that is capable of adding the hexose monosaccharide present at the reducing end of the first repeat unit of the donor oligosaccharide or polysaccharide to the hexose monosaccharide derivative.

*Oligosaccharyl Transferases*

N-linked protein glycosylation -the addition of carbohydrate molecules to an asparagine residue in the polypeptide chain of the target protein- is the most common type of post-translational modification occurring in the endoplasmic reticulum of eukaryotic organisms. The process is accomplished by the enzymatic oligosaccharyltransferase complex (OST) responsible for the transfer of a preassembled oligosaccharide from a lipid carrier (dolichol phosphate) to an asparagine residue of a nascent protein within the conserved sequence Asn-X-Ser/Thr (where X is any amino acid except proline) in the Endoplasmic reticulum.

It has been shown that a bacterium, the food-borne pathogen *Campylobacter jejuni,* can also N-glycosylate its proteins (Wacker et al. Science. 2002; 298(5599): 1790-3) due to the fact that it possesses its own glycosylation machinery. The machinery responsible of this reaction is encoded by a cluster called "pgl" (for protein glycosylation).

The *C. jejuni* glycosylation machinery can be transferred to *E. coli* to allow for the glycosylation of recombinant proteins expressed by the *E. coli* cells. Previous studies have demonstrated how to generate *E. coli* strains that can perform N-glycosylation (see, e.g. Wacker et al. Science. 2002; 298 (5599):1790-3; Nita-Lazar et al. Glycobiology. 2005; 15(4):361-7; Feldman et al. Proc Natl Acad Sci U S A. 2005; 102(8):3016-21; Kowarik et al. EMBO J. 2006; 25(9):1957-66; Wacker et al. Proc Natl Acad Sci U S A. 2006; 103(18):7088-93; International Patent Application Publication Nos. WO2003/074687, WO2006/119987, WO 2009/104074, and WO/2011/06261, and WO2011/138361).PglB mutants having optimised properties are described in WO2016/107818. A preferred mutant is PglB$_{cuo}$ N311V-K482R-D483H-A669V.

Oligosaccharyl transferases transfer lipid-linked oligosaccharides to asparagine residues of nascent polypeptide chains that comprise a N-glycosylation consensus motif, e.g. Asn-X-Ser(Thr), wherein X can be any amino acid except Pro; or Asp(Glu)-X-Asn-Z-Ser(Thr), wherein X and Z are independently selected from any natural amino acid except Pro (see WO 2006/119987). See, e.g. WO 2003/074687 and WO 2006/119987, the disclosures of which are herein incorporated by reference in their entirety.

In an embodiment, the host cells of the invention comprise a nucleic acid that encodes an oligosaccharyl transferase. The nucleic acid that encodes an oligosaccharyl transferase can be native to the host cell, or can be introduced into the host cell using genetic approaches, as described above. In a specific embodiment, the oligosaccharyl transferase is an oligosaccharyl transferase from *Campylobacter*. In another specific embodiment, the oligosaccharyl transferase is an oligosaccharyl transferase from *Campylobacter jejuni* (i.e. *pglB;* see, e.g. Wacker et al. 2002, Science 298:1790-1793; see also, e.g. NCBI Gene ID: 3231775, UniProt Accession No. O86154). In another specific embodiment, the oligosaccharyl transferase is an oligosaccharyl transferase from *Campylobacter lari* (see, e.g. NCBI Gene ID: 7410986).

In a specific embodiment, the host cells of the invention comprise a nucleic acid sequence encoding an oligosaccharyl transferase, wherein said nucleic acid sequence encoding an oligosaccharyl transferase (*e.g. pglB* from *Campylobacter jejuni*) is integrated into the genome of the host cell.

In a specific embodiment, the host cells of the invention comprise a nucleic acid sequence encoding an oligosaccharyl transferase, wherein said nucleic acid sequence encoding an oligosaccharyl transferase (*e.g. pglB* from *Campylobacter jejuni*) is plasmid-borne.

In another specific embodiment, provided herein is a modified prokaryotic host cell comprising (i) a glycosyltransferase derived from an capsular polysaccharide cluster from S. *aureus,* wherein said glycosyltransferase is integrated into the genome of said host cell; (ii) a nucleic acid encoding an oligosaccharyl transferase (*e.g. pglB* from *Campylobacter jejuni),* wherein said nucleic acid encoding an oligosaccharyl transferase is plasmid-borne and/or integrated into the genome of the host cell; and (iii) a modified carrier protein of the invention, wherein said modified carrier protein is either plasmid-borne or integrated into the genome of the host cell. There is also provided a method of making a modified prokaryotic host cell comprising (i) integrating a glycosyltransferase derived from an capsular polysaccharide cluster into the genome of said host cell; (ii) integrating into the host cell one or more nucleic acids encoding an oligosaccharyl transferase (*e.g. pglB* from *Campylobacter jejuni*) which is plasmid-borne and/or integrated into the genome of the host cell; and (iii) integrating into a host cell a modified carrier protein of the invention either plasmid-borne or integrated into the genome of the host cell.

In specific embodiment is a host cell of the invention, wherein at least one gene of the host cell has been functionally inactivated or deleted, optionally wherein the *waaL* gene of the host cell has been functionally inactivated or deleted, optionally wherein the *waaL* gene of the host cell has been replaced by a nucleic acid encoding an oligosaccharyltransferase, optionally wherein the *waaL* gene of the host cell has been replaced by *C. jejuni pglB*.

*Polymerases*

In an embodiment, a polymerase (*e.g. wzy*) is introduced into a host cell of the invention (i.e. the polymerase is heterologous to the host cell). In an embodiment, the polymerase is a bacterial polymerase. In an embodiment, the polymerase is a capsular polysaccharide polymerase (*e.g. wzy*) or an O antigen polymerase (*e.g. wzy*). In an embodiment, the polymerase is a capsular polysaccharide polymerase (*e.g. wzy*).

In an embodiment, a polymerase of a capsular polysaccharide biosynthetic pathway is introduced into a host cell of the invention.

*Flippases*

In an embodiment, a flippase (wzx or homologue) is introduced into a host cell of the invention (i.e. the flippase is heterologous to the host cell). Thus, a host cell of the invention may further comprise a flippase. In an embodiment, the flippase is a bacterial flippase. Flippases translocate wild type repeating units and/or their corresponding engineered (hybrid) repeat units from the cytoplasm into the periplam of host cells (e.g. *E. coli*). Thus, a host cell of the invention may comprise a nucleic acid that encodes a flippase (*wzx*).

In a specific embodiment, a flippase of a capsular polysaccharide biosynthetic pathway is introduced into a host cell of the invention.

*Genetic Background*

Exemplary host cells that can be used to generate the host cells of the invention include, without limitation, *Escherichia* species, *Shigella* species, *Klebsiella* species, *Xhantomonas* species, *Salmonella* species, *Yersinia* species, *Lactococcus* species, *Lactobacillus* species, *Pseudomonas* species, *Corynebacterium* species, *Streptomyces* species, *Streptococcus* species, *Staphylococcus* species, *Bacillus* species, and *Clostridium* species. In a specific embodiment, the host cell used herein is *E. coli.*

In an embodiment, the host cell genetic background is modified by, e.g. deletion of one or more genes. Exemplary genes that can be deleted in host cells (and, in some cases, replaced with other desired nucleic acid sequences) include genes of host cells involved in glycolipid biosynthesis, such as *waaL* (see, e.g. Feldman et al. 2005, PNAS USA 102:3016-3021), the O antigen cluster (*rfb* or *wb*), enterobacterial common antigen cluster (*wec*), the lipid A core biosynthesis cluster (*waa*), and prophage O antigen modification clusters like the *gtrABS* cluster. In a specific embodiment, one or more of the *waaL* gene, *gtrA* gene, *gtrB* gene, *gtrS* gene, or a gene or genes from the wec cluster or a gene or genes from the *rfb* gene cluster are deleted or functionally inactivated from the genome of a prokaryotic host cell of the invention. In one embodiment, a host cell used herein is *E. coli,* wherein the *waaL* gene, *gtrA* gene, *gtrB* gene, *gtrS* gene are deleted or functionally inactivated from the genome of the host cell. In another embodiment, a host cell used herein is *E. coli,* wherein the *waaL* gene and *gtrS* gene are deleted or functionally inactivated from the genome of the host cell. In another embodiment, a host cell used herein is *E. coli,* wherein the *waaL* gene and genes from the wec cluster are deleted or functionally inactivated from the genome of the host cell.

Bioconjugates

The host cells of the invention can be used to produce bioconjugates comprising a saccharide antigen, for example a bacterial capsular polysaccharide antigen linked to a modified carrier protein of the invention. In an embodiment, the polysaccharide is linked to asparagine in the modified carrier protein, for example via N-acetylglucosamine. Methods of producing bioconjugates using host cells are described for example in WO 2003/074687, WO 2006/119987 and WO2011/138361. Bioconjugates, as described herein, have advantageous properties over chemical conjugates of antigen-carrier protein, in that they require less chemicals in manufacture and are more consistent in terms of the final product generated.

In an embodiment, provided herein is a bioconjugate comprising a modified carrier protein of the invention linked to a polysaccharide, in particular a polysaccharide antigen. In an embodiment, provided herein is a bioconjugate comprising a modified carrier protein of the invention and an antigen selected from *Staphylococcus aureus* type 5 capsular saccharide, *Staphylococcus aureus* type 8 capsular saccharide, *N. meningitidis* serogroup A capsular saccharide (MenA), *N. meningitidis* serogroup C capsular saccharide (MenC), *N. meningitidis* serogroup Y capsular saccharide (MenY), *N. meningitidis* serogroup W capsular saccharide (MenW), *H. influenzae* type b capsular saccharide (Hib), Group B Streptococcus group I capsular saccharide, Group B Streptococcus group II capsular saccharide, Group B Streptococcus group III capsular saccharide, Group B Streptococcus group IV capsular saccharide, Group B Streptococcus group V capsular saccharide, Vi saccharide from *Salmonella typhi, N. meningitidis* LPS (such as L3 and/or L2), *M. catarrhalis* LPS, *H. influenzae* LPS, Shigella O-antigens, *P.aeruginosa* O-antigens, *E. coli* O-antigens or *S. pneumoniae* capsular polysaccharide.

The bioconjugates of the invention can be purified for example, by chromatography (e.g. ion exchange, cationic exchange, anionic exchange, affinity, and sizing column chromatography), centrifugation, differential solubility, or by any other standard technique for the purification of proteins. See, e.g. Saraswat et al. 2013, Biomed. Res. Int. ID#312709 (p. 1-18); see also the methods described in WO 2009/104074. Further, the bioconjugates may be fused to heterologous polypeptide sequences described herein or otherwise known in the art to facilitate purification. The actual conditions used to purify a particular bioconjugate will depend, in part, on the synthesis strategy and on factors such as net charge, hydrophobicity, and/or hydrophilicity of the bioconjugate, and will be apparent to those having skill in the art.

A further aspect of the invention is a process for producing a bioconjugate that comprises (or consists of) a modified carrier protein linked to a polysaccharide, said method comprising (i) culturing the host cell of the invention under conditions suitable for the production of proteins (and optionally under conditions suitable for the production of saccharides) and (ii) isolating the bioconjugate produced by said host cell.

A further aspect of the invention is a bioconjugate produced by the process of the invention, wherein said bioconjugate comprises a saccharide linked to a modified carrier protein.

Mass spectrometry methods

The present invention consists in the generation of carrier proteins by an analytics driven design approach that allows to measure the glycosylation site occupancy by liquid chromatography coupled to mass spectrometry (LC-MS). This is particularly relevant to (i) quantify the unglycosylated carrier in the final product, (ii) follow in process the rate of bioconjugation and (iii) quantify the extent of glycosylation on single sites, in the case of carrier proteins designed where multiple sites for glycosylation are introduced, to increase the rate of glycosylation.

The strategy is based on the quantification of the natively unglycosylated form of the glycopeptide, using isotopically labeled internal standards. In particular, two sets of heavy isotope labeled peptide standards are spiked into the sample before proteolysis, and the digested sample is analyzed by LC-MS. One set of peptide standards is employed to determine the total glycoprotein amount, while the other standard monitors the unglycosylated amount of the glycoprotein. In this way, the abundance of the glycosylated portion of the protein is calculated by subtracting the unglycosylated protein amount from the total protein amount, and the site occupancy is then determined.

Immunogenic Compositions

The modified carrier proteins and conjugates (e.g. bioconjugate), of the invention are particularly suited for inclusion in immunogenic compositions and vaccines. The present invention provides an immunogenic composition comprising a modified carrier protein of the invention, or the conjugate of the invention, or the bioconjugate of the invention.

Also provided is a method of making the immunogenic composition of the invention comprising the step of mixing the modified carrier protein or the conjugate (e.g. bioconjugate) of the invention with a pharmaceutically acceptable excipient or carrier.

Immunogenic compositions comprise an immunologically effective amount of the modified carrier protein or conjugate (e.g. bioconjugate) of the invention, as well as any other components. By "immunologically effective amount", it is meant that the administration of that amount to an individual, either as a single dose or as part of a series is effective for treatment or prevention. This amount varies depending on the health and physical condition of the individual to be treated, age, the degree of protection desired, the formulation of the vaccine and other relevant factors. It is expected that the amount will fall in a relatively broad range that can be determined through routine trials.

Immunogenic compositions if the invention may also contain diluents such as water, saline, glycerol etc. Additionally, auxiliary substances, such as wetting or emulsifying agents, pH buffering substances, polyols and the like may be present.

The immunogenic compositions comprising the modified carrier protein of the invention or conjugates (or bioconjugates) may comprise any additional components suitable for use in pharmaceutical administration. In specific embodiments, the immunogenic compositions of the invention are monovalent formulations. In other embodiments, the immunogenic compositions of the invention are multivalent formulations, e.g. bivalent, trivalent, and tetravalent formulations. For example, a multivalent formulation comprises more than one antigen for example more than one conjugate.

Vaccines

The present invention also provides a vaccine comprising an immunogenic composition of the invention and a pharmaceutically acceptable excipient or carrier.

Pharmaceutically acceptable excipients and carriers can be selected by those of skill in the art. For example, the pharmaceutically acceptable excipient or carrier can include a buffer, such as Tris (trimethamine), phosphate (e.g. sodium phosphate), acetate, borate (e.g. sodium borate), citrate, glycine, histidine and succinate (e.g. sodium succinate), suitably sodium chloride, histidine, sodium phosphate or sodium succinate. The pharmaceutically acceptable excipient may include a salt, for example sodium chloride, potassium chloride or magnesium chloride. Optionally, the pharmaceutically acceptable excipient contains at least one component that stabilizes solubility and/or stability. Examples of solubilizing/stabilizing agents include detergents, for example, laurel sarcosine and/or polysorbate (e.g. TWEEN™ 80). Examples of stabilizing agents also include poloxamer (e.g. poloxamer 124, poloxamer 188, poloxamer 237, poloxamer 338 and poloxamer 407). The pharmaceutically acceptable excipient may include a non-ionic surfactant, for example polyoxyethylene sorbitan fatty acid esters, Polysorbate-80 (TWEEN™ 80), Polysorbate-60 (TWEEN™ 60), Polysorbate-40 (TWEEN™ 40) and Polysorbate-20 (TWEEN™ 20), or polyoxyethylene alkyl ethers (suitably polysorbate-80). Alternative solubilizing/stabilizing agents include arginine, and glass forming polyols (such as sucrose, trehalose and the like). The pharmaceutically excipient may be a preservative, for example phenol, 2-phenoxyethanol, or thiomersal. Other pharmaceutically acceptable excipients include sugars (e.g. lactose, sucrose), and proteins (e.g. gelatine and albumin). Pharmaceutically acceptable carriers include water, saline solutions, aqueous dextrose and glycerol solutions. Numerous pharmaceutically acceptable excipients and carriers are described, for example, in Remington's Pharmaceutical Sciences, by E. W. Martin, Mack Publishing Co. Easton, PA, 5th Edition (975).

In an embodiment, the immunogenic composition or vaccine of the invention additionally comprises one or more buffers, *e.g.* phosphate buffer and/or sucrose phosphate glutamate buffer. In other embodiments, the immunogenic composition or vaccine of the invention does not comprise a buffer.

In an embodiment, the immunogenic composition or vaccine of the invention additionally comprises one or more salts, *e.g.* sodium chloride, calcium chloride, sodium phosphate, monosodium glutamate, and aluminum salts (*e.g.* aluminum hydroxide, aluminum phosphate, alum (potassium aluminum sulfate), or a mixture of such aluminum salts). In other embodiments, the immunogenic composition or vaccine of the invention does not comprise a salt.

The immunogenic composition or vaccine of the invention may additionally comprise a preservative, e.g. a mercury derivative thimerosal. In a specific embodiment, the immunogenic composition or vaccine of the invention comprises 0.001% to 0.01% thimerosal. In other embodiments, the immunogenic composition or vaccine of the invention do not comprise a preservative.

The vaccine or immunogenic composition of the invention may also comprise an antimicrobial, typically when package in multiple dose format. For example, the immunogenic composition or vaccine of the invention may comprise 2-phenoxyethanol.

The vaccine or immunogenic composition of the invention may also comprise a detergent *e.g.* polysorbate, such as TWEEN™ 80. Detergents are generally present at low levels e.g. <0.01%, but higher levels have been suggested for stabilising antigen formulations e.g. up to 10%.

The immunogenic compositions of the invention can be included in a container, pack, or dispenser together with instructions for administration.

The immunogenic compositions or vaccines of the invention can be stored before use, *e.g.* the compositions can be stored frozen (*e.g.* at about -20°C or at about -70°C); stored in refrigerated conditions (*e.g.* at about 4°C); or stored at room temperature.

The immunogenic compositions or vaccines of the invention may be stored in solution or lyophilized. In an embodiment, the solution is lyophilized in the presence of a sugar such as sucrose, trehalose or lactose. In another embodiment, the vaccines of the invention are lyophilized and extemporaneously reconstituted prior to use.

Vaccine preparation is generally described in Vaccine Design ("The subunit and adjuvant approach" (eds Powell M.F. & Newman M.J.) (1995) Plenum Press New York). Encapsulation within liposomes is described by Fullerton, US Patent 4,235,877.

The present invention also provides a vaccine comprising an immunogenic composition of the invention and a pharmaceutically acceptable excipient or carrier.

Adjuvants

In an embodiment, the immunogenic compositions or vaccines of the invention comprise, or are administered in combination with, an adjuvant. The adjuvant for administration in combination with an immunogenic composition or vaccine of the invention may be administered before, concomitantly with, or after administration of said immunogenic composition or vaccine. In some embodiments, the term "adjuvant" refers to a compound that when administered in conjunction with or as part of an immunogenic composition of vaccine of the invention augments, enhances and/or boosts the immune response to a bioconjugate, but when the compound is administered alone does not generate an immune response to the modified carrier protein/conjugate/bioconjugate. In some embodiments, the adjuvant generates an immune response to the modified carrier protein, conjugate or bioconjugate and does not produce an allergy or other adverse reaction.

In an embodiment, the immunogenic composition or vaccine of the invention is adjuvanted. Adjuvants can enhance an immune response by several mechanisms including, e.g. lymphocyte recruitment, stimulation of B and/or T cells, and stimulation of macrophages. Specific examples of adjuvants include, but are not limited to, aluminum salts (alum) (such as aluminum hydroxide, aluminum phosphate, and aluminum sulfate), 3 De-O-acylated monophosphoryl lipid A (MPL) (see United Kingdom Patent GB2220211), MF59 (Novartis), AS03 (GlaxoSmithKline), AS04 (GlaxoSmith-Kline), polysorbate 80 (TWEEN™ 80; ICL Americas, Inc.), imidazopyridine compounds (see International Application No. PCT/US2007/064857, published as International Publication No. WO2007/109812), imidazoquinoxaline compounds (see International Application No. PCT/US2007/064858, published as International Publication No. WO2007/109813) and saponins, such as QS21 (see Kensil et al. in Vaccine Design: The Subunit and Adjuvant Approach (eds. Powell & Newman, Plenum Press, NY, 1995); U.S. Pat. No. 5,057,540). In some embodiments, the adjuvant is Freund's adjuvant (complete or incomplete). Other adjuvants are oil in water emulsions (such as squalene or peanut oil), optionally in combination with immune stimulants, such as monophosphoryl lipid A (see Stoute et al. N. Engl. J. Med. 336, 86-91 (1997)). Another adjuvant is CpG (Bioworld Today, Nov. 15, 1998).

In one aspect of the invention, the adjuvant is an aluminum salt such as aluminum hydroxide gel (alum) or aluminium phosphate.

In another aspect of the invention, the adjuvant is selected to be a preferential inducer of either a TH1 or a TH2 type of response. High levels of Th1-type cytokines tend to favor the induction of cell mediated immune responses to a given antigen, whilst high levels of Th2-type cytokines tend to favour the induction of humoral immune responses to the antigen. It is important to remember that the distinction of Th1 and Th2-type immune response is not absolute. In reality an individual will support an immune response which is described as being predominantly Th1 or predominantly Th2. However, it is often convenient to consider the families of cytokines in terms of that described in murine CD4 +ve T cell clones by Mosmann and Coffman (Mosmann, T.R. and Coffman, R.L. (1989) TH1 and TH2 cells: different patterns of lymphokine secretion lead to different functional properties. Annual Review of Immunology, 7, p145-173). Traditionally, Th1-type responses are associated with the production of the INF-γ and IL-2 cytokines by T-lymphocytes. Other cytokines often directly associated with the induction of Th1-type immune responses are not produced by T-cells, such as IL-12. In contrast, Th2-type responses are associated with the secretion of II-4, IL-5, IL-6, IL-10. Suitable adjuvant systems which promote a predominantly Th1 response include: Monophosphoryl lipid A or a derivative thereof, particularly 3-de-O-acylated monophosphoryl lipid A (3D-MPL) (for its preparation see GB 2220211 A); MPL, e.g. 3D-MPL and the saponin QS21 in a liposome, for example a liposome comprising cholesterol and DPOC; and a combination of monophosphoryl lipid A, for example 3-de-O-acylated monophosphoryl lipid A, together with either an Aluminium salt (for instance Aluminium phosphate or Aluminium hydroxide) or an oil-in-water emulsion. In such combinations, the antigen and 3D-MPL may be contained in the same particulate structures, allowing for more efficient delivery of antigenic and immunostimulatory signals. Studies have shown that 3D-MPL is able to further enhance the immunogenicity of an Alum-adsorbed antigen (Thoelen et al. Vaccine (1998) 16:708-14; EP 689454-B1). Unmethylated CpG containing oligonucleotides (WO 96/02555) are also preferential inducers of a TH1 response and are suitable for use in the present invention.

The vaccine or immunogenic composition of the invention may contain an oil in water emulsion, since these have been suggested to be useful as adjuvant compositions (EP 399843; WO 95/17210). Oil in water emulsions such as those described in WO95/17210 (which discloses oil in water emulsions comprising from 2 to 10% squalene, from 2 to 10% alpha tocopherol and from 0.3 to 3% tween 80 and their use alone or in combination with QS21 and/or 3D-MPL), WO99/12565 (which discloses oil in water emulsion compositions comprising a metabolisable oil, a saponin and a sterol and MPL) or WO99/11241 may be used. Further oil in water emulsions such as those disclosed in WO 09/127676 and WO 09/127677 are also suitable. A particularly potent adjuvant formulation involving QS21, 3D-MPL and tocopherol in an oil in water emulsion is described in WO 95/17210. In a specific embodiment, the immunogenic composition or vaccine additionally comprises a saponin, for example QS21. The immunogenic composition or vaccine may also comprise an oil in water emulsion and tocopherol (WO 95/17210).

Prophylactic and Therapeutic Uses

The present invention also provides methods of treating and/or preventing bacterial infections of a subject comprising administering to the subject a modified carrier protein, conjugate or bioconjugate of the invention. The modified carrier protein, conjugate or bioconjugate may be in the form of an immunogenic composition or vaccine. In a specific embodiment, the immunogenic composition or vaccine of the invention is used in the prevention of infection of a subject (e.g. human subjects) by a bacterium. Bacterial infections that can be treated and/or prevented using the modified Hla protein, conjugate or bioconjugate of the invention include those caused by *Staphylococcus* species, *Escherichia* species, *Shigella* species, *Neisseria* species, *Moraxella* species, *Haemophilus* species, *Klebsiella* species, *Xhantomonas* species, *Salmonella* species, *Yersinia* species, *Aeromonas* species, *Francisella* species, *Helicobacter* species, *Proteus* species, *Lactococcus* species, *Lactobacillus* species, *Pseudomonas* species, *Corynebacterium* species, *Streptomyces* species, *Streptococcus* species, *Enterococcus* species, *Bacillus* species, *Clostridium* species, *Listeria* species, or *Campylobacter* species, *Staphylococcus aureus, N. meningitidis, H. influenzae, H. influenzae* type b, Group B Streptococcus, *S. typhi, M. catarrhalis* LPS, *S. flexneri, P.aeruginosa, E. coli* or *S. pneumoniae.*

Also provided herein are methods of inducing an immune response in a subject against a bacterium, comprising administering to the subject a modified carrier protein, or conjugate or bioconjugate of the invention (or immunogenic composition or vaccine). In one embodiment, said subject has bacterial infection at the time of administration. In another embodiment, said subject does not have a bacterial infection at the time of administration. The modified carrier protein, conjugate or bioconjugate of the invention can be used to induce an immune response against *Staphylococcus* species, *Escherichia* species, *Shigella* species, *Klebsiella* species, *Xhantomonas* species, *Salmonella* species, *Yersinia* species, *Aeromonas* species, *Francisella* species, *Helicobacter* species, *Proteus* species, *Lactococcus* species, *Lactobacillus* species, *Pseudomonas* species, *Corynebacterium* species, *Streptomyces* species, *Streptococcus* species, *Enterococcus* species, *Bacillus* species, *Clostridium* species, *Listeria* species, or *Campylobacter* species. In a specific embodiment, modified Hla protein, or conjugate or bioconjugate of the invention is used to induce an immune response against *Staphylococcus* species (e.g. *Staphylococcus aureus).*

Also provided herein are methods of inducing the production of opsonophagocytic antibodies in a subject against a bacterium, comprising administering to the subject a modified carrier protein, or conjugate or bioconjugate of the invention (or immunogenic composition or vaccine). In one embodiment, said subject has bacterial infection at the time of administration. In another embodiment, said subject does not have a bacterial infection at the time of administration. The modified carrier protein, or conjugate or bioconjugate of the invention (or immunogenic composition or vaccine) provided herein can be used to induce the production of opsonophagocytic antibodies against *Staphylococcus* species, *Escherichia* species, *Shigella* species, *Klebsiella* species, *Xhantomonas* species, *Salmonella* species, *Yersinia* species, *Aeromonas* species, *Francisella* species, *Helicobacter* species, *Proteus* species, *Lactococcus* species, *Lactobacillus* species, *Pseudomonas* species, *Corynebacterium* species, *Streptomyces* species, *Streptococcus* species, *Enterococcus* species, *Bacillus* species, *Clostridium* species, *Listeria* species, or *Campylobacter* species. In a specific embodiment, a modified carrier protein, or conjugate or bioconjugate of the invention (or immunogenic composition or vaccine) is used to induce the production of opsonophagocytic antibodies against *Staphylococcus* species (e.g. *Staphylococcus aureus*).

The present invention also provides methods of treating and/or preventing bacterial infections of a subject comprising administering to the subject a modified carrier protein, conjugate or bioconjugate of the invention. The modified carrier protein, conjugate or bioconjugate may be in the form of an immunogenic composition or vaccine. In a specific embodiment, the immunogenic composition or vaccine of the invention is used in the prevention of infection of a subject (e.g. human subjects) by a bacterium. Bacteria infections that can be treated and/or prevented using the modified carrier protein, conjugate or bioconjugate of the invention include those caused by *Escherichia* species, *Shigella* species, *Klebsiella* species, *Xhantomonas* species, *Salmonella* species, *Yersinia* species, *Aeromonas* species, *Francisella* species, *Helicobacter* species, *Proteus* species, *Lactococcus* species, *Lactobacillus* species, *Pseudomonas* species, *Corynebacterium* species, *Streptomyces* species, *Streptococcus* species, *Enterococcus* species, *Staphylococcus* species, *Bacillus* species, *Clostridium* species, *Listeria* species, or *Campylobacter* species. In a specific embodiment, the immunogenic composition or vaccine of the invention is used to treat or prevent an infection by *Streptococcus* species (e.g. *Streptococcus pneumoniae*).

Also provided herein are methods of inducing an immune response in a subject against a bacterium, comprising administering to the subject a modified carrier protein, or conjugate or bioconjugate of the invention (or immunogenic composition or vaccine). In one embodiment, said subject has bacterial infection at the time of administration. In another embodiment, said subject does not have a bacterial infection at the time of administration. The modified carrier protein, conjugate or bioconjugate of the invention can be used to induce an immune response against *Escherichia* species, *Shigella* species, *Klebsiella* species, *Xhantomonas* species, *Salmonella* species, *Yersinia* species, *Aeromonas* species, *Francisella* species, *Helicobacter* species, *Proteus* species, *Lactococcus* species, *Lactobacillus* species, *Pseudomonas* species, *Corynebacterium* species, *Streptomyces* species, *Streptococcus* species, *Enterococcus* species, *Staphylococcus* species, *Bacillus* species, *Clostridium* species, *Listeria* species, or *Campylobacter* species. In a specific embodiment, modified carrier protein, or conjugate or bioconjugate of the invention is used to induce an immune response against *Streptococcus* species (e.g. *Streptococcus pneumoniae*).

Also provided herein are methods of inducing the production of opsonophagocytic antibodies in a subject against a bacterium, comprising administering to the subject a modified carrier protein, or conjugate or bioconjugate of the invention (or immunogenic composition or vaccine). In one embodiment, said subject has bacterial infection at the time of administration. In another embodiment, said subject does not have a bacterial infection at the time of administration. The modified carrier protein, or conjugate or bioconjugate of the invention (or immunogenic composition or vaccine) provided herein can be used to induce the production of opsonophagocytic antibodies against *Escherichia* species, *Shigella* species, *Klebsiella* species, *Xhantomonas* species, *Salmonella* species, *Yersinia* species, *Aeromonas* species, *Francisella* species, *Helicobacter* species, *Proteus* species, *Lactococcus* species, *Lactobacillus* species, *Pseudomonas* species, *Corynebacterium* species, *Streptomyces* species, *Streptococcus* species, *Enterococcus* species, *Staphylococcus* species, *Bacillus* species, *Clostridium* species, *Listeria* species, or *Campylobacter* species. In a specific embodiment, a modified carrier protein, or conjugate or bioconjugate of the invention (or immunogenic composition or vaccine) is used to induce the production of opsonophagocytic antibodies against *Streptococcus* species (e.g. *Streptococcus pneumoniae*).

In an embodiment, the present invention is an improved method to elicit an immune response in infants (defined as 0-2 years old in the context of the present invention) by administering a therapeutically effective amount of an immunogenic composition or vaccine of the invention (a paediatric vaccine). In an embodiment, the vaccine is a paediatric vaccine.

In an embodiment, the present invention is an improved method to elicit an immune response in the elderly population (in the context of the present invention a patient is considered elderly if they are 50 years or over in age, typically over 55 years and more generally over 60 years) by administering a therapeutically effective amount of the immunogenic composition or vaccine of the invention. In an embodiment, the vaccine is a vaccine for the elderly.

All references or patent applications cited within this patent specification are incorporated by reference herein.

In order that this invention may be better understood, the following examples are set forth. These examples are for purposes of illustration only, and are not to be construed as limiting the scope of the invention in any manner.

**EXAMPLES**

**MATERIALS AND METHODS**

*Bacterial strains, cloning and growing conditions.*

The Polymerase Incomplete Primer Extension (PIPE) (14) method was applied for mutagenesis and cloning experiments to obtain the plasmids Bcp218, Bcp233, Bcp234 and Bcp235 carrying the sequence of the newly designed Hla-i Hla-s, Hla-v and Hla-a carriers, in which the bioconjugation consensus sequence in position 130 of a recombinant form of Hla (SEQ ID NO: 9 in patent PCT/EP2018/085854, published as WO2019/121924; plasmid: pGVXN2872; see also Wacker, M. et al J. Infect. Dis. 2014, 209, 1551-1561) was substituted with the sequences KDSNITSAR, KDSNSTSAR, KDSNVTSAR and KDSNATSAR, respectively.

*E. coli* W3110 StGVXN9268 was co-transformed by electroporation with the plasmids encoding the *S. aureus* Hla-i Hla-v Hla-s isoforms and the *C. jejuni* oligosaccharyltransferase PgtBN311V-K482R-D483H-A669V (pGVXN1221).

Transformed bacteria were grown overnight on selective agar plates supplemented with the two antibiotics kanamycin [50 μg/ml] and spectinomycin [80 μg/ml] for the maintenance of the plasmid encoding for Hla and PgIB, respectively. Bacteria were inoculated in 50 ml Lysogeny broth (LB) containing antibiotics and shaken in Erlenmeyer flask overnight at 37°C, 180 rpm. A culture of 50 ml HTMC medium supplemented with kanamycin [50 μg/ml] and spectinomycin [80 μg/ml] was inoculated to a dilution of 0.1 optical density at 600nm ($OD_{600nm}$), incubated at 37°C in a shaker at 180 rpm, until an average $OD_{600nm}$ of 0.8- 1.0 and then induced overnight, by using 0.2% arabinose for the induction of Hla expression and 1 mM IPTG for the induction of PgIB expression. The cultures were shaken overnight at 37°C, 180rpm, and 20 $OD_{600nm}$ of bacteria were harvested after 20 hours of induction. The supernatants were discarded and the pellets were immediately used for periplasmic extraction.

*Periplasmic extraction*

Periplasmic extraction (PPE) was performed on 20 $OD_{600nm}$ of bacterial pellets recovered after 20 min centrifugation (4000 rcf at 4°C), resuspended in 600 uL of Lysis buffer (30 mM Tris HCI pH 8.5, 1mM EDTA, 20% (wt/vol) Sucrose) and then treated for 20 min in a rotating shaker at 4°C with 1 mg/mL final Lysozyme. After 20 min of centrifugation at 16000 rcf 4°C, supernatants were immediately collected and stored at -20°C until their use. Total protein content was assessed by Bicinchoninic acid assay (Kit Reducing Agent Compatible, Thermo Fischer Scientific).

*Western Blot analysis*

Glycosylation status of the periplasmic Hla was analyzed by SDS-PAGE (4-12% Bis Tris in MES 1X at 150V for 1h) and immunoblotting Hla-CP5 antisera (1:1000). A commercial secondary Goat Anti-Rabbit HRP-conjugated antibody was used in a 1:5000 diluition (DAKO Ab, Agilent, CA, USA).

*Selection of PTPs for Hla total protein amount quantification.*

20 ug of recombinant Hla H35L was boiled at 95°C for 5 minutes in 50 mM ammonium bicarbonate containing 5 mM of DTT and 0.1% (wt/vol) RapiGestSF (Waters, USA), and digested overnight at 37 °C with trypsin [1/25 (wt/wt), enzyme/substrate ratio] (Promega). Peptides mixture were analyzed by LC-MS/MS performed on a Acquity UPLC system (Waters®) coupled with a Thermo Scientific Q Exactive plus mass spectrometer equipped with a micro electron spray ESI source (Thermo). Samples were loaded using a full loop injection at a flow rate of 40 uL/min in a mobile phase A (0.1% Formic Acid FA). Peptide were than separated on a nano Acquity UPLC Peptide BEH C18 Column 75 μm x 100 mm (Waters®) using a 60 min gradient 3-98% mobile phase B (98% (v/v) Acetonitrile, 0.1% (v/v) FA) at a flow rate of 40 ul/min.

The eluted peptides were run with an automated data-dependent acquisition (DDA) on top ten *m/z* using Xcalibur software. Peptides identification was run using Peaks X software on an *E. coli K*-12 database downloaded from NCBInr database, in which Hla H35L sequence was inserted. Search parameters as variable modifications were: methionine oxidation, glutamine and asparagine deamidation, trypsin cleavage (cleaves the C-term side of K or R unless next residue is P), peptide mass tolerance as 0.15 Da, peptide MS/MS tolerance as 0.15 Da, missed cleavage= 2, ion charge states: +2, +3, +4).

Suitable PTPs were selected based on the following criteria: (i) peptides specific for Hla, (ii) peptides showing strong MS signal intensities either for the parental or fragment ions, (iii) peptides that do not contain methionine and tryptophan residues, which are susceptible to oxidation, or N-terminal glutamine, to avoid cyclization.

*SRM-MS method set-up*

Detection and chromatographic elution optimizations of the peptides were performed with 1 pmol of synthetic peptides in a mix solution of light and heavy forms in 0.1% of FA using a reverse phase column (ACQUITY UPLC HSS T3 Column, 100Å, 1.8 μm, 2.1 mm X 30 mm, Waters, USA) coupled to a Xevo TQ triple quadrupole mass spectrometer associated to an UPLC (Waters, USA). The elution gradient is developed with mobile phase A 0.1% formic acid (FA) in water and B 0.1% FA in acetonitrile. The synthetic peptides were used to optimize collision energy (CE) values starting from the theoretical value, computed in silico by using PinPoint software calculated using the formula CE = 0.034 × (parent *m/z*) + 1.314 (15), and to validate the transitions in 50 μg matrix. The optimization of the chromatographic separation was performed in a SRM acquisition mode by using the optimized CE and the selected transitions, both in neat and in matrix background (see Table 3).

*Sample preparation for LC-SRM analysis of bacterial periplasmic extract.*

50 ug of PPE fractions were digested by using an in-stage-tips (iST) sample preparation method supplied by PreOmics (Martinsried, Germany). It is a 3-step protocol performed on a cartridge: 1) lysis denaturation reduction and Alkylation; 2) proteolytic digestion by LysC and Trypsin; 3) peptide elution operated as recommended by the provider. Recovered peptides were dried under vacuum at 45°C and resuspended in 0.1% FA to a final concentration of 1ug/uL and stored at - 20°C until the MS analysis.

*SRM-MS analysis.*

SRM was performed by injecting 10 ug of a periplasmic fraction digested with trypsin in column per run, and each sample were analyzed in triplicate. The following parameters were used: Q1 isolation window 1.0 m/z, Q3 Isolation window 0.7 m/z, 0.03 s of switching time (dwell time) from MS to MS/MS and collision cell exit and entrance potential set at 30 V. A spray voltage of 1,700 V was used with a heated ion transfer setting of 270 °C for desolvation. Data were acquired using MassLynx software (version 2.1.0; Waters). The dwell time was set to 30 ms and the scan width to 0.02 m/z. The peak area quantification was determined with TargetLynx software (version 1.0.0.1; Waters) after confirming the coelution of all transitions for each peptide and following the best practices reported in Carr et al. (Mol Cell Proteomics 13(3):907-917, 2014).

*PTP Dose-Range Linearity Responses and Hla Quantification.*

The dose-range linearity response of the selected PTPs was assessed in a lysed periplasmic bacterial sample prepared from *E. coli* glycocompetent cells (stGVXN9268 transformed with PglB plasmid) used as reference background to consider the matrix effect.

For Hla quantification, labeled PTPs (final concentration 0.1 pmol/μL) and non-labeled PTPs (final concentration from 1.6 pmol/ μl to 0,0125 pmol/μl) were spiked in 50 μg of periplasmic fraction prior to trypsin digestion, and SRM experiments.

For each PTP, concentrations were plotted as ratio of peak area light (variable)/peak area heavy (constant) and the fitted curve was used to obtain the concentration of selected PTP. The LLOQ for each PTP was set as the lowest concentration point on the fitted curve with an accuracy deviation ≤20%.

The Hla concentrations were reported in picograms per microgram of total protein extract and considering for all proteins the molecular mass average of the Hla-I (36981.54 Da), Hla-s (36955.46 Da) and Hla-v (36967.52 Da) isoforms. The quantification was obtained by the interpolation of each peptide-response value in the related dose-response linearity curve (Fig 4).

**RESULTS**

*Workflow for the design of new carrier for bioconjugation*

The workflow undertaken to design new carrier proteins for bioconjugation is reported in Fig 1.

The first step was the *in-silico* design of consensus sequences, predicted to be substrates of the PglB enzyme (Kowarik et al, 2006, EMBO J. 25:1957-1966) and able to generate tryptic peptides (referred as proteotypic peptides PTPs) suitable for the quantification of the extent of glycosylation by MS (Zhu et al, 2014, J Am Soc Mass Spectrom. 25:1012-7).

The designed PTPs were chemically synthetized in natural or heavy-labelled forms by incorporating 13C-15N in the arginine residue and investigated for their behavior in MS/MS analysis using a triple quadrupole instrument.

Once PTPs suitable for quantification by MS were identified, the corresponding sequences were introduced in a carrier protein, and the efficiency of PglB enzyme to recognize and glycosylate the new carrier is evaluated.

The site occupancy for each consensus sequence was then determined from the absolute quantification of the non-glycosylated form of the glycopeptide, by using isotopically labeled internal standards and a Selected Reaction Monitoring (SRM) approach. Two sets of heavy isotope labeled peptide standards are spiked into the sample before proteolysis, and the digested sample was analyzed by LC-SRM MS. One set of peptide standard was employed to determine the total carrier concentration, while the other standard set monitors the non-glycosylated part of the carrier. In this way, the abundance of the glycosylated portion of the protein was calculated by subtracting the non-glycosylated protein abundance from the overall protein concentration, and the site occupancy was then determined. The approach has been demonstrated to be successful for the quantification of naturally glycosylated eukaryotic protein (Zhu et al, 2014, J Am Soc Mass Spectrom. 25:1012-7).

As a proof of concept, new designed consensus sequences were introduced in a recombinant form of *S. aureus* Hla, substrate used as a carrier protein for the bioconjugation of *S. aureus* CP5 (see PCT/EP2018/085854, published as WO2019/121924). The carrier has been reported to be efficiently glycosylated by the insertion, in the position 130 of the consensus sequence, (-3)KDQNRTK(+3), where the Asn residue (in position 0) is the glycan acceptor. Unfortunately, it was found that this antigen design was not adapted for the quantification of glycosylation extent since digestion of the carrier by trypsin generates an unmodified peptide (-2)DQNR(+1) that is too short and hydrophilic to be monitored by LC-MS/MS. Different resin and gradients were tested without any success.

*In-silico design of consensus sequences*

The consensus sequence substrate of C. *jejuni* PglB has been well characterized (Kowarik et al, 2006, EMBO J. 25:1957-1966). The sequence is characterized by the presence of negatively charged side chain amino acid residues in the -2 position (Asp or Glu), and a Ser or Thr in position +2 of the Asn acceptor of the saccharide. Moreover, an efficient bioconjugation also requires that the consensus sites are in accessible and flexible loops of the carrier protein (Silverman and Imperiali, 2016, J. Biol. Chem. 291, 22001- 22010).

A statistical analysis of the occurrence of amino acids in the region from -6 to +6 of the glycosylated Asn residue found in 32 native C. *jejuni* glycoproteins is reported in Kowarik et al. EMBO J. 2006; 25(9): 1957-66, as shown in Fig 2A. The amino acid residues in position -3, -1, +1, +3 and +4, respectively, represented in bold black in grey boxes, were selected for the design of the four consensus sequences (Fig 2B). These residues were selected as frequently found and responding to the set-up criteria (limited selection of amino acid residues susceptible to post translational modification, hydrophobic and aromatic, see text "In-silico design of consensus sequences"). The amino acid Arg in position +5 (not reported in the statistical analysis), and the Lys residue in position -3 are the substrates of trypsin, required for the generation PTPs. The PTPs differ from each other only from the amino acid residue in position +1

With these minimal requirements in mind, four consensus sequences predicted to be substrates of the PglB and able to generate tryptic peptides were designed (Fig 2B). In detail, the following criteria were taken in to account: (i) to circumvent possible interference with carrier structure, the inserted consensus sequences did not exceed nine amino acid residues and the insertion of hydrophobic and aromatic amino acid residues was limited, (ii) to avoid underestimated quantification, amino acid residues prone to post-translational modifications such as oxidation (Met, Cys, Trp) and deamination (Asn and Gln) were limited, and the consensus sequences were designed to be substrate of trypsin, selected for its high specificity, efficacy and ability to generate C-terminal positively charged peptides, (iii) preferential amino acid residues surrounding the Asn, acceptor of the saccharide, evidenced from the comparison of a data set containing 32 active C. *jejuni* N-glycosylation sites were taken in consideration (4), and (iv) the newly designed consensus sequences were unique for the newly designed carrier isoforms.

The four designed consensus sequences were (-3)KDS**N**XTSAR(+5) in which X is an Ile, Ser, Val or Ala amino acid residue. After trypsin digestion, PTPs (-2)KDS**N**XTSAR(+5), named PTP-i, PTP-s, PTP-v and PTP-a according to the amino acid residue present in position +1 are generated (Fig. 2B).

*SRM assay for quantification of extent of glycosylation*

The behaviors of chemically synthesized PTP-i-s-v-a were evaluated in an SRM assays. The four PTPs were separated on a reverse phase C18 on-line with a triple-quadrupole mass spectrometer, with well distinct retention time ranging from 1.65 to 5.74 min (the minimal difference of 0,5 min was observed between peptide PTP-a and PTP-s) and with strong MS signals.

For each PTPs, four transitions (precursor/product pairs b4, y5, and y6 containing the selective amino acid residue specific for each glycosylation site, and y4, common to all peptides) were computed by PinPoint software and first optimized by in-neat injection (Table 1).

The transitions were then validated in an *E. coli* periplasmic fraction digested with trypsin to evaluate the effect of the matrix (Lange et al, 2008, Mol. Syst. Biol. 4:222). While the matrix had minor effects on the PTP-s, PTP-v and PTP-i performances (Table 1), it had deleterious effect on PTP-a, for which neither retention time and transitions were stable over repetitive analysis. For this reason, the bioconjugation consensus sequence (-5)KDSNATSAR(+3) was not further investigated.

For PTP-s, PTP-v and PTP-i, collision energies were optimized and a dose-response linearity curve was established adding to 50 μg *E. coli* periplasmic fraction a fixed amount of heavy forms of PTPs (0.1 pmol/μg) and scalar concentration of light PTPs (ranging from 0.0125 to 1.6 pmol/μg), before the trypsin digestion. The LLOQ for each PTP was set as the lowest concentration point on the fitted curve with an accuracy deviation ≤20% and was shown to be 0.05 pmoles/ug of periplasmic proteins for each peptide (Fig 4).

*The selected consensus sequences are substrates of PglB*

The three newly designed consensus sequences were inserted in position 130 of an optimized Hla antigen (see PCT/EP2018/085854, published as WO2019/121924) to generate the bioconjugates Hla-i-CP5, Hla-v-CP5 and Hla-s-CP5, which produce the PTP-i, PTP-v, PTP-s peptides, respectively, once digested with trypsin. Periplasms of glycocompetent *E. coli* strains bearing the machinery required for the bioconjugation were isolated and the conjugation of CP5 to Hla was assessed by Western-blot analysis using a murine serum that recognizes Hla-CP5 bioconjugate (Fig. 3). The carriers are characterized by a partial glycosylation pattern that was comparable among the three different constructs, although the extent of glycosylation could not be quantified from the Western blot.

*Quantification of extent of Hla-CP5 glycosylation*

The extent of glycosylation were assessed by SRM. In detail, site occupancies were determined by the following equation (Zhu et al 2015, J Am Soc Mass Spectrom. 25:1012-7):

$$\text{Site Occupancy } (\%) = \frac{(Total - unmodified) \; carrier \; concentration}{Total \; carrier \; concentration} x100$$

where the unmodified carrier concentrations were determined by the quantification of endogen PTP-i, PTP-v, PTP-s and the total carrier concentration are quantified by peptides specific for Hla.

To identify suitable Hla-specific peptides, recombinant Hla was digested with trypsin and the generated peptides were analyzed by LC-MS/MS. Three peptides 58V-66K (VFYSDFIDDK), 42T-50K (TGDLVTYK) and 225A-234K (AADNFLDPNK), named PTP-1, PTP-2 and PTP-3, respectively were selected. All the information regarding the three peptides (transitions, optimized CE, retention time and LLOQ in the matrix) are reported in Table 3.

Moreover, the SRM assay requires effective protease digestions of the carrier to ensure consistency in the quantification of each selected PTP. The efficiency of the digestion was checked by SDS/PAGE and by assessing that the number of missed cleavages was inferior to 2% (of identified peptide by LC-MS/MS (Biagini M et al, 2016, Proc Natl Acad Sci USA. 113:2714-9)

The interchangeability of the PTP-i-v-s and PTP-1-3 was demonstrated by spiking recombinant Hla, and known amount of each isotopically labeled PTPs in an *E. coli* periplasmic fraction before trypsin digestion.

Periplasmic fractions were isolated from *E. coli* glycocompetent strains expressing the different newly designed carriers, and known amount of each isotopically labeled PTPs were added before the trypsin digestion and analysis by LC-SRM. The experiments were performed from three independent digestions run in triplicate. The quantification of the PTPs were very reproducible between the runs with almost all coefficient of variation (CV) inferior to 1% while almost all the CV associated to the reproducibility of the digestion were inferior to 25% (Table 1); this value is in agreement with the error intended of the analysis (Hüttenhain et al 2009, Curr. Opin. Chem. Biol. 13 518-525). The concentrations of total and un-glycosylated amount of the carriers allowed to determine that Hla-i-CP5, Hla-v-CP5 and Hla-s-CP5 represented 46,9%, 52,7% and 48,3% of the total amount of the carrier expressed (Table 4). The similar extent of glycosylation was in agreement with the pattern observed in Western blot analysis. The introduction of the variable amino acid residue (Ser, Val, or Ile) in the consensus sequence did not significantly affected the efficacy of PglB enzyme to bioconjugate Hla carrier.

**Table 1: List of optimized SRM transitions for the selected PTPs.** For each PTP, PTPs name, peptide sequence, molecular mass, and the optimized transition and chromatographic condition stablished in SRM analysis are reported. For each selected fragment, the reproducibility of detection was assesed monitoring the TIC signal.

| Peptide name | Sequence | Molecular mass (Da) | Charge state | Q1 (m/z) precursor ion | CE | Q3 (m/z) fragment ions | Fragment ions | 1pmol loaded on column | | | 1pmol in matrix loaded on column | | |
|---|---|---|---|---|---|---|---|---|---|---|---|---|---|
| | | | | | | | | RT | TIC | Channel intensity | RT | TIC | Channel intensity |
| PTP-i | DSNITSAR | 862.89 | 2 | 432.21 | 16 | 434.21 | y4 | 5.74 | 4.4E+04 | 30700 | 4.97 | 4.49E+04 | 33800 |
| | | | | | 14 | 547,15 | y5 | | | 8030 | | | 5140 |
| | | | | | 14 | 561,47 | y6 | | | 7480 | | | 5700 |
| | | | | | 12 | 430,20 | b4 | | | 3980 | | | 2610 |
| PTP-s | DSNSTSAR | 836.81 | 2 | 419.19 | 16 | 434,27 | y4 | 1.65 | 3.32E+04 | 12000 | 1.66 | 3.3E+03 | 1070 |
| | | | | | 14 | 521,39 | y5 | | | 15100 | | | 1640 |
| | | | | | 14 | 634,96 | y6 | | | 8010 | | | 1180 |
| | | | | | 16 | 404,14 | b4 | | | 2480 | | | 145 |
| PTP-v | DSNVTSAR | 848.97 | 2 | 425.21 | 16 | 434,23 | y4 | 2.02 | 800E+04 | 46000 | 2.05 | 3.47E+04 | 18600 |
| | | | | | 18 | 533,30 | y5 | | | 12400 | | | 5400 |
| | | | | | 18 | 647,34 | y6 | | | 9670 | | | 6070 |
| | | | | | 12 | 416,38 | 54 | | | 12000 | | | 6690 |
| PTP-a | DSNATSAR | 320.81 | 2 | 411.19 | 15 | 434,24 | y4 | 1.70 | 3.68E+04 | 11400 | ----- | ------- | ------- |
| | | | | | 17 | 505,27 | y5 | | | 10300 | | | ------- |
| | | | | | 17 | 619,32 | y6 | | | 12400 | | | ------- |
| | | | | | 13 | 388,27 | b4 | | | 4710 | | | ------- |

Table 2: Optimised chromatographic gradient

| Time | mL/min | %A | %B |
|------|--------|------|------|
| 0.00 | 0.080 | 97.0 | 3,0 |
| 1.00 | 0.080 | 97.0 | 3.0 |
| 3.00 | 0.080 | 95.0 | 5.0 |
| 10.00 | 0.080 | 65.0 | 35.0 |
| 13.00 | 0.100 | 10.0 | 90.0 |
| 15.00 | 0.100 | 10.0 | 90.0 |
| 15.01 | 0.100 | 10.0 | 90.0 |
| 17.00 | 0.100 | 93.0 | 7.0 |
| 17.01 | 0.100 | 93.0 | 7.0 |

Table 3 Information regarding the three peptides 58V-66K (VFYSDFIDDK), 42T-50K (TGDLVTYK) and 225A-234K (AADNFLDPNK), named PTP-1, PTP-2 and PTP-3:
(transitions, optimized CE, retention time and LLOQ in the matrix

| Peptide name | Sequence | Q1 Precursor ion (m/z) | Q3 Fragment ions | Fragment ions | CE optimized | Charge state | RT (min) | LLOQ in matrix (pmol/ug) |
|---|---|---|---|---|---|---|---|---|
| PTP-1 | VFYSFIDDK | 567,2793 | 724,35 | y6 | 18 | 2 | 9.60 | 0,05 |
| | | | 887,41 | y7 | 18 | 2 | | |
| | | | 377,17 | y3 | 24 | 2 | | |
| PTP-1+ | VFYSFIDDK[HeavyK] | 571,2864 | 732,36 | y6 | 18 | 2 | 9.60 | 0,05 |
| | | | 895,43 | y7 | 18 | 2 | | |
| | | | 385,18 | y3 | 24 | 2 | | |
| PTP-2 | TGDLVTYDK | 506,2532 | 526,25 | y4 | 18 | 2 | 7.42 | 0,025 |
| | | | 625,32 | y5 | 18 | 2 | | |
| | | | 853,43 | y7 | 18 | 2 | | |
| ) PTP-2+ | TGDLVTYDK[HeavyK] | 510,2603 | 534,26 | y4 | 18 | 2 | 7.42 | 0,025 |
| | | | 633,33 | y5 | 18 | 2 | | |
| | | | 861,44 | y7 | 18 | 2 | | |
| PTP-3 | AADNFLDPNK | 552,7696 | 473,23 | y4 | 18 | 2 | 8.30 | 0,05 |
| | | | 586,32 | y5 | 18 | 2 | | |
| | | | 962,46 | y8 | 18 | 2 | | |
| | | | 733,39 | y6 | 18 | 2 | | |
| | | | 358,21 | y3 | 18 | 2 | | |
| PTP-3+ | AADNFLDPNK [HeavyK] | 556,7767 | 481,25 | y4 | 18 | 2 | 8.30 | 0,05 |
| | | | 594,33 | y5 | 18 | 2 | | |
| | | | 970,47 | y8 | 18 | 2 | | |
| | | | 741,40 | y6 | 18 | 2 | | |
| | | | 366,22 | y3 | 18 | 2 | | |

EP 3 770 269 A1

**Table 4: Quantification of the PTPs for the definition of % site occupancy. For** each Hla isoform (Hla-i-v-s) 3 proteolityc digestion were performed, quantifiyng both the total HLA and the unglycosilated form using the 2 sets of PTPs PTP-1-2-3 and PTP-i-v-s respectively. For each digestion the total protein amount avarage expressed as ng/$\mu$g of total extract and the CV between runs and digestion are reported. The obtained % site occupancy for each isoform were highlighted in grey.

| | | Total Hla | | | | | | | | | | unmodified glycosite | | |
|---|---|---|---|---|---|---|---|---|---|---|---|---|---|---|
| | Dig1 | Dig2 | Dig3 | | Dig1 | Dig2 | Dig3 | | Dig1 | Dig2 | Dig3 | | Dig1 | Dig2 | Dig3 |
| | VF***** | | | | AD***** | | | | TG***** | | | | PTP-1 | | |
| Hla-1 | 38,30 | 25,88 | 37,74 | | 27,05 | 22,34 | 32,71 | | 24,72 | 19,06 | 30,23 | | 16,06 | 10,58 | 19,46 |
| | 38,23 | 25,98 | 37,85 | | 26,58 | 22,28 | 32,50 | | 24,60 | 18,92 | 30,20 | | 15,46 | 10,98 | 20,14 |
| | 38,10 | 25,92 | 37,52 | | 26,93 | 22,34 | 32,70 | | 24,76 | 18,91 | 30,62 | | 15,15 | 10,91 | 19,73 |
| | Average | | | | Average | | | | Average | | | | Average | | |
| | 33,95 | | | | 27,27 | | | | 24,67 | | | | 15,38 | | |
| | CV (%) run | | | | CV(%) run | | | | CV (%) run | | | | CV (%) run | | |
| | 0,21 | 0,16 | 0,36 | | 0,73 | 0,12 | 0,29 | | 0,29 | 0,36 | 0,63 | | 2,43 | 1,60 | 1,41 |
| | CV (%) digestion | | | | CV (%) digestion | | | | CV (%) digestion | | | | CV (%) digestion | | |
| | 16,72 | | | | 15,48 | | | | 18,85 | | | | 23,76 | | |
| | VF**** | | | | AD***** | | | | TG***** | | | | PTP-2 | | |
| Hla-2 | 29,11 | *23,67 | 47,65 | | 20,72 | 17,55 | 31,23 | | 19,30 | 16,04 | 29,76 | | 10,64 | 8,54 | 18,36 |
| | 29,21 | 23,52 | 47,24 | | 20,72 | 17,62 | 31,41 | | 19,16 | 16,07 | 29,36 | | 10,81 | 8,98 | 16,65 |
| | 29,47 | 23,44 | 47,45 | | 19,95 | 17,28 | 31,95 | | 19,33 | 16,02 | 29,44 | | 10,74 | 9,19 | 17,54 |
| | Average | | | | Average | | | | Average | | | | Average | | |
| | 33,42 | | | | 23,16 | | | | 21,61 | | | | 12,38 | | |
| | CV(%) run | | | | CV (%) run | | | | CV (%) run | | | | CV (%) run | | |
| | 0,15 | 0,10 | 0,17 | | 0,37 | 0,15 | 0,31 | | 0,07 | 0,02 | 0,17 | | 0,07 | 0,27 | 0,70 |
| | CV (%) digestion | | | | CV (%) digestion | | | | CV (%) digestion | | | | CV (%) digestion | | |
| | 30,50 | | | | 26,10 | | | | 26,58 | | | | 29,93 | | |

|  | VF**** | | | | AD***** | | | | TG***** | | | | PTP-3 | | |
|---|---|---|---|---|---|---|---|---|---|---|---|---|---|---|---|
| Hla-3 | 30,72 | *24,80 | 39,98 | | 25,59 | 20,16 | 29,09 | | 23,03 | 18,57 | 26,89 | | 12,41 | 10,56 | 16,65 |
| | 30,72 | 25,08 | 39,58 | | 25,58 | 21,36 | 29,15 | | 23,17 | 18,45 | 27,40 | | 13,75 | 10,87 | 15,85 |
| | 31,06 | 24,91 | 39,98 | | 25,38 | 20,13 | 28,73 | | 23,20 | 18,40 | 27,11 | | 14,30 | 11,87 | 17,53 |
| | **Average** | | | | **Average** | | | | **Average** | | | | **Average** | | |
| | 31,87 | | | | 25,02 | | | | 22,91 | | | | 13,75 | | |
| | **CV (%) run** | | | | **CV (%) run** | | | | **CV (%) run** | | | | **CV (%) run** | | |
| | 0,52 | 0,47 | 0,48 | | 0,39 | 2,77 | 0,63 | | 0,31 | 0,39 | 0,77 | | 5,88 | 5,05 | 4,11 |
| | **CV (%) digestion** | | | | **CV (%) digestion** | | | | **CV (%) digestion** | | | | **CV (%) digestion** | | |
| | 19,25 | | | | 13,84 | | | | 15,44 | | | | 16,61 | | |

Aspects of the invention are summarized in following numbered paragraphs:

1. A modified carrier protein, modified in that it comprises one or more consensus sequence(s) comprising or consisting of a consensus sequence comprising or consisting of the following amino acid sequence:

$K/R-Z_{0-9}-D/E-X-N-Y-S/T-Z_{0-9}-K/R$

wherein X and Y are independently any amino acid except proline, and Z represents any amino acid; wherein optionally X and Y are independently any amino acid except proline, lysine or arginine, Z represents any amino acid except lysine or arginine, and preferably Z represents any amino acid except cysteine, methionine, asparagine, glutamine, lysine or arginine (eg SEQ ID NO: 47);
wherein said consensus sequence is optionally the amino acid sequence $K/R-D/E-X_1-N-X_2-S/T-Z_1-Z_2-K/R$ (SEQ ID NO 19), wherein $X_1$ and $X_2$ are independently any amino acid apart from proline, and wherein $X_1$ and $X_2$ and $Z_1$ and $Z_2$ are preferably not lysine or arginine, wherein optionally, wherein $X_1$ and $X_2$ and $Z_1$ and $Z_2$ are not cysteine, asparagine, glutamine, methionine or arginine.

1. A modified carrier protein according to paragraph 1 or paragraph 2, wherein said consensus sequence comprises or consists of the amino acid sequence of SEQ ID NO: 20, optionally any one of SEQ ID Nos: 42-45, optionally SEQ ID Nos 42-44.

2. A modified carrier protein according to anyone of paragraphs 1-3, wherein said consensus sequence (i) has been substituted for one or more amino acids of the carrier protein sequence, or (ii) has been inserted into the carrier protein sequence.

3. A modified carrier protein according to any one of paragraphs 1- 5, comprising more than one said consensus sequence, optionally at least 2, 3, 4 or 5 consensus sequences.

4. A modified carrier protein according to paragraph 6, wherein all of said consensus sequences have a different amino acid sequence.

5. A modified carrier protein according to any one of paragraphs 1-7, wherein the carrier protein is CRM197, TT from *Clostridium tetani,* EPA from *P. aeruginosa,* Hcp1 from *P. aeruginosa,* Hla from *S. aureus,* ClfA from *S. aureus,* MBP from *E.,* PspA from *E. coli,* or MtrE from *N. gonorrhoeae.*

6. A modified carrier protein according to paragraph 8, wherein the carrier protein comprises or consists of an amino acid sequence of any one of SEQ ID Nos: 1 to 16 or an amino acid sequence at least 80%, 85%, 90%, 92%, 95%, 96%, 97%, 98% or 99% identical to any one of SEQ ID NOs. 1 to 16.

7. A modified carrier protein according to paragraph 8, wherein the modified carrier protein comprises or consists of an amino acid sequence at least 80%, 85%, 90%, 92%, 95%, 96% or 97% identical to any one of SEQ ID NOs. 1 to 16.

8. The modified carrier protein of any one of paragraphs 1-10, wherein the modified carrier protein is glycosylated.

9. A conjugate (e.g. bioconjugate) comprising a modified carrier protein of any one of paragraphs 1-11, wherein the modified carrier protein is linked to a polysaccharide.

10. The conjugate (e.g. bioconjugate) of paragraph 12, wherein the polysccharide is linked to an amino acid on the modified carrier protein selected from asparagine, aspartic acid, glutamic acid, lysine, cysteine, tyrosine, histidine, arginine or tryptophan (e.g. asparagine).

11. The conjugate (e.g. bioconjugate) of paragraph 12 or paragraph 13, wherein the polysaccharide is a bacterial capsular polysaccharide

12. The conjugate (e.g. bioconjugate) of paragraph 14, wherein the capsular polysaccharide is selected from the group consisting of: *Staphylococcus aureus* type 5 capsular saccharide, *Staphylococcus aureus* type 8 capsular saccharide, *N. meningitidis* serogroup A capsular saccharide (MenA), *N. meningitidis* serogroup C capsular saccharide (MenC), *N. meningitidis* serogroup Y capsular saccharide (MenY), *N. men-*

*ingitidis* serogroup W capsular saccharide (MenW), *H. influenzae* type b capsular saccharide (Hib), Group B Streptococcus group I capsular saccharide, Group B Streptococcus group II capsular saccharide, Group B Streptococcus group III capsular saccharide, Group B Streptococcus group IV capsular saccharide, Group B Streptococcus group V capsular saccharide, Vi saccharide from *Salmonella typhi, N. meningitidis* LPS (such as L3 and/or L2), *M. catarrhalis* LPS, *H. influenzae* LPS, Shigella O-antigens, *P.aeruginosa* O-antigens, *E. coli* O-antigens or *S. pneumoniae* a *S. aureus* capsular polysaccharide.

13. The conjugate (e.g. bioconjugate) of paragraph 14 or paragraph 15, wherein the capsular polysaccharide is from the same organism as the carrier protein.

14. The conjugate (e.g. bioconjugate) of paragraph 16, wherein the capsular polysaccharide is from a different organism to the carrier protein.

15. A polynucleotide encoding the modified carrier protein of any one of paragraphs 1-10.

16. A vector comprising the polynucleotide of paragraph 18.

17. A host cell comprising:

    a. one or more nucleic acids that encode glycosyltransferase(s);

    b. a nucleic acid that encodes an oligosaccharyl transferase;

    c. a nucleic acid that encodes a modified carrier protein according to any one of paragraphs 1-10; and optionally

    d. a nucleic acid that encodes a polymerase (*e.g. wzy*).

18. The host cell of paragraph 20, wherein said host cell comprises (a) a glycosyltransferase that assembles a hexose monosaccharide derivative onto undecaprenyl pyrophosphate (Und-PP) and (b) one or more glycosyltransferases capable of adding a monosaccharide to the hexose monosaccharide derivative assembled on Und-PP.

19. The host cell of paragraph 21, wherein said glycosyltransferase that assembles a hexose monosaccharide derivative onto Und-PP is heterologous to the host cell and/or heterologous to one or more of the genes that encode glycosyltransferase(s) optionally wherein said glycosyltransferase that assembles a hexose monosaccharide derivative onto Und-PP is from *Escherichia* species, *Shigella* species, *Klebsiella* species, *Xhantomonas* species, *Salmonella* species, *Yersinia* species, *Aeromonas* species, *Francisella* species, *Helicobacter* species, *Proteus* species, *Lactococcus* species, *Lactobacillus* species, *Pseudomonas* species, *Corynebacterium* species, *Streptomyces* species, *Streptococcus* species, *Enterococcus* species, *Staphylococcus* species, *Bacillus* species, *Clostridium* species, *Listeria* species, or *Campylobacter* species, optionally *wecA* (e.g. *wecA* from *E. coli*).

20. The host cell of any one of paragraphs 19-22, wherein said hexose monosaccharide derivative is any monosaccharide in which C-2 position is modified with an acetamido group such as N-acetylglucosamine (GlcNAc), N-acetylgalactoseamine (GalNAc), 2,4-Diacetamido-2,4,6-trideoxyhexose (DATDH). N-acetylfucoseamine (FucNAc), or N-acetylquinovosamine (QuiNAc).

21. The host cell of any one of paragraphs 19-23, wherein said one or more glycosyltransferases capable of adding a monosaccharide to the hexose monosaccharide derivative assembled on Und-PP is the galactofuranosyltransferase (*wbeY*) from *E. coli* O28 or the galactofuranosyltransferase (*wfdK*) from *E. coli* O167 or are the galactofuranosyltransferase (*wbeY*) from *E. coli* O28 and the galactofuranosyltransferase (*wfdK*) from *E. coli* O167.

22. The host cell of any one of paragraphs 19-24, wherein the glycosyltransferases comprise a glycosyltransferase that is capable of adding the hexose monosaccharide present at the reducing end of the first repeat unit of the donor oligosaccharide or polysaccharide to the hexose monosaccharide derivative, optionally wherein said one or more glycosyltransferases capable of adding a monosaccharide to the hexose monosaccharide derivative comprise galactosyltransferase (*wciP*), optionally from *E. coli* O21, and optionally comprising a glycosyltransferase that is capable of adding the monosaccharide that is adjacent to the hexose monosaccharide present at the reducing end of the first repeat unit of the donor oligosaccharide or polysaccharide to the hexose monosaccharide present at the reducing end of the first repeat unit of the donor oligosaccharide or polysaccharide, optionally glucosyltransferase (*wciQ*), optionally from *E. coli* O21.

23. The host cell of any one of paragraphs 19-25 wherein the oligosaccharyl transferase is derived from *Campylobacter jejuni,* optionally wherein said oligosaccharyl transferase is pglB of *C. jejuni,* optionally wherein the pglB gene of *C. jejuni* is integrated into the host cell genome and optionally wherein at least one gene of the host cell has been functionally inactivated or deleted, optionally wherein the *waaL* gene of the host cell has been functionally inactivated or deleted, optionally wherein the *waaL* gene of the host cell has been replaced by a nucleic acid encoding an oligosaccharyltransferase, optionally wherein the *waaL* gene of the host cell has been replaced by *C. jejuni pglB.*

24. The host cell of any one of paragraphs 19-26, wherein the nucleic acid that encodes the modified carrier protein is in a plasmid in the host cell.

25. The host cell of any one of paragraphs 19-26, wherein the nucleic acid that encodes the modified carrier protein is integrated into the genome of the host cell.

26. The host cell of any one of paragraphs 19-28, wherein the host cell is *E. coli.*

27. A method of producing a bioconjugate that comprises a modified carrier protein linked to a saccharide, said method comprising (i) culturing the host cell of any one of paragraphs 19-29 under conditions suitable for the production of proteins and (ii) isolating the bioconjugate.

28. A bioconjugate produced by the process of paragraph 30, wherein said bioconjugate comprises a polysaccharide linked to a modified carrier protein.

29. An immunogenic composition comprising the modified carrier protein of any one of paragraphs 1-11, or the conjugate of any one of paragraphs 12-17, or the bioconjugate of paragraph 31.

30. A method of making the immunogenic composition of paragraph 32 comprising the step of mixing the modified carrier protein or the conjugate or the bioconjugate with a pharmaceutically acceptable excipient or carrier.

31. A vaccine comprising the immunogenic composition of paragraph 33 and a pharmaceutically acceptable excipient or carrier.

32. A method for the treatment or prevention of a bacterial infection in a subject in need thereof comprising administering to said subject a therapeutically effective amount of the modified carrier protein of any one of paragraphs 1-11, or the conjugate of any one of paragraphs 12-17, or the bioconjugate of paragraph 31.

33. A method of immunising a human host against a bacterial infection comprising administering to the host an immunoprotective dose of the modified carrier protein of any one of paragraphs 1-11, or the conjugate of any one of paragraphs 12-17, or the bioconjugate of paragraph 31.

34. A method of inducing an immune response to a bacterium in a subject, the method comprising administering a therapeutically or prophylactically effective amount of the modified carrier protein any one of paragraphs 1-11, or the conjugate of any one of paragraphs 12-17, or the bioconjugate of paragraph 31 to said subject.

35. A modified carrier protein of any one of paragraphs 1-11, or the conjugate of any one of paragraphs 12-17, or the bioconjugate of paragraph 31, for use in the treatment or prevention of a disease caused by bacterial infection.

36. Use of the modified carrier protein of any one of paragraphs 1-11, or the conjugate of any one of paragraphs 12-17, or the bioconjugate of paragraph 31 in the manufacture of a medicament for the treatment or prevention of a disease caused by bacterial infection.

37. The method of any one of paragraphs 35-37, or a carrier protein, conjugate or bioconjugate for use of paragraph 38, or the use of paragraph 39, wherein said bacterium or bacterial infection is selected from the group consisting of *Staphylococcus aureus, N. meningitidis, H. influenzae, H. influenzae* type b, Group B Streptococcus, *S. typhi, M. catarrhalis* LPS, *S. flexneri, P.aeruginosa, E. coli* or *S. pneumoniae.*

38. A method of measuring the level of glycosylation site occupancy of a carrier protein according to any one of paragraphs 1 to 11, said method comprising: digesting the glycosylated carrier protein with a protease; subjecting the digested protein to LC-MS; determining the concentration U of unmodified carrier protein; determining the concentration T of total carrier protein; and calculating glycosylation site occupancy according to the following equation:

$$\text{Site Occupancy (\%)} = \frac{(Total - unmodified)\ carrier\ concentration}{Total\ carrier\ concentration} x100$$

39. A method according to paragraph 39, wherein the concentration U of unmodified carrier protein is determined by determining the concentration of a peptide fragment corresponding to a consensus sequence .

40. A method according to paragraph 39 or paragraph 40, wherein the concentration T of total carrier protein is determined by determining the concentration of one or more peptide fragments which are unique to said carrier protein.

41. A method according to any one of paragraphs 39 to 41, wherein the protease is trypsin.

SEQUENCE LISTINGS

SEQ ID NO:1 Amino acid sequence of mature wild-type EPA. Bold and underlined are the residues substituted/removed for detoxification. Organism: *Pseudomonas aeruginosa.*

```
AEEAFDLWNECAKACVLDLKDGVRSSRMSVDPAIADTNGQGVLHYSMVLEGGNDALKLAIDNALSITSDGLTIR
LEGGVEPNKPVRYSYTRQARGSWSLNWLVPIGHEKPSNIKVFIHELNAGNQLSHMSPIYTIEMGDELLAKLARD
ATFFVRAHESNEMQPTLAISHAGVSVVMAQAQPRREKRWSEWASGKVLCLLDPLDGVYNYLAQQRCNLDDTWEG
KIYRVLAGNPAKHDLDIKPTVISHRLHFPEGGSLAALTAHQACHLPLEAFTRHRQPRGWEQLEQCGYPVQRLVA
LYLAARLSWNQVDQVIRNALASPGSGGDLGEAIREQPEQARLALTLAAAESERFVRQGTGNDEAGAASADVVSL
TCPVAAGECAGPADSGDALLERNYPTGAEFLGDGGDVSFSTRGTQNWTVERLLQAHRQLEERGYVFVGYHGTFL
EAAQSIVFGGVRARSQDLDAIWRGFYIAGDPALAYGYAQDQEPDARGRIRNGALLRVYVPRWSLPGFYRTGLTL
AAPEAAGEVERLIGHPLPLRLDAITGPEEEGGR**LE**TILGWPLAERTVVIPSAIPTDPRNVGGDLDPSSIPDKEQ
AISALPDYASQPGKPPREDLK
```

SEQ ID NO:2 Amino acid sequence of EPA with L552V/ΔE553 detoxifying mutation (bold, underlined). Artificial sequence.

```
AEEAFDLWNECAKACVLDLKDGVRSSRMSVDPAIADTNGQGVLHYSMVLEGGNDALKLAIDNALSITSDGLTIR
LEGGVEPNKPVRYSYTRQARGSWSLNWLVPIGHEKPSNIKVFIHELNAGNQLSHMSPIYTIEMGDELLAKLARD
ATFFVRAHESNEMQPTLAISHAGVSVVMAQAQPRREKRWSEWASGKVLCLLDPLDGVYNYLAQQRCNLDDTWEG
KIYRVLAGNPAKHDLDIKPTVISHRLHFPEGGSLAALTAHQACHLPLEAFTRHRQPRGWEQLEQCGYPVQRLVA
LYLAARLSWNQVDQVIRNALASPGSGGDLGEAIREQPEQARLALTLAAAESERFVRQGTGNDEAGAASADVVSL
TCPVAAGECAGPADSGDALLERNYPTGAEFLGDGGDVSFSTRGTQNWTVERLLQAHRQLEERGYVFVGYHGTFL
EAAQSIVFGGVRARSQDLDAIWRGFYIAGDPALAYGYAQDQEPDARGRIRNGALLRVYVPRWSLPGFYRTGLTL
AAPEAAGEVERLIGHPLPLRLDAITGPEEEGGRVTILGWPLAERTVVIPSAIPTDPRNVGGDLDPSSIPDKEQA
ISALPDYASQPGKPPREDLK
```

SEQ ID NO: 3: Tetanus toxin precursor TT (AA 1-1315) without initial methionine. Organism: *Clostridium tetani*

```
MPITINNFRYSDPVNNDTIIMMEPPYCKGLDIYYKAFKITDRIWIVPERYEFGTKPEDFNPPSSLIEGASEYYDPNY
LRTDSDKDRFLQTMVKLFNRIKNNVAGEALLDKIINAIPYLGNSYSLLDKFDTNSNSVSFNLLEQDPSGATTKSAML
TNLIIFGPGPVLNKNEVRGIVLRVDNKNYFPCRDGFGSIMQMAFCPEYVPTFDNVIENITSLTIGKSKYFQDPALLL
MHELIHVLHGLYGMQVSSHEIIPSKQEIYMQHTYPISAEELFTFGGQDANLISIDIKNDLYEKTLNDYKAIANKLSQ
VTSCNDPNIDIDSYKQIYQQKYQFDKDSNGQYIVNEDKFQILYNSIMYGFTEIELGKKFNIKTRLSYFSMNHDPVKI
PNLLDDTIYNDTEGFNIESKDLKSEYKGQNMRVNTNAFRNVDGSGLVSKLIGLCKKIIPPTNIRENLYNRTASLTDL
GGELCIKIKNEDLTFIAEKNSFSEEPFQDEIVSYNTKNKPLNFNYSLDKIIVDYNLQSKITLPNDRTTPVTKGIPYA
PEYKSNAASTIEIHNIDDNTIYQYLYAQKSPTTLQRITMTNSVDDALINSTKIYSYFPSVISKVNQGAQGILFLQWV
RDIIDDFTNESSQKTTIDKISDVSTIVPYIGPALNIVKQGYEGNFIGALETTGVVLLLEYIPEITLPVIAALSIAES
STQKEKIIKTIDNFLEKRYEKWIEVYKLVKAKWLGTVNTQFQKRSYQMYRSLEYQVDAIKKIIDYEYKIYSGPDKEQ
IADEINNLKNKLEEKANKAMININIFMRESSRSFLVNQMINEAKKQLLEFDTQSKNILMQYIKANSKFIGITELKKL
ESKINKVFSTPIPFSYSKNLDCWVDNEEDIDVILKKSTILNLDINNDIISDISGFNSSVITYPDAQLVPGINGKAIH
LVNNESSEVIVHKAMDIEYNDMFNNFTVSFWLRVPKVSASHLEQYGTNEYSIISSMKKHSLSIGSGWSVSLKGNNLI
WTLKDSAGEVRQITFRDLPDKFNAYLANKWVFITITNDRLSSANLYINGVLMGSAEITGLGAIREDNNITLKLDRCN
NNNQYVSIDKFRIFCKALNPKEIEKLYTSYLSITFLRDFWGNPLRYDTEYYLIPVASSSKDVQLKNITDYMYLTNAP
SYTNGKLNIYYRRLYNGLKFIIKRYTPNNEIDSFVKSGDFIKLYVSYNNNEHIVGYPKDGNAFNNLDRILRVGYNAP
GIPLYKKMEAVKLRDLKTYSVQLKLYDDKNASLGLVGTHNGQIGNDPNRDILIASNWYFNHLKDKILGCDWYFVPTD
EGWTND
```

SEQ ID NO: 4 Diphtheria toxin (DT). Organism: *Corynebacterium diphtheriai*

```
GADDVVDSSKSFVMENFSSYHGTKPGYVDSIQKGIQKPKSGTQGNYDDDWKGFYSTDNKYDAAGYSVDNENPLS
GKAGGVVKVTYPGLTKVLALKVDNAETIKKELGLSLTEPLMEQVGTEEFIKRFGDGASRVVLSLPFAEGSSSVE
YINNWEQAKALSVELEINFETRGKRGQDAMYEYMAQACAGNRVRRSVGSSLSCINLDWDVIRDKTKTKIESLKE
HGPIKNKMSESPNKTVSEEKAKQYLEEFHQTALEHPELSELKTVTGTNPVFAGANYAAWAVNVAQVIDSETADN
LEKTTAALSILPGIGSVMGIADGAVHHNTEEIVAQSIALSSLMVAQAIPLVGELVDIGFAAYNFVESIINLFQV
VHNSYNRPAYSPGHKTQPFLHDGYAVSWNTVEDSIIRTGFQGESGHDIKITAENTPLPIAGVLLPTIPGKLDVN
KSKTHISVNGRKIRMRCRAIDGDVTFCRPKSPVYVGNGVHANLHVAFHRSSSEKIHSNEISSDSIGVLGYQKTV
DHTKVNSKLSLFFEIKS
```

SEQ ID NO: 5: CRM197, non-toxic mutant of diphtheria toxin. Artificial sequence.

```
GADDVVDSSKSFVMENFSSYHGTKPGYVDSIQKGIQKPKSGTQGNYDDDWKEFYSTDNKYDAAGYSVDNENPLS
GKAGGVVKVTYPGLTKVLALKVDNAETIKKELGLSLTEPLMEQVGTEEFIKRFGDGASRVVLSLPFAEGSSSVE
YINNWEQAKALSVELEINFETRGKRGQDAMYEYMAQACAGNRVRRSVGSSLSCINLDWDVIRDKTKTKIESLKE
HGPIKNKMSESPNKTVSEEKAKQYLEEFHQTALEHPELSELKTVTGTNPVFAGANYAAWAVNVAQVIDSETADN
LEKTTAALSILPGIGSVMGIADGAVHHNTEEIVAQSIALSSLMVAQAIPLVGELVDIGFAAYNFVESIINLFQV
VHNSYNRPAYSPGHKTQPFLHDGYAVSWNTVEDSIIRTGFQGESGHDIKITAENTPLPIAGVLLPTIPGKLDVN
KSKTHISVNGRKIRMRCRAIDGDVTFCRPKSPVYVGNGVHANLHVAFHRSSSEKIHSNEISSDSIGVLGYQKTV
DHTKVNSKLSLFFEIKS
```

SEQ ID NO: 6: Hcp1. Organism: *Pseudomonas aeruginosa*

```
MAVDMFIKIGDVKGESKDKTHAEEIDVLAWSWGMSQSGSMHMGGGGGAGKVNVQDLSFTKYIDKSTPNLMMACS
SGKHYPQAKLTIRKAGGENQVEYLIITLKEVLVSSVSTGGSGGEDRLTENVTLNFAQVQVDYQPQKADGAKDGG
PIKYGWNIRQNVQA
```

SEQ ID NO: 7: PspA, phage shock protein A without initial methionine. Organism: *Escherichia coli*

```
GIFSRFADIVNANINALLEKAEDPQKLVRLMIQEMEDTLVEVRSTSARALAEKKQLTRRIEQASAREVEWQEKA
ELALLKEREDLARAALIEKQKLTDLIKSLEHEVTLVDDTLARMKKEIGELENKLSETRARQQALMLRHQAANSS
RDVRRQLDSGKLDEAMARFESFERRIDQMEAEAESHSFGKQKSLDDQFAELKADDAISEQLAQLKAKMKQDNQ
```

SEQ ID NO: 8: MBP, Maltose/maltodextrin binding protein. Organism: *Escherichia coli*

```
MKIKTGARILALSALTTMMFSASALAKIEEGKLVIWINGDKGYNGLAEVGKKFEKDTGIKVTVEHPDKLEEKFP
QVAATGDGPDIIFWAHDRFGGYAQSGLLAEITPDKAFQDKLYPFTWDAVRYNGKLIAYPIAVEALSLIYNKDLL
PNPPKTWEEIPALDKELKAKGKSALMFNLQEPYFTWPLIAADGGYAFKYENGKYDIKDVGVDNAGAKAGLTFLV
DLIKNKHMNADTDYSIAEAAFNKGETAMTINGPWAWSNIDTSKVNYGVTVLPTFKGQPSKPFVGVLSAGINAAS
PNKELAKEFLENYLLTDEGLEAVNKDKPLGAVALKSYEEELAKDPRIAATMENAQKGEIMPNIPQMSAFWYAVR
TAVINAASGRQTVDEALKDAQTRITK
```

SEQ ID NO: 9: mature mtrE, Membrante Transporter E. Organism: *Neisseria gonorrhoeae*

```
MIPQYEQPKVEVAETFQNDTSVSSIRAVDLGWHDYFADPRLQKLIDIALERNTSLRTAVLNSEIYRKQYMIERN
NLLPTLAANANGSRQGSLSGGNVSSSYNVGLGAASYELDLFGRVRSSSEAALQGYFASVANRDAAHLSLIATVA
KAYFNERYAEEAMSLAQRVLKTREETYNAVRIAVQGRRDFRRRPAPAEALIESAKADYAHAARSREQARNALAT
LINRPIPEDLPAGLPLDKQFFVEKLPAGLSSEVLLDRPDIRAAEHALKQANANIGAARAAFFPSIRLTGSVGTG
SVELGGLFKSGTGVWAFAPSITLPIFTWGTNKANLDVAKLRQQAQIVAYESAVQSAFQDVANALAAREQLDKAY
DALSKQSRASKEALRLVGLRYKHGVSGALDLLDAERSSYSAEGAALSAQLTRAENLADLYKALGGGLKRDTQTG
K
```

SEQ ID NO: 10 - Wild-type mature ClfAN1 N2N3. Organism: *Staphylococcus aureus.*

```
ASENSVTQSDSASNESKSNDSSSVSAAPKTDDTNVSDTKTSSNTNNGETSVAQNPAQQETTQSSSTNATTEETP
VTGEATTTTTNQANTPATTQSSNTNAEELVNQTSNETTFNDTNTVSSVNSPQNSTNAENVSTTQDTSTEATPSN
NESAPQSTDASNKDVVNQAVNTSAPRMRAFSLAAVAADAPAAGTDITNQLTNVTVGIDSGTTVYPHQAGYVKLN
YGFSVPNSAVKGDTFKITVPKELNLNGVTSTAKVPPIMAGDQVLANGVIDSDGNVIYFTDYVNTKDDVKATLT
MPAYIDPENVKKTGNVTLATGIGSTTANKTVLVDYEKYGKFYNLSIKGTIDQIDKTNNTYRQTIYVNPSGDNVI
APVLTGNLKPNTDSNALIDQQNTSIKVYKVDNAADLSESYFVNPENFEDVTNSVNITFPNPNQYKVEFNTPDDQ
ITTPYIVVVNGHIDPNSKGDLALRSTLYGYNSNIIWRSMSWDNEVAFNNGSGSGDGIDKPVVPEQPDEPGEIEP
IPED
```

SEQ ID NO:11 - Wild-type mature ClfAN2N3. Organism: *Staphylococcus aureus.*

```
VAADAPAAGTDITNQLTNVTVGIDSGTTVYPHQAGYVKLNYGFSVPNSAVKGDTFKITVPKELNLNGVTSTAKV
PPIMAGDQVLANGVIDSDGNVIYFTDYVNTKDDVKATLTMPAYIDPENVKKTGNVTLATGIGSTTANKTVLVD
YEKYGKFYNLSIKGTIDQIDKTNNTYRQTIYVNPSGDNVIAPVLTGNLKPNTDSNALIDQQNTSIKVYKVDNAA
DLSESYFVNPENFEDVTNSVNITFPNPNQYKVEFNTPDDQITTPYIVVVNGHIDPNSKGDLALRSTLYGYNSNI
IWRSMSWDNEVAFNNGSGSGDGIDKPVVPEQPDEPGEIEPIPED
```

SEQ ID NO: 12 - ClfAN2N3P116S/Y118A. Artificial sequence.

```
VAADAPAAGTDITNQLTNVTVGIDSGTTVYPHQAGYVKLNYGFSVPNSAVKGDTFKITVPKELNLNGVTSTAKV
PPIMAGDQVLANGVIDSDGNVIYFTDYVNTKDDVKATLTMSAIDPENVKKTGNVTLATGIGSTTANKTVLVD
YEKYGKFYNLSIKGTIDQIDKTNNTYRQTIYVNPSGDNVIAPVLTGNLKPNTDSNALIDQQNTSIKVYKVDNAA
DLSESYFVNPENFEDVTNSVNITFPNPNQYKVEFNTPDDQITTPYIVVVNGHIDPNSKGDLALRSTLYGYNSNI
IWRSMSWDNEVAFNNGSGSGDGIDKPVVPEQPDEPGEIEP*I*PED
```

SEQ ID NO: 13: Amino acid sequence of mature wild-type Hla. Organism: *Staphylococcus aureus.*

ADSDINIKTGTTDIGSNTTVKTGDLVTYDKENGMHKKVFYSFIDDKNHNKKLLVIRTKGTIAGQYRVYSE
EGANKSGLAWPSAFKVQLQLPDNEVAQISDYYPRNSIDTKEYMSTLTYGFNGNVTGDDTGKIGGLIGANV
SIGHTLKYVQPDFKTILESPTDKKVGWKVIFNNMVNQNWGPYDRDSWNPVYGNQLFMKTRNGSMKAADNF
LDPNKASSLLSSGFSPDFATVITMDRKASKQQTNIDVIYERVRDDYQLHWTSTNWKGTNTKDKWIDRSSE
RYKIDWEKEEMTN

SEQ ID NO: 14 - Amino acid sequence of mature HlaH35L. Artificial sequence.

ADSDINIKTGTTDIGSNTTVKTGDLVTYDKENGMLKKVFYSFIDDKNHNKKLLVIRTKGTIAGQYRVYSE
EGANKSGLAWPSAFKVQLQLPDNEVAQISDYYPRNSIDTKEYMSTLTYGFNGNVTGDDTGKIGGLIGANV
SIGHTLKYVQPDFKTILESPTDKKVGWKVIFNNMVNQNWGPYDRDSWNPVYGNQLFMKTRNGSMKAADNF
LDPNKASSLLSSGFSPDFATVITMDRKASKQQTNIDVIYERVRDDYQLHWTSTNWKGTNTKDKWIDRSSE
RYKIDWEKEEMTN

SEQ ID NO: 15 - Amino acid sequence of mature Hla H35L/H48C/G122C, Artificial sequence.

ADSDINIKTGTTDIGSNTTVKTGDLVTYDKENGMLKKVFYSFIDDKNCNKKLLVIRTKGTIAGQYRVYSEEGAN
KSGLAWPSAFKVQLQLPDNEVAQISDYYPRNSIDTKEYMSTLTYGFNCNVTGDDTGKIGGLIGANVSIGHTLKY
VQPDFKTILESPTDKKVGWKVIFNNMVNQNWGPYDRDSWNPVYGNQLFMKTRNGSMKAADNFLDPNKASSLLSS
GFSPDFATVITMDRKASKQQTNIDVIYERVRDDYQLHWTSTNWKGTNTKDKWIDRSSERYKIDWEKEEMTN

SEQ ID NO: 16: HlaPSGS. Artificial sequence.

ADSDINIKTGTTDIGSNTTVKTGDLVTYDKENGMHKKVFYSFIDDKNHNKKLLVIRTKGTIAGQYRVYSEEGAN
KSGLAWPSAFKVQLQLPDNEVAQISDYYPRNSIDTPSGSVQPDFKTILESPTDKKVGWKVIFNNMVNQNWGPYD
RDSWNPVYGNQLFMKTRNGSMKAADNFLDPNKASSLLSSGFSPDFATVITMDRKASKQQTNIDVIYERVRDDYQ
LHWTSTNWKGTNTKDKWIDRSSERYKIDWEKEEMTN

SEQ ID NO: 17: Minimal PglB glycosite consensus sequence. Artificial sequence.
D/E-$X_1$-N-$X_2$-S/T
wherein $X_1$ and $X_2$ are any amino acid apart from proline.

SEQ ID NO : 18: Full PglB glycosite consensus sequence. Artificial sequence
K-D/E-$X_1$-N-$X_2$-S/T-K
wherein $X_1$ and $X_2$ are any amino acid apart from proline.

SEQ ID NO: 19: MS quantification-compatible PglB glycosite consensus sequence. Artificial sequence.
K/R-D/E-$X_1$-N-$X_2$-S/T-$Z_1$-$Z_2$-R/K
wherein $X_1$ and $X_2$ are any amino acid apart from proline, and $Z_1$ and $Z_2$.are not lysine or arginine.

SEQ ID NO: 20: MS quantification-compatible PglB glycosite consensus sequence. Artificial sequence.
K-D/E-$X_1$-N-$X_2$-S/T-S-A-R
wherein $X_1$ and $X_2$ are any amino acid apart from proline.

SEQ ID NO: 21 - FlgI signal sequence. Organism: *Shigella flexneri*
MIKFLSALILLLVTTAAQA

SEQ ID NO: 22 - OmpA signal sequence. Organism: *Escherichia coli*
MKKTAIAIAVALAGFATVAQA

SEQ ID NO: 23 - MalE signal sequence. Organism: *Escherichia coli*
MKIKTGARILALSALTTMMFSASALA

SEQ ID NO: 24 - PelB signal sequence. Organism: *Pectobacterium carotovorum (Erwinia carotovora).*

MKYLLPTAAAGLLLLAAQPAMA

SEQ ID NO: 25 - LTIIb signal sequence. Organism: *Escherichia coli*
MSFKKIIKAFVIMAALVSVQAHA

SEQ ID NO: 26 - XynA signal sequence. Organism: *Bacillus subtilis*
MFKFKKKFLVGLTAAFMSISMFSATASA

SEQ ID NO: 27 - DsbA signal sequence. Organism: *Escherichia coli*
MKKIWLALAGLVLAFSASA

SEQ ID NO: 28 - TolB signal sequence. Organism: *Escherichia coli*
MKQALRVAFGFLILWASVLHA

SEQ ID NO: 29 - SipA signal sequence. Organism: *Streptococcus agalactiae*
MKMNKKVLLTSTMAASLLSVASVQAS

SEQ ID NO: 30: Amino acid sequence of Hla H35L/H48C/G122C with N-terminal S, FlgI signal sequence, C-terminal GSHRHR, and KDQNRTK substitution for residue K131. Artificial sequence.

MIKFLSALILLLVTTAAQASADSDINIKTGTTDIGSNTTVKTGDLVTYDKENGMLKKVFYSFIDDKNCNKKLLV
IRTKGTIAGQYRVYSEEGANKSGLAWPSAFKVQLQLPDNEVAQISDYYPRNSIDTKEYMSTLTYGFNCNVTGDD
TGKDQNRTKIGGLIGANVSIGHTLKYVQPDFKTILESPTDKKVGWKVIFNNMVNQNWGPYDRDSWNPVYGNQLF
MKTRNGSMKAADNFLDPNKASSLLSSGFSPDFATVITMDRKASKQQTNIDVIYERVRDDYQLHWTSTNWKGTNT
KDKWIDRSSERYKIDWEKEEMTNGSHRHR

SEQ ID NO: 31: Amino acid sequence of mature Hla H35L/G122C/H48C with N-terminal S, FlgI signal sequence, C-terminal GSHRHR and KDSNITSAR substitution for residue K131, Artificial sequence.

MIKFLSALILLLVTTAAQASADSDINIKTGTTDIGSNTTVKTGDLVTYDKENGMLKKVFYSFIDDKNCNKKLLV
IRTKGTIAGQYRVYSEEGANKSGLAWPSAFKVQLQLPDNEVAQISDYYPRNSIDTKEYMSTLTYGFNCNVTGDD
TGKDSNITSARIGGLIGANVSIGHTLKYVQPDFKTILESPTDKKVGWKVIFNNMVNQNWGPYDRDSWNPVYGNQ

LFMKTRNGSMKAADNFLDPNKASSLLSSGFSPDFATVITMDRKASKQQTNIDVIYERVRDDYQLHWTSTNWKGT
NTKDKWIDRSSERYKIDWEKEEMTNGSHRHR

SEQ ID NO: 32: Amino acid sequence of mature Hla H35L/G122C/H48C with N-terminal S, FlgI signal sequence, C-terminal GSHRHR and KDSNSTSAR substitution for residue K131, Artificial sequence.

MIKFLSALILLLVTTAAQASADSDINIKTGTTDIGSNTTVKTGDLVTYDKENGMLKKVFYSFIDDKNCNKKLLV
IRTKGTIAGQYRVYSEEGANKSGLAWPSAFKVQLQLPDNEVAQISDYYPRNSIDTKEYMSTLTYGFNCNVTGDD
TGKDSNSTSARIGGLIGANVSIGHTLKYVQPDFKTILESPTDKKVGWKVIFNNMVNQNWGPYDRDSWNPVYGNQ
LFMKTRNGSMKAADNFLDPNKASSLLSSGFSPDFATVITMDRKASKQQTNIDVIYERVRDDYQLHWTSTNWKGT
NTKDKWIDRSSERYKIDWEKEEMTNGSHRHR

SEQ ID NO: 33: Amino acid sequence of mature Hla H35L/G122C/H48C with N-terminal S, FlgI signal sequence, C-terminal GSHRHR and KDSNVTSAR substitution for residue K131, Artificial sequence.

MIKFLSALILLLVTTAAQASADSDINIKTGTTDIGSNTTVKTGDLVTYDKENGMLKKVFYSFIDDKNCNKKLLV
IRTKGTIAGQYRVYSEEGANKSGLAWPSAFKVQLQLPDNEVAQISDYYPRNSIDTKEYMSTLTYGFNCNVTGDD
TGKDSNVTSARIGGLIGANVSIGHTLKYVQPDFKTILESPTDKKVGWKVIFNNMVNQNWGPYDRDSWNPVYGNQ
LFMKTRNGSMKAADNFLDPNKASSLLSSGFSPDFATVITMDRKASKQQTNIDVIYERVRDDYQLHWTSTNWKGT
NTKDKWIDRSSERYKIDWEKEEMTNGSHRHR

SEQ ID NO: 34: Amino acid sequence of mature Hla H35L/G122C/H48C with N-terminal S, FlgI signal sequence, C-terminal GSHRHR and KDSNATSAR substitution for residue K131. Artificial sequence.

```
MIKFLSALILLLVTTAAQASADSDINIKTGTTDIGSNTTVKTGDLVTYDKENGMLKKVFYSFIDDKNCNKKLLV
IRTKGTIAGQYRVYSEEGANKSGLAWPSAFKVQLQLPDNEVAQISDYYPRNSIDTKEYMSTLTYGFNCNVTGDD
TGKDSNVTSARIGGLIGANVSIGHTLKYVQPDFKTILESPTDKKVGWKVIFNNMVNQNWGPYDRDSWNPVYGNQ
LFMKTRNGSMKAADNFLDPNKASSLLSSGFSPDFATVITMDRKASKQQTNIDVIYERVRDDYQLHWTSTNWKGT
NTKDKWIDRSSERYKIDWEKEEMTNGSHRHR
```

SEQ ID NO: 35: Amino acid sequence of mature Hla H35L/H48C/G122C with KDQNRTK substitution for residue K131. Artificial sequence.

```
ADSDINIKTGTTDIGSNTTVKTGDLVTYDKENGMLKKVFYSFIDDKNCNKKLLVIRTKGTIAGQYRVYSEEGAN
KSGLAWPSAFKVQLQLPDNEVAQISDYYPRNSIDTKEYMSTLTYGFNCNVTGDDTGKDQNRTKIGGLIGANVSI
GHTLKYVQPDFKTILESPTDKKVGWKVIFNNMVNQNWGPYDRDSWNPVYGNQLFMKTRNGSMKAADNFLDPNKA
SSLLSSGFSPDFATVITMDRKASKQQTNIDVIYERVRDDYQLHWTSTNWKGTNTKDKWIDRSSERYKIDWEKEE
MTN
```

SEQ ID NO: 36: Amino acid sequence of mature Hla H35L/G122C/H48C with KDSNITSAR substitution for residue K131. Artificial sequence.

```
ADSDINIKTGTTDIGSNTTVKTGDLVTYDKENGMLKKVFYSFIDDKNCNKKLLVIRTKGTIAGQYRVYSEEGAN
KSGLAWPSAFKVQLQLPDNEVAQISDYYPRNSIDTKEYMSTLTYGFNCNVTGDDTGKDSNITSARIGGLIGANV
SIGHTLKYVQPDFKTILESPTDKKVGWKVIFNNMVNQNWGPYDRDSWNPVYGNQLFMKTRNGSMKAADNFLDPN
KASSLLSSGFSPDFATVITMDRKASKQQTNIDVIYERVRDDYQLHWTSTNWKGTNTKDKWIDRSSERYKIDWEK
EEMTN
```

SEQ ID NO: 37: Amino acid sequence of mature Hla H35L/G122C/H48C with KDSNSTSAR substitution for residue K131. Artificial sequence.

```
ADSDINIKTGTTDIGSNTTVKTGDLVTYDKENGMLKKVFYSFIDDKNCNKKLLVIRTKGTIAGQYRVYSEEGAN
KSGLAWPSAFKVQLQLPDNEVAQISDYYPRNSIDTKEYMSTLTYGFNCNVTGDDTGKDSNSTSARIGGLIGANV
SIGHTLKYVQPDFKTILESPTDKKVGWKVIFNNMVNQNWGPYDRDSWNPVYGNQLFMKTRNGSMKAADNFLDPN
KASSLLSSGFSPDFATVITMDRKASKQQTNIDVIYERVRDDYQLHWTSTNWKGTNTKDKWIDRSSERYKIDWEK
EEMTN
```

SEQ ID NO: 38: Amino acid sequence of mature Hla H35L/G122C/H48C with KDSNVTSAR substitution for residue K131. Artificial sequence.

```
ADSDINIKTGTTDIGSNTTVKTGDLVTYDKENGMLKKVFYSFIDDKNCNKKLLVIRTKGTIAGQYRVYSEEGAN
KSGLAWPSAFKVQLQLPDNEVAQISDYYPRNSIDTKEYMSTLTYGFNCNVTGDDTGKDSNVTSARIGGLIGANV
SIGHTLKYVQPDFKTILESPTDKKVGWKVIFNNMVNQNWGPYDRDSWNPVYGNQLFMKTRNGSMKAADNFLDPN
KASSLLSSGFSPDFATVITMDRKASKQQTNIDVIYERVRDDYQLHWTSTNWKGTNTKDKWIDRSSERYKIDWEK
EEMTN
```

SEQ ID NO: 39: Amino acid sequence of mature Hla H35L/G122C/H48C with KDSNATSAR substitution for residue K131. Artificial sequence.

```
ADSDINIKTGTTDIGSNTTVKTGDLVTYDKENGMLKKVFYSFIDDKNCNKKLLVIRTKGTIAGQYRVYSEEGAN
KSGLAWPSAFKVQLQLPDNEVAQISDYYPRNSIDTKEYMSTLTYGFNCNVTGDDTGKDSNVTSARIGGLIGANV
SIGHTLKYVQPDFKTILESPTDKKVGWKVIFNNMVNQNWGPYDRDSWNPVYGNQLFMKTRNGSMKAADNFLDPN
KASSLLSSGFSPDFATVITMDRKASKQQTNIDVIYERVRDDYQLHWTSTNWKGTNTKDKWIDRSSERYKIDWEK
EEMTN
```

SEQ ID NO: 40: KDQNRTK glycosite. Artificial sequence.
KDQNRTK

SEQ ID NO: 41: KDQNATK glycosite. Artificial sequence.
KDQNATK

SEQ ID NO: 42: KDSNITSAR glycosite. Artificial sequence.
KDSNITSAR

SEQ ID NO: 43: KDSNSTSAR glycosite. Artificial sequence.
KDSNSTSAR

SEQ ID NO: 44: KDSNVTSAR glycosite. Artificial sequence.
KDSNVTSAR

SEQ ID NO: 45: KDSNATSAR glycosite. Artificial sequence.
KDSNATSAR

SEQ ID NO: 46: MS quantification-compatible PglB glycosite consensus sequence. Artificial sequence.
$K/R-Z_{0-9}-D/E-X-N-Y-S/T-Z_{0-9}-K/R$
wherein X and Y are independently any amino acid except proline, lysine or arginine, and Z represents any amino acid except cysteine, methionine, asparagine, glutamine, lysine or arginine.

SEQ ID NO: 47: MS quantification-compatible PglB glycosite consensus sequence. Artificial sequence.
$K/R-Z_{0-9}-D/E-X-N-Y-S/T-Z_{0-9}-K/R$
wherein X and Y are independently any amino acid except proline, cysteine, methionine, asparagine or glutamine, lysine or arginine, and Z represents any amino acid except cysteine, methionine, asparagine, glutamine, lysine or arginine.

**Claims**

1.  A modified carrier protein, modified in that it comprises one or more consensus sequence(s) comprising or consisting of a consensus sequence comprising or consisting of the following amino acid sequence:
K/R-$Z_{0-9}$-D/E-X-N-Y-S/T-$Z_{0-9}$-K/R

    a. wherein X and Y are independently any amino acid except proline, and Z represents any amino acid; wherein optionally X and Y are independently any amino acid except proline, lysine or arginine, Z represents any amino acid except lysine or arginine, and preferably Z represents any amino acid except cysteine, methionine, asparagine, glutamine, lysine or arginine (eg SEQ ID NO: 47);

    b. wherein said consensus sequence is optionally the amino acid sequence K/R-D/E-$X_1$-N-$X_2$-S/T-$Z_1$-$Z_2$-K/R (SEQ ID NO 19), wherein $X_1$ and $X_2$ are independently any amino acid apart from proline, and wherein $X_1$ and $X_2$ and $Z_1$ and $Z_2$.are preferably not lysine or arginine, wherein optionally, wherein $X_1$ and $X_2$ and $Z_1$ and $Z_2$ are not cysteine, asparagine, glutamine, methionine or arginine.

2. A modified carrier protein according to claim 1, wherein said consensus sequence comprises or consists of the amino acid sequence of SEQ ID NO: 20, optionally any one of SEQ ID Nos: 42-45, optionally SEQ ID Nos 42-44.

3. A modified carrier protein according to claim 1 or 2, wherein said consensus sequence (i) has been substituted for one or more amino acids of the carrier protein sequence, or (ii) has been inserted into the carrier protein sequence.

4. A modified carrier protein according to any one of claims 1-3, comprising more than one said consensus sequence, optionally at least 2, 3, 4 or 5 consensus sequences, wherein all of said consensus sequences have a different amino acid sequence.

5. A modified carrier protein according to any one of claims 1-4, wherein the carrier protein is CRM197, TT from *Clostridium tetani,* EPA from *P. aeruginosa,* Hcp1 from *P. aeruginosa,* Hla from *S. aureus,* ClfA from *S. aureus,* MBP from *E.,* PspA from *E. coli,* or MtrE from *N. gonorrhoeae.*

6. A modified carrier protein according to claim 5, wherein the carrier protein comprises or consists of an amino acid sequence of any one of SEQ ID Nos: 1 to 16 or an amino acid sequence at least 80%, 85%, 90%, 92%, 95%, 96%, 97%, 98% or 99% identical to any one of SEQ ID NOs. 1 to 16.

7. A conjugate comprising a modified carrier protein of any one of claims 1-6, wherein a polysaccharide is linked to an amino acid on the modified carrier protein selected from the group consisting of asparagine, aspartic acid, glutamic acid, lysine, cysteine, tyrosine, histidine, arginine and tryptophan, preferably asparagine.

8. The conjugate of claim 7, wherein the polysaccharide is a bacterial capsular polysaccharide selected from the group consisting of: *Staphylococcus aureus* type 5 capsular saccharide, *Staphylococcus aureus* type 8 capsular saccharide, *N. meningitidis* serogroup A capsular saccharide (MenA), *N. meningitidis* serogroup C capsular saccharide (MenC), *N. meningitidis* serogroup Y capsular saccharide (MenY), *N. meningitidis* serogroup W capsular saccharide (MenW), *H. influenzae* type b capsular saccharide (Hib), Group B Streptococcus group I capsular saccharide, Group B Streptococcus group II capsular saccharide, Group B Streptococcus group III capsular saccharide, Group B Streptococcus group IV capsular saccharide, Group B Streptococcus group V capsular saccharide, Vi saccharide from *Salmonella typhi, N. meningitidis* LPS (such as L3 and/or L2), *M. catarrhalis* LPS, *H. influenzae* LPS, Shigella O-antigens, *P.aeruginosa* O-antigens, *E. coli* O-antigens and *S. pneumoniae* or *S. aureus* capsular polysaccharide.

9. A polynucleotide encoding the modified carrier protein of any one of claims 1-6.

10. A vector comprising the polynucleotide of claim 9.

11. A host cell comprising:

    a. one or more nucleic acids that encode glycosyltransferase(s);
    b. a nucleic acid that encodes an oligosaccharyl transferase;
    c. a nucleic acid that encodes a modified carrier protein according to any one of claims 1-6; and optionally
    d. a nucleic acid that encodes a polymerase (*e.g. wzy*).

12. A process of producing a conjugate that comprises a modified carrier protein linked to a saccharide, said method comprising (i) culturing the host cell of claim 11 under conditions suitable for the production of proteins and (ii) isolating the conjugate.

**13.** An immunogenic composition comprising the modified carrier protein of any one of claims 1-6, or the conjugate of any one of claims 7-8.

**14.** A vaccine comprising the immunogenic composition of claim 13 and a pharmaceutically acceptable excipient or carrier.

**15.** A modified carrier protein of any one of claims 1-6, or the conjugate of any one of claims 7-8, or the immunogenic composition of claim 13, or the vaccine of claim 14, for use in the treatment or prevention of a disease caused by a bacterial infection.

**16.** The modified carrier protein, the conjugate, the immunogenic composition or the vaccine for use of claim 15, wherein said bacterial infection is an infection caused by a bacterium selected from the group consisting of *Staphylococcus aureus, N. meningitidis, H. influenzae, H. influenzae* type b, Group B Streptococcus, *S. typhi, M. catarrhalis* LPS, *S. flexneri, P.aeruginosa, E. coli* and *S. pneumoniae.*

**17.** A method of measuring the level of glycosylation site occupancy of a modified carrier protein according to any one of claims 1 to 6, said method comprising: digesting the glycosylated carrier protein with a protease; subjecting the digested protein to LC-MS; determining the concentration U of unmodified carrier protein; determining the concentration T of total carrier protein; and calculating glycosylation site occupancy according to the following equation:

$$\text{Site Occupancy (\%)} = \frac{(Total - unmodified)\ carrier\ concentration}{Total\ carrier\ concentration} x100$$

**18.** A method according to claim 17, wherein the concentration U of unmodified carrier protein is determined by determining the concentration of a peptide fragment corresponding to a consensus sequence.

**19.** A method according to claim 17 or claim 18, wherein the concentration T of total carrier protein is determined by determining the concentration of one or more peptide fragments which are unique to said carrier protein.

Fig 1

1. In silico Design of consensus sequences

2. Assess the behavior of the PTP in MS

3. Assess the efficacy of bioconjugation of the selected consensus sequence

4. Quantification of the extent of glycosylation of a carrier protein with single consensus sequence

5. Quantification of the extend of glycosylation of a carrier with multiple consensus sequence

**A**

Fig 2

**B**

| PTP-i | K | D | S | N | I | T | S | A | R |
| PTP-s | K | D | S | N | S | T | S | A | R |
| PTP-n | K | D | S | N | V | T | S | A | R |
| PTP-a | K | D | S | N | A | T | S | A | R |

Fig 3

Fig 4

Europäisches Patentamt

European Patent Office

Office européen des brevets

# EUROPEAN SEARCH REPORT

Application Number

EP 19 18 7963

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2019/121924 A1 (GLAXOSMITHKLINE BIOLOGICALS SA [BE]) 27 June 2019 (2019-06-27) | 1,3-19 | INV. C12P21/00 C07K14/31 |
| A | * the whole document * | 2 | C07K14/205 C07K14/21 |
| X,D | WO 2019/121926 A1 (GLAXOSMITHKLINE BIOLOGICALS SA [BE]) 27 June 2019 (2019-06-27) | 1,3-19 | C07K14/22 C07K14/245 C07K14/33 |
| A | * the whole document * | 2 | C12Q1/37 A61K39/085 |
| A | ZHU Z. ET AL: "Absolute Quantitation of Glycosylation Site Occupancy Using Isotopically Labeled Standards and LC-MS", JOURNAL OF THE AMERICAN SOCIETY FOR MASS SPECTROMETRY, vol. 25, no. 6, 27 March 2014 (2014-03-27), pages 1012-1017, XP035315802, ISSN: 1044-0305, DOI: 10.1007/S13361-014-0859-2 [retrieved on 2014-03-27] * the whole document * | 17-19 | A61K39/02 G01N33/68 |

**TECHNICAL FIELDS SEARCHED (IPC)**

| Category | Citation of document with indication, where appropriate, of relevant passages | Relevant to claim | |
|---|---|---|---|
| A | KAY E. ET AL: "Recent advances in the production of recombinant glycoconjugate vaccines", NPJ VACCINES, vol. 4, 16, 1 May 2019 (2019-05-01), pages 1-8, XP055612407, DOI: 10.1038/s41541-019-0110-z * the whole document * | 1-19 | C12P C07K A61K C12Q G01N |

The present search report has been drawn up for all claims

| Place of search | Date of completion of the search | Examiner |
|---|---|---|
| The Hague | 6 August 2020 | van de Kamp, Mart |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 18 7963

This annex lists the patent family members relating to the patent documents cited in the above-mentioned European search report.
The members are as contained in the European Patent Office EDP file on
The European Patent Office is in no way liable for these particulars which are merely given for the purpose of information.

06-08-2020

| Patent document cited in search report | | | Publication date | Patent family member(s) | | | Publication date |
|---|---|---|---|---|---|---|---|
| WO 2019121924 | | A1 | 27-06-2019 | CA | 3086262 | A1 | 27-06-2019 |
| | | | | WO | 2019121924 | A1 | 27-06-2019 |
| WO 2019121926 | | A1 | 27-06-2019 | CA | 3086264 | A1 | 27-06-2019 |
| | | | | WO | 2019121926 | A1 | 27-06-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2019121924 A
- WO 20119121926 A
- WO 2019121926 A
- EP 2019053463 W
- EP 2013068737 W
- WO 14037585 A
- WO 06119987 A
- WO 09104074 A
- WO 1162615 A
- WO 11138361 A
- WO 1457109 A
- WO 1472405 A
- WO 1620499 A
- WO 2003074687 A
- WO 2006119987 A
- WO 2009104074 A
- WO 201106261 A
- WO 2011138361 A
- WO 2016107818 A
- US 4235877 A, Fullerton
- GB 2220211 A
- US 2007064857 W
- WO 2007109812 A
- US 2007064858 W
- WO 2007109813 A
- US 5057540 A
- EP 689454 B1
- WO 9602555 A
- EP 399843 A
- WO 9517210 A
- WO 9912565 A
- WO 9911241 A
- WO 09127676 A
- WO 09127677 A
- EP 2018085854 W

### Non-patent literature cited in the description

- **WACKER et al.** *Science,* 2002, vol. 298, 1790-3
- **WETTER et al.** *Glycoconj J.,* 2013, vol. 30, 511-22
- **WACKER et al.** *J. Infect. Dis.,* 2014, vol. 209, 1551-1561
- **VAN DEN DOBBELSTEEN et al.** *Vaccine,* 2016, vol. 34, 4152-60
- **MICOLI, F. et al.** *Molecules,* 2018, vol. 23, 1451
- **ZHU et al.** *J Am Soc Mass Spectrom.,* 2015, vol. 25, 1012-7
- **KOWARIK et al.** *EMBO J.,* 2006, vol. 25 (9), 1957-66
- Current Protocols in Molecular Biology. 1987
- **SMITH ; WATERMAN.** *Adv. Appl. Math.,* 1981, vol. 2, 482-489
- **WACKER et al.** *J Infect. Dis.,* 2014, vol. 209, 1551-1561
- **JAMESON ; WOLF.** *CABIOS,* 1988, vol. 4, 181-186
- **MENZIES ; KERNODLE.** *Infect Immun,* 1994, vol. 62, 1843-1847
- **FELDMAN et al.** *PNAS USA,* 2005, vol. 102, 3016-3021
- **WACKER et al.** *Science,* 2002, vol. 298 (5599), 1790-3
- **NITA-LAZAR et al.** *Glycobiology,* 2005, vol. 15 (4), 361-7
- **FELDMAN et al.** *Proc Natl Acad Sci U S A,* 2005, vol. 102 (8), 3016-21
- **WACKER et al.** *Proc Natl Acad Sci U S A.,* 2006, vol. 103 (18), 7088-93
- **WACKER et al.** *Science,* 2002, vol. 298, 1790-1793
- **SARASWAT et al.** *Biomed. Res. Int. ID#312709,* 2013, 1-18
- **E. W. MARTIN.** Remington's Pharmaceutical Sciences. Mack Publishing Co,
- The subunit and adjuvant approach. Vaccine Design. Plenum Press, 1995
- **KENSIL et al.** Vaccine Design: The Subunit and Adjuvant Approach. Plenum Press, 1995
- **STOUTE et al.** *N. Engl. J. Med.,* 1997, vol. 336, 86-91
- *Bioworld Today,* 15 November 1998
- **MOSMANN, T.R. ; COFFMAN, R.L.** TH1 and TH2 cells: different patterns of lymphokine secretion lead to different functional properties. *Annual Review of Immunology,* 1989, vol. 7, 145-173
- **THOELEN et al.** *Vaccine,* 1998, vol. 16, 708-14

- **WACKER, M. et al.** *J. Infect. Dis.,* 2014, vol. 209, 1551-1561
- **CARR et al.** *Mol Cell Proteomics,* 2014, vol. 13 (3), 907-917
- **KOWARIK et al.** *EMBO J.,* 2006, vol. 25, 1957-1966
- **ZHU et al.** *J Am Soc Mass Spectrom.,* 2014, vol. 25, 1012-7
- **SILVERMAN ; IMPERIALI.** *J. Biol. Chem.,* 2016, vol. 291, 22001-22010
- **LANGE et al.** *Mol. Syst. Biol.,* 2008, vol. 4, 222
- **BIAGINI M et al.** *Proc Natl Acad Sci USA.,* 2016, vol. 113, 2714-9
- **HÜTTENHAIN et al.** *Curr. Opin. Chem. Biol.,* 2009, vol. 13, 518-525